# EUROPEAN PATENT SPECIFICATION

(11) **EP 0 654 089 B1**
(45) Date of publication and mention of the grant of the patent: **02.07.2003**
(21) Application number: 93919918.8
(22) Date of filing: 06.08.1993
(51) Int. Cl.: C12N 15/38, C12N 15/86, C12N 7/01, C12N 7/04, C07K 14/03, A61K 39/245

(54) **RECOMBINANT EQUINE HERPESVIRUSES**
REKOMBITANTE, EQUINE HERPESVIREN
VIRUS DE L'HERPES EQUIN RECOMBINANT

(30) Priority: 07.08.1992 US 926784
(43) Date of publication of application: 24.05.1995
(73) Proprietor: SYNTRO CORPORATION, San Diego California 92121 (US)
(72) Inventor: COCHRAN, Mark, D., Carlsbad, CA 92008 (US); CHIANG, Christina H., San Diego, California 92130 (US)
(74) Representative: VOSSIUS & PARTNER
(86) International application number: US9307424
(87) International publication number: WO94003628

(56) References cited:
- EP-A- 0 507 179
- WO-A-92/01045
- WO-A-93/24528
- DATABASE EMBL Heidelberg,BRD AC M80429, 3 February 1992 XP002022451 & VIROLOGY, vol. 191, no. 2, December 1992, pages 649-660, BREEDEN C. ET AL.: "Identification and transcriptional mapping of genes encoded at the IR/US junction of Equine Herpesvirus Type 1"
- ALAN R. LISS, "Technological Advances in Vaccine Development", published 1988 by Alan R. Liss, Inc., pages 183-195, see entire document.
- Proceedings of the National Academy of Sciences, Vol. 81, issued September 1984, M-F. SHIH et al., "Expression of Hepatitis B Virus S Gene by Herpes Simplex Virus Type 1 Vectors Carrying A and B-Regulated Gene Chimeras", pages 5867-5870, see Figures 1 and 2 and page 5870.

## Description

Within this application, several publications are referenced by Arabic numerals within parentheses. Full citations for these references may be found at the end of the specification immediately preceding the claims. The disclosures of these publications describe the state of the art to which this invention pertains.

The present invention involves live recombinant equine herpesviruses which comprises the genomic DNA of equine herpesvirus 1 (EHV-1) or 4 (EHV-4) from which a DNA sequence has been deleted from the US2 gene, shown for EHV-1 in SEQ ID NO: 1 and for EHV-4 in SEQ ID NO: 3 inactivating said US2 gene. They are useful in the preparation of vaccines to protect horses from various species of naturally-occurring infectious equine herpesvirus. The equine herpesvirus is a member of the family herpesviridae, which are commonly known as the herpesviruses.

Generally, herpesviruses contain 100,000 to 200,000 base pairs of DNA as their genetic material, and several areas of the genomes of various members have been identified that are not essential for the replication of virus in vitro in cell culture. Modifications of these regions of the DNA have been known to lower the pathogenicity of the virus, i.e. to attenuate the virus when it infects an animal species. For example, inactivation of the thymidine kinase gene of either human herpes simplex virus (29) or pseudorabies virus of swine (38) renders these herpesviruses less pathogenic.

Removal of specific regions of the repeat region of a human herpes simplex virus have been shown to render the virus less pathogenic (32, 39). Furthermore, a repeat region has been identified in Marek's disease virus that is associated with viral oncogenicity (13). A region in herpesvirus saimiri has similarly been correlated with oncogenicity (21). Removal of a specific region of the repeat region renders pseudorabies virus less pathogenic (US Pat. 4,877,737). A region'in pseudorabies virus has been shown to be deleted in naturally-occurring vaccine strains (22). These deletions are at least in part responsible for the lack of pathogenicity of these strains.

It is generally agreed that herpesviruses contain non-essential regions of DNA in various parts of the genome, and that modification of these regions can attenuate the virus, leading to a non-pathogenic strain from which a vaccine may be derived. An attenuated EHV-4 vaccine with a deletion of and/or an insertion in the thymidine kinase gene has been described (53). The degree of attenuation of the virus is important to the utility of the virus as a vaccine. Deletions which cause too much attenuation of the virus will result in a vaccine that fails to elicit an adequate immune response. Although several examples of attenuating deletions are known, the appropriate combination of deletions for any herpesvirus is not readily apparent.

Major economic losses to the equine industry result from infection by two species of equine herpesvirus (17). These two equine herpesvirus species, currently identified in the literature as EHV-1 and EHV-4, belong to the herpesvirus sub-family alpha-herpesvirus and are characterized by a class D genome (33). Formerly, both species were identified as EHV-1 and further differentiated as EHV-1 subtype 1 (EHV-1) and EHV-1 subtype 2 (EHV-4) respectively. EHV-1 is the primary cause of abortion in pregnant mares and EHV-4 is the primary cause of respiratory disease in foals and yearlings. Currently available products are not designed to address both disease syndromes, with the result that these products are marginally effective.

EHV-1 and EHV-4 have been analyzed at the molecular level. Restriction maps of the genomes of EHV-1 and EHV-4 have been reported (42 and 8).

Although several of the herpesviruses have been genetically engineered, no examples of recombinant EHV have been reported.

EHV can become latent in healthy animals which makes them potential carriers of the virus. For this reason, it is clearly advantageous to be able to distinguish animals vaccinated with non-virulent virus from animals infected with disease-causing wild-type or naturally-occurring virus. The development of differential vaccines and companion diagnostic tests has proven valuable in the management of pseudorabies disease (Federal Register, Vol. 55, No. 90, pp. 19245-19253). A similar differential marker vaccine would be of great value in the management of EHV caused disease.

The present invention provides a method of producing a fetal-safe, live recombinant EHV virus which comprises treating viral DNA from a naturally-occurring live EHV so as to delete from such viral DNA, DNA corresponding to the US2 gene of the naturally-occurring EHV. The present invention also provides viruses in which (a) DNA corresponding to the US2 gene has been deleted, and (b) DNA encoding gpG, gpE, and/or TK has been altered or deleted. Such viruses are useful for the creation of vaccines which require diagnostic markers and safety in pregnant animals.

The ability to engineer DNA viruses with large genomes, such as vaccinia virus and the herpesviruses, has led to the finding that these recombinant viruses can be used as vectors to deliver vaccine antigens and therapeutic agents for animals. The herpesviruses are attractive candidates for development as vectors because their host range is primarily limited to a single target species (16) and they have the capacity for establishing latent infection (7) that could provide for stable in vivo expression of a foreign gene. Although several herpesvirus species have been engineered to express foreign gene products, recombinant equine herpesviruses expressing foreign gene products have not been constructed. The equine herpesviruses described above may be used as vectors for the delivery of vaccine antigens from microorganisms causing important equine diseases. Such multivalent recombinant viruses would protect against EHV as well as other diseases. Similarly the equine herpesviruses may be used as vectors for the delivery of therapeutic agents. The therapeutic agent that is delivered by a viral vector of the present invention must be a biological molecule that is a byproduct of equine herpesvirus replication. This limits the therapeutic agent in the first analysis to either DNA, RNA or protein. There are examples of therapeutic agents from each of these classes of compounds in the form of anti-sense DNA, anti-sense RNA (19), ribozymes (41), suppressor tRNAs (3), interferon-inducing double stranded RNA and numerous examples of protein therapeutics, from hormones, e.g., insulin, to lymphokines, e.g., interferons and interleukins, to natural opiates. The discovery of these therapeutic agents and the elucidation of their structure and function does not necessarily allow one to use them in a viral vector delivery system, however, because of the experimentation necessary to determine whether an appropriate insertion site exists.

The invention provides a live recombinant equine herpesvirus which comprises the genomic DNA of equine herpesvirus 1 (EHV-1) or 4 (EHV-4) from which a DNA sequence has been deleted from the US2 gene, shown for EHV-1 in SEQ ID NO: 1 and for EHV-4 in SEQ ID NO: 3 inactivating said US2 gene. The invention discloses isolated DNA encoding the US2 protein of an equine herpesvirus.

The invention provides a recombinant equine herpesvirus as defined above capable of replication which further contains a DNA sequence inserted therein. This herpesvirus comprises viral DNA from the species of the naturally-occuring equine herpesvirus 1 or 4 with the deletions indicated above and foreign DNA encoding RNA which does not naturally occur in an animal into which the recombinant equine herpesvirus is introduced, the foreign DNA being inserted into the recombinant equine herpesviral DNA at a site which is not essential for replication of the equine herpesvirus.

The invention describes a homology vector for producing the recombinant equine herpesvirus of the present invention by inserting foreign DNA into a genome of an equine herpesvirus which comprises a double-stranded DNA molecule consisting essentially of: a) a double-stranded foreign DNA sequence encoding RNA which does not naturally occur in an animal into which the recombinant equine herpesvirus is introduced; b)at one end of the foreign DNA sequence, double-stranded equine herpesviral DNA homologous to genomic DNA located at one side of a site on the genome which is not essential for replication of the equine herpesvirus; and c) at the other end of the foreign DNA, double-stranded equine herpesviral DNA homologous to genomic DNA located at the other side of the same site on the genome.

The invention discloses a method of producing the fetal-safe, live recombinant equine herpesvirus of the present invention which comprises treating viral DNA from a naturally-occurring live equine herpesvirus so as to delete from the virus DNA from the US2 region of the naturally-occurring equine herpesvirus.

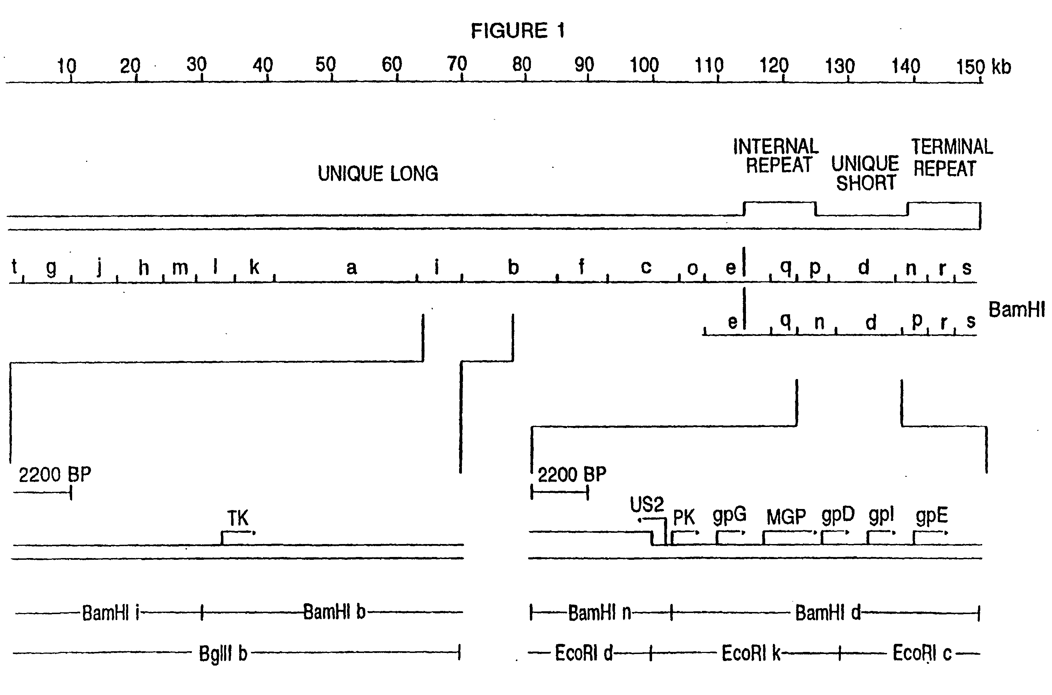

- FIGURE 1: Details of the EHV1 Dutta Strain. Diagram of EHV1 genomic DNA showing the unique long, internal repeat, unique short, and Terminal repeat regions. A restriction map for the enzyme BamHI is indicated (42). Fragments are lettered in order of decreasing size. The unique short region and the thymidine kinase region are expanded showing the locations of fragments BglII b, EcoRI d, k and c. The location of several genes is indicated they are thymidine kinase (Tk), unique short 2 (US2), glycoproteins G (gpG), D (gpD), I (gpI), and E (gpE) (1).

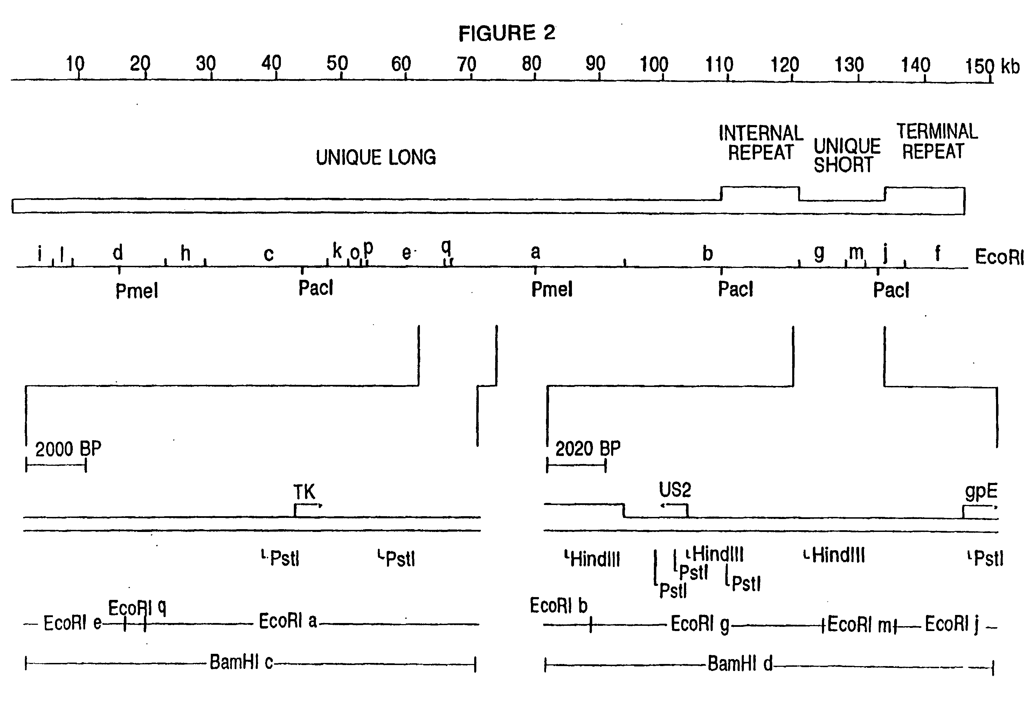

- FIGURE 2: Details of the EHV4 Dutta Strain. Diagram of EHV4 genomic DNA showing the unique long, internal repeat, unique short, and Terminal repeat regions. Restriction maps for the enzymes EcoRI, PacI and PmeI are indicated. Fragments are lettered in order of decreasing size. The unique short region and the thymidine kinase region are expanded showing. the locations of fragments BamHI c, d. The locations of two genes are also indicated, they are thymidine kinase (Tk) (27, 28) and unique short 2 (US2).

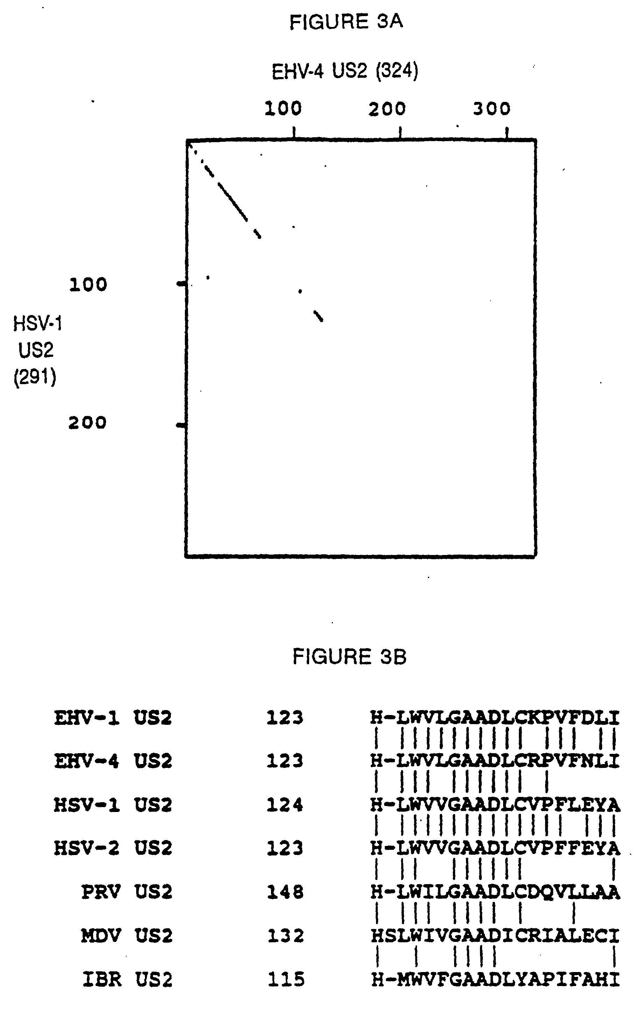

- FIGURE 3: Homology between the equine herpesvirus US2 proteins and the US2 Proteins of HSV-1, PRV, HSV-2, and MDV. (a) Matrix plot of the amino acid sequence of the EHV-4 US2 protein (324 amino acids) (SEQ ID NO: 4) against the amino acid sequence of the HSV-1 US2 protein (291 amino acids) (24). (b) Alignment of the conserved region (SEQ ID HO: 7) between EHV-1 US2 protein (303 amino acids) (SEQ ID NO: 2), EHV-4 US2 protein (SEQ ID NO: 8), HSV-1 US2 protein (SEQ ID NO: 9), PRV US2 protein (SEQ. ID NO: 11) (256 amino acids) (49) HSV-2 US2 protein (SEQ ID NO: 10) (291 amino acids) (25), MDV US2 protein (SEQ ID NO: 12) (270 amino acids) (4), and IBR US2 (SEQ ID NO: 13).

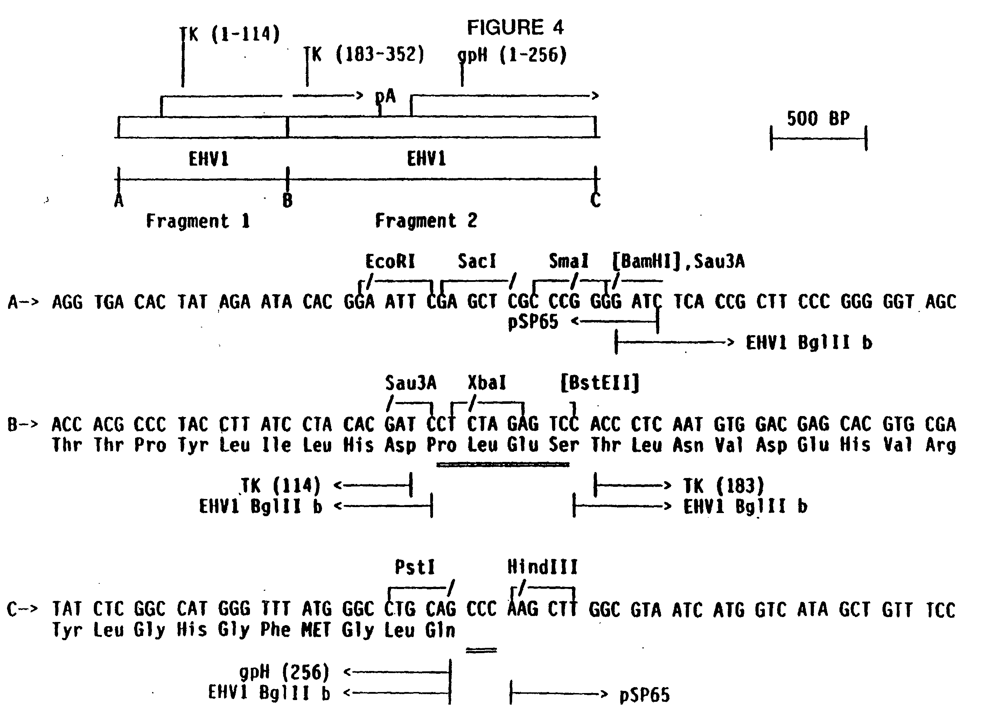

- FIGURE 4: Detailed description of the DNA insertion in Homology Vector 450-46.B4. The diagram shows the orientation of DNA fragments assembled in plasmid 450-46.B4. The origin of each fragment is described in the Materials and Methods section. The sequences located at the junctions between each fragment are shown, including junction A (SEQ ID NO: 14), junction B (SEQ ID NO: 15), and junction C (SEQ ID NO: 17). The restriction sites used to generate each fragment as well as synthetic linker sequences which were used to join the fragments are described for each junction. The synthetic linker sequences are underlined by a double bar. The location of several gene coding regions and regulatory elements is also given. The following two conventions are used: numbers in parenthesis () refer to amino acids, and restriction sites in brackets [] indicate the remnants of sites which were destroyed during construction. The following abbreviations are used, equine herpesvirus 1 (EHV1), thymidine kinase (TK), glycoprotein H (gpH), and poly adenylation signal (pA).

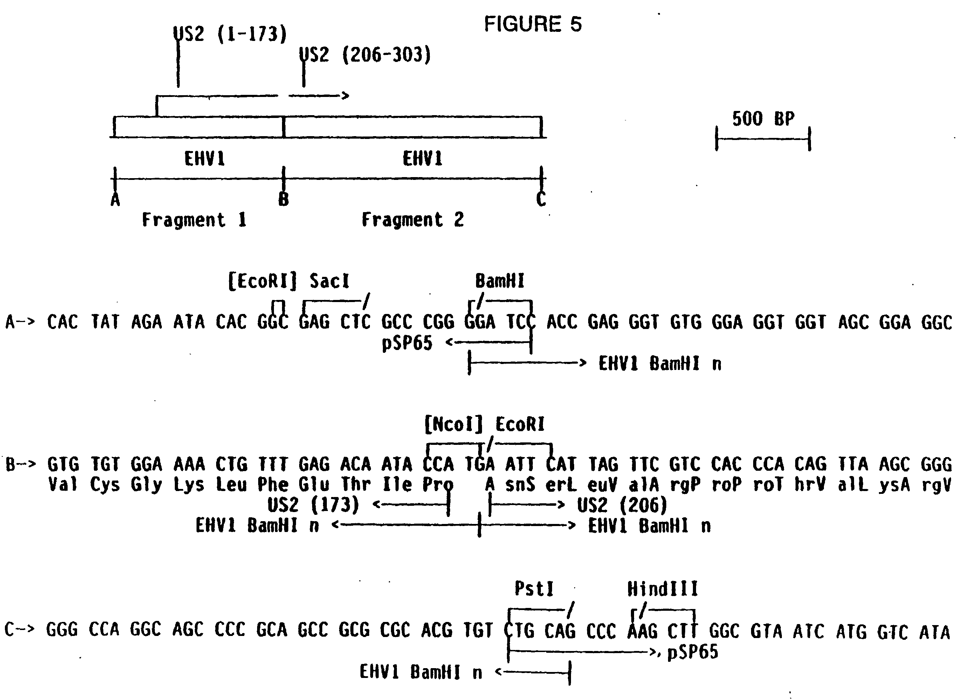

- FIGURE 5: Detailed description of the DNA insertion in Homology Vector 467-21.19. The diagram shows the orientation of DNA fragments assembled in plasmid 467-21.19. The origin of each fragment is described in the Materials and Methods section. The sequences located at the junctions between each fragment are shown, including junction A (SEQ ID NO: 19), junction B (SEQ ID NO: 20) and junction C (SEQ ID NO: 23). The restriction sites used to generate each fragment are indicated at the appropriate junction. The location of the US2 gene coding region is also given. The following two conventions are used: numbers in parenthesis () refer to amino acids, and restriction sites in brackets [] indicate the remnants of sites which were destroyed during construction. The following abbreviations are used, equine herpesvirus 1 (EHV1) and unique short 2 (US2).

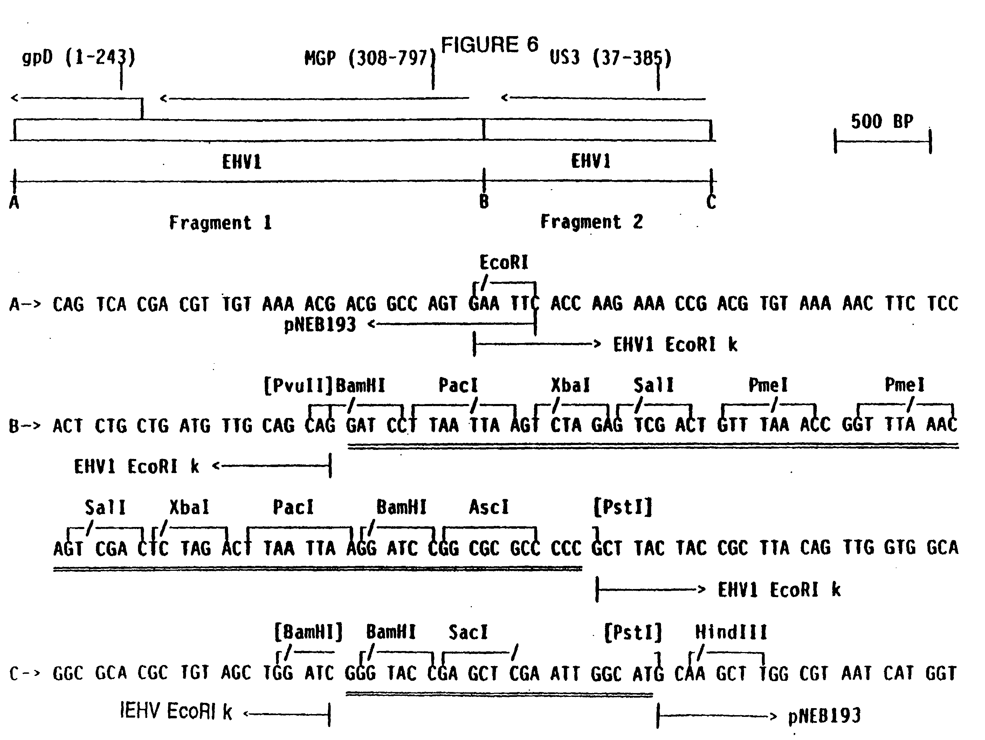

- FIGURE 6: Detailed description of the DNA insertion in Homology Vector 536-85.30. The diagram shows the orientation of DNA fragments assembled in plasmid 536-85.30. The origin of each fragment is described in the Materials and Methods section. The sequences located at the junctions between each fragment are shown, including junction A (SEQ ID NO: 24), junction B (SEQ ID NO: 25), and junction C (SEQ ID NO: 26). The restriction sites used to generate each fragment are indicated at the appropriate junction. The location of the gpD, MGP, and US3 gene coding regions are also given. Restriction sites in brackets [] indicate the remnants of sites which were destroyed during construction. The following abbreviations are used, equine herpesvirus 1 (EHV1), membrane glycoprotein (MGP), unique short 3 (US3) glycoprotein D (gpD).

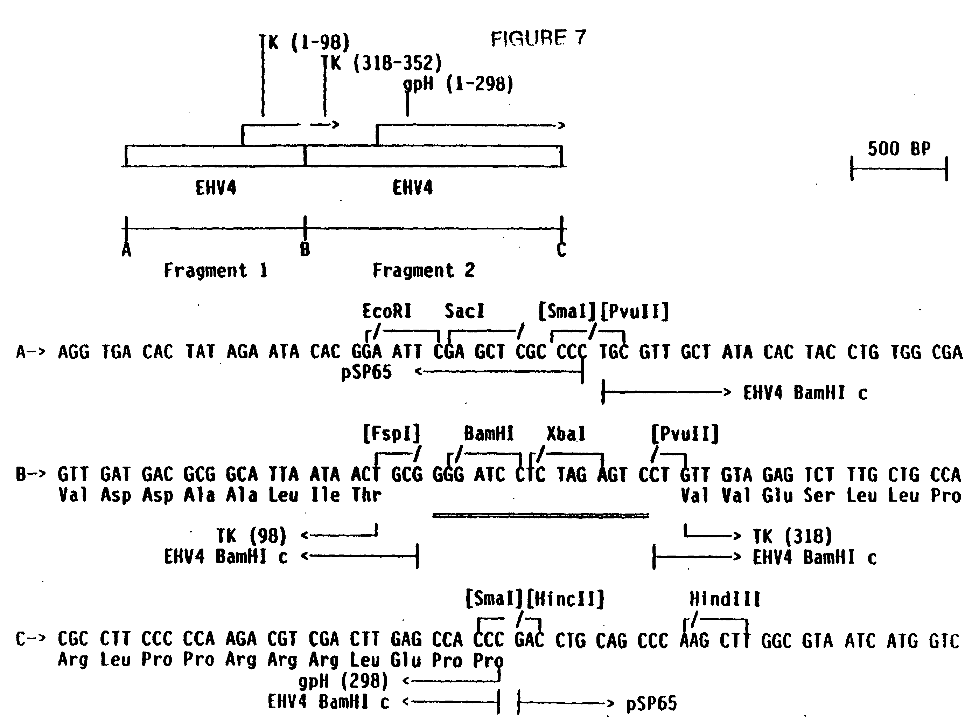

- FIGURE 7: Detailed description of the DNA insertion in Homology Vector 495-61.39. The diagram shows the orientation of DNA fragments assembled in plasmid 495-61.39. The origin of each fragment is described in the Materials and Methods section. The sequences located at the junctions between each fragment are shown, including junction A (SEQ ID NO: 27), junction B (SEQ ID NO: 28), and junction C (SEQ ID NO: 31). The restriction sites used to generate each fragment as well as synthetic linker sequences which were used to join the fragments are described for each junction. The synthetic linker sequences are underlined by a double bar. The location of the TK and gpH gene coding regions are also given. The following two conventions are used: numbers in parenthesis () refer to amino acids, and restriction sites in brackets [] indicate the remnants of sites which were destroyed during construction. The following abbreviations are used, equine herpesvirus 4 (EHV4) and glycoprotein H (gpH).

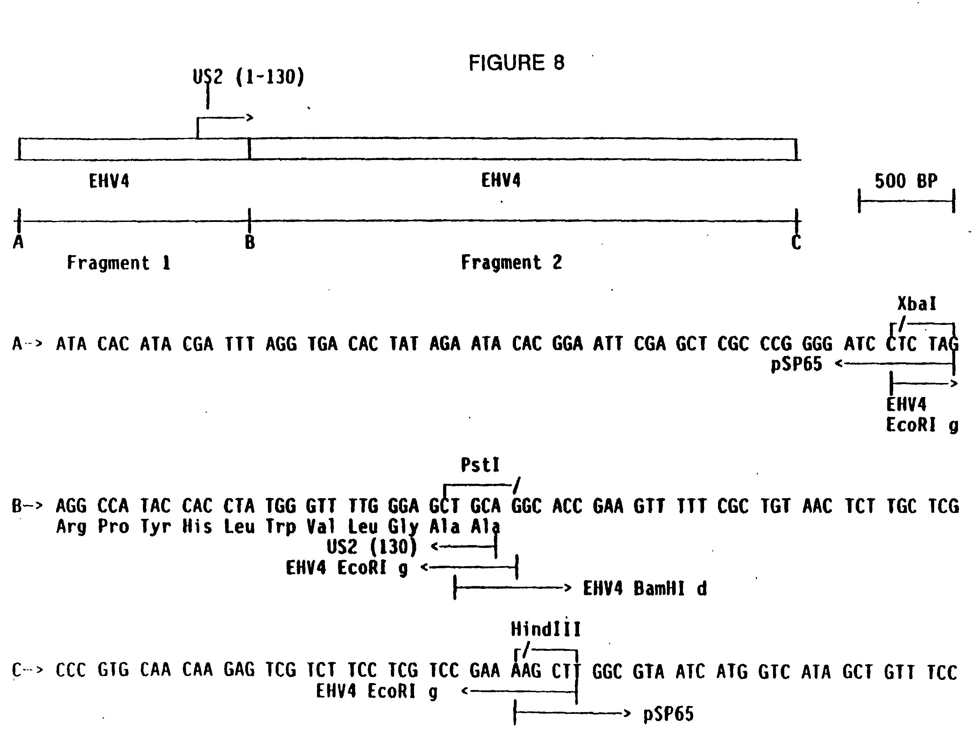

- FIGURE 8: Detailed description of the DNA insertion in Homology Vector 523-38.9. The diagram shows the orientation of DNA fragments assembled in plasmid 523-38.9. The origin of each fragment is described in the Materials and Methods section. The sequences located at the junctions between each fragment are shown, including junction A (SEQ ID NO: 33), junction B (SEQ ID NO: 34), and junction C (SEQ ID HO: 36). The restriction sites used to generate each fragment are indicated at the appropriate junction. The location of the US2 gene coding region is also given. The following two conventions are used: numbers in parenthesis () refer to amino acids, and restriction sites in brackets [] indicate the remnants of sites which were destroyed during construction. The following abbreviations are used, equine herpesvirus 4 (EHV4) and unique short 2 (US2).

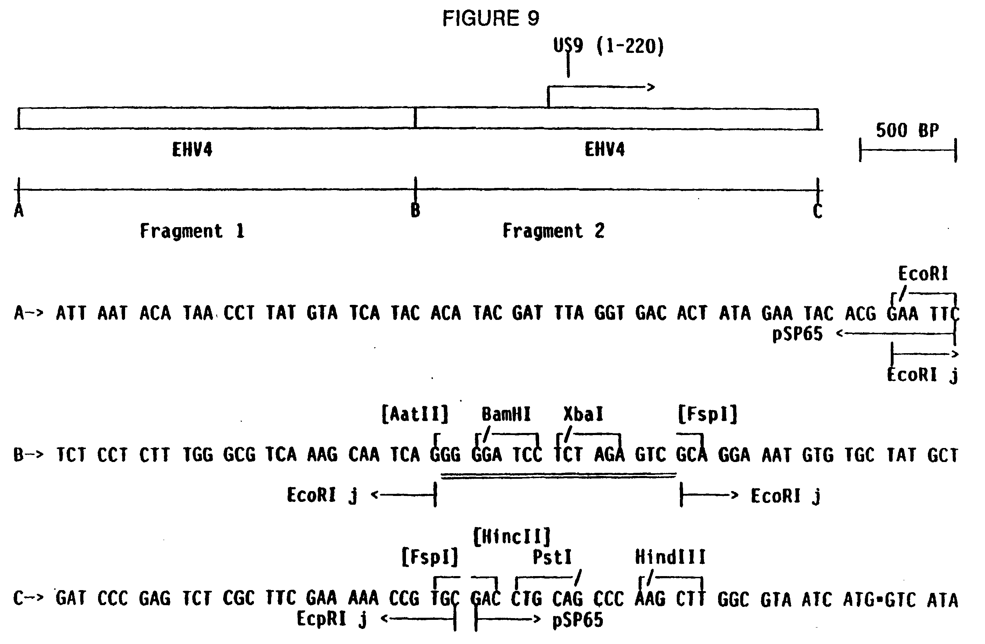

- FIGURE 9: Detailed description of the DNA insertion in Homology Vector 580-57.25. The diagram shows the orientation of DNA fragments assembled in plasmid 580-57.25. The origin of each fragment is described in the Materials and Methods section. The sequences located at the junctions between each fragment are shown, including junction A (SEQ ID NO: 37), junction B (SEQ ID NO: 38), and junction C (SEQ ID NO: 39). The restriction sites used to generate each fragment are indicated at the appropriate junction. The location of the US9 gene coding region is also given. The following two conventions are used: numbers in parenthesis () refer to amino acids, and restriction sites in brackets [] indicate the remnants of sites which were destroyed during construction. The following abbreviations are used, equine herpesvirus 4 (EHV4) and unique short 9 (US9).

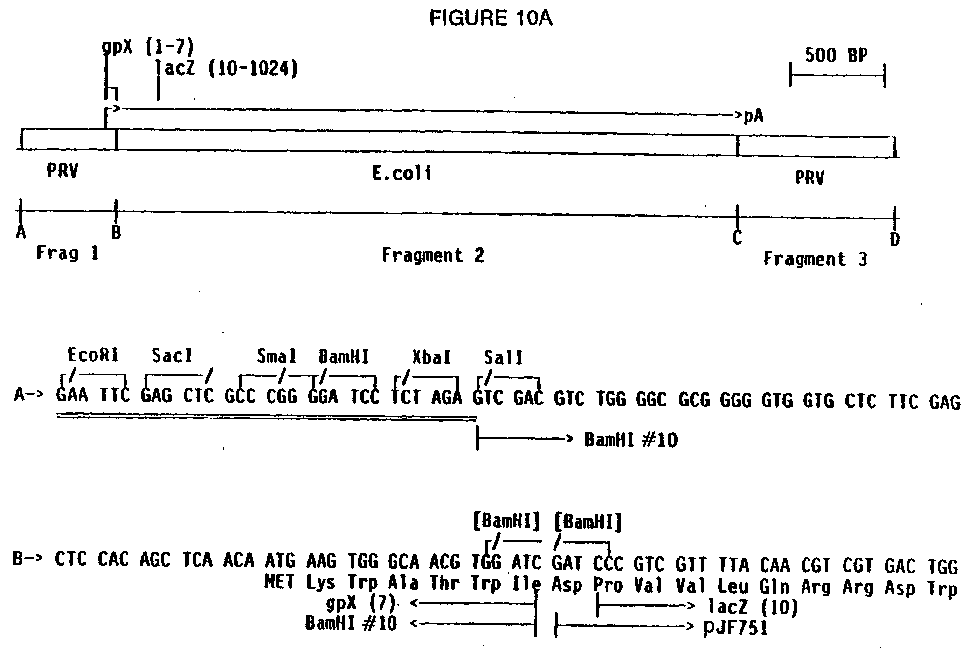

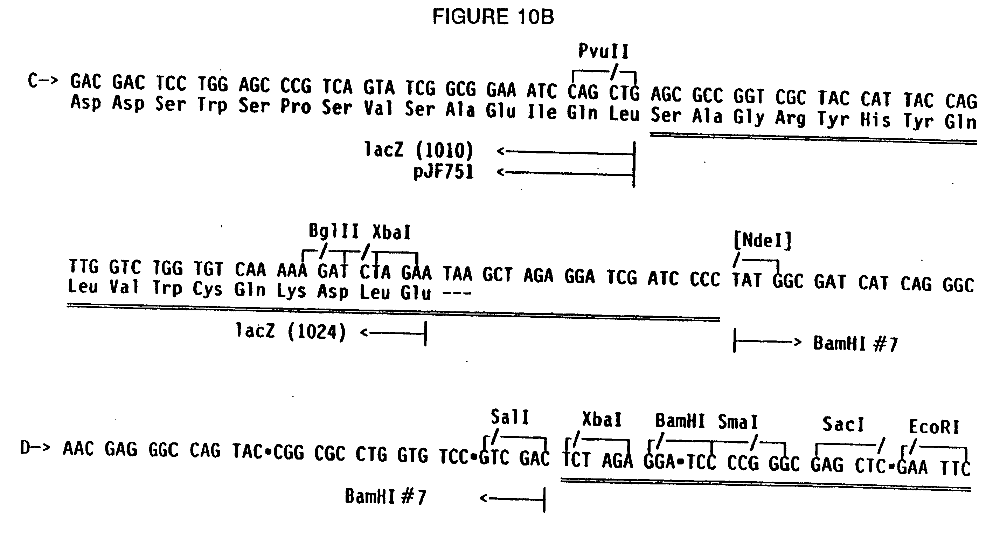

- FIGURE 10: Detailed description of the marker gene insertion in Homology Vector 467-22.A12. The diagram shows the orientation of DNA fragments assembled in the marker gene. The origin of each fragment is described in the Materials and Methods section. The sequences located at the junctions between each fragment and at the ends of the marker gene are shown, including junction A (SEQ ID NO: 40), junction B (SEQ ID NO: 41), junction C (SEQ ID NO: 43) and junction D (SEQ ID NO: 43). The restriction sites used to generate each fragment are indicated at the appropriate junction. The location of the lacZ gene coding region is also given. The following two conventions are used: numbers in parenthesis () refer to amino acids, and restriction sites in brackets [] indicate the remnants of sites which were destroyed during construction. The following abbreviations are used, pseudorabies virus (PRV), lactose operon Z gene (lacZ), Escherichia coli (E.coli), poly adenylation signal (pA), and glycoprotein X (gpX).

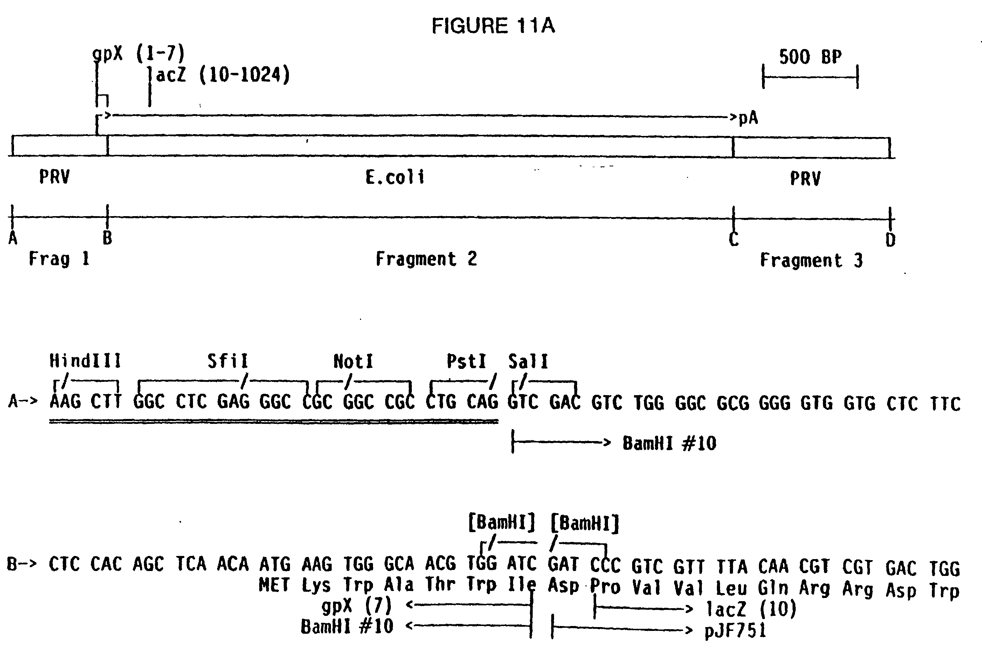

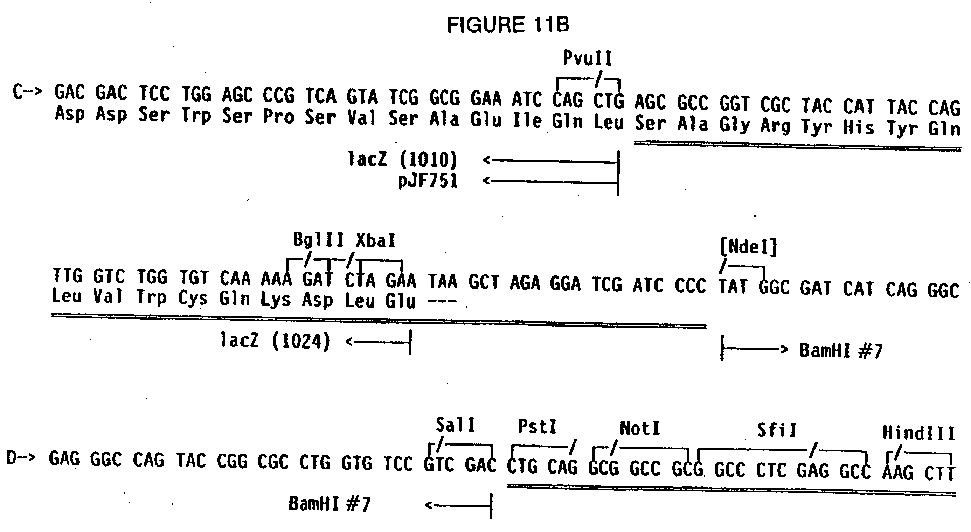

- FIGURE 11: Detailed description of the marker gene insertion in Homology Vector 523-42.A18. The diagram shows the orientation of DNA fragments assembled in the marker gene. The origin of each fragment is described in the Materials and Methods section. The sequences located at the junctions between each fragment and at the ends of the marker gene are shown, including junction A (SEQ ID NO: 46), 1 junction B (SEQ ID NO: 47), junction C (SEQ ID NO: 49), and junction D (SEQ ID NO: 51). The restriction sites used to generate each fragment are indicated at the appropriate junction. The location of the lacZ gene coding region is also given. The following two conventions are used: numbers in parenthesis () refer to amino acids, and restriction sites in brackets [] indicate the remnants of sites which were destroyed during construction. The following abbreviations are used, pseudorabies virus (PRV), lactose operon Z gene (lacZ), Escherichia coli (E.coli), poly adenylation signal (pA) and glycoprotein X (gpX).

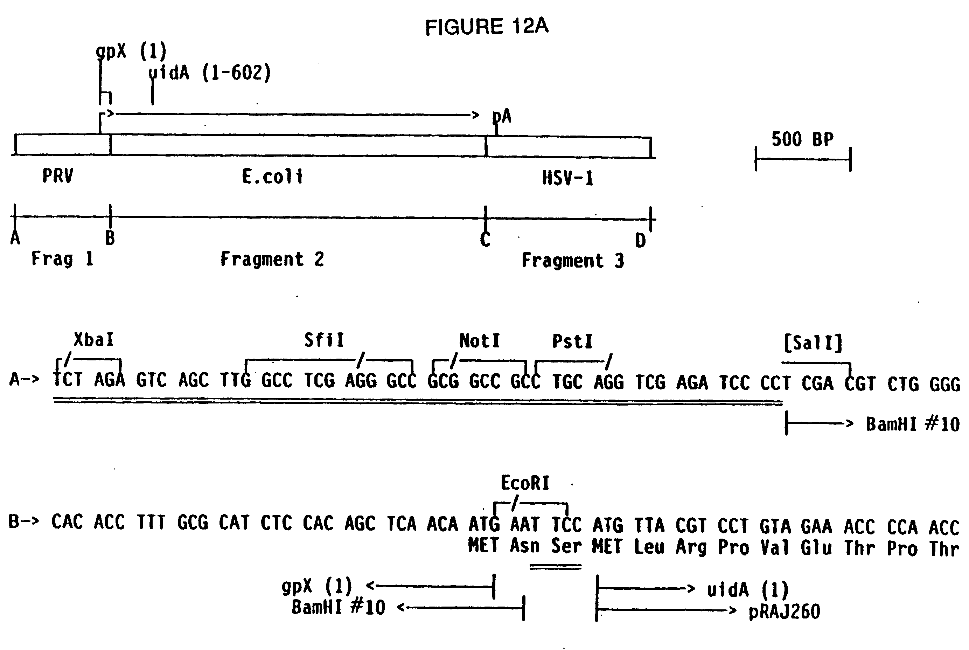

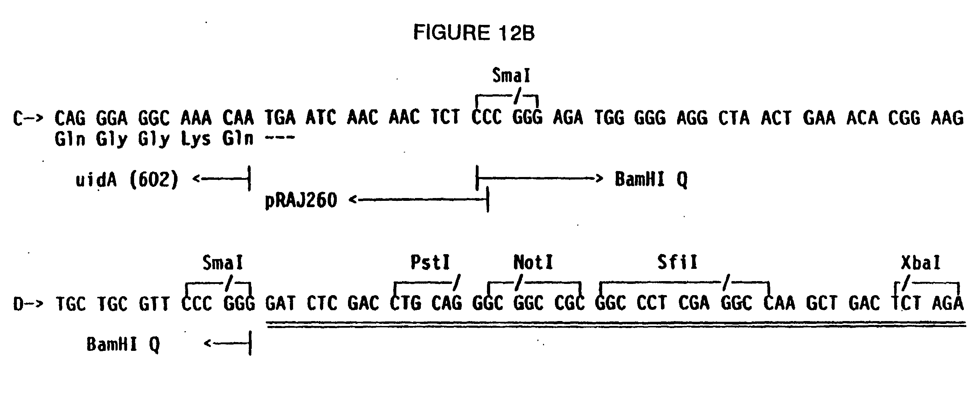

- FIGURE 12: Detailed description of the marker gene insertion in Homology Vector 552-45.19. The diagram shows the orientation of DNA fragments assembled in the marker gene. The origin of each fragment is described in the Materials and Methods section. The sequences located at the junctions between each fragment and at the ends of the marker gene are shown, including junction A (SEQ ID NO: 52), junction B (SEQ ID NO: 53), junction C (SEQ ID NO: 55) and junction D (SEQ ID NO: 57). The restriction sites used to generate each fragment are indicated at the appropriate junction. The location of the uidA gene coding region is also given. The following two conventions are used: numbers in parenthesis () refer to amino acids, and restriction sites in brackets [] indicate the remnants of sites which were destroyed during construction. The following abbreviations are used, pseudorabies virus (PRV), uronidase A gene (uidA), Escherichia coli (E.coli), herpes simplexvirus type 1 (HSV- 1), poly adenylation signal (pA), and glycoprotein X (gpX).

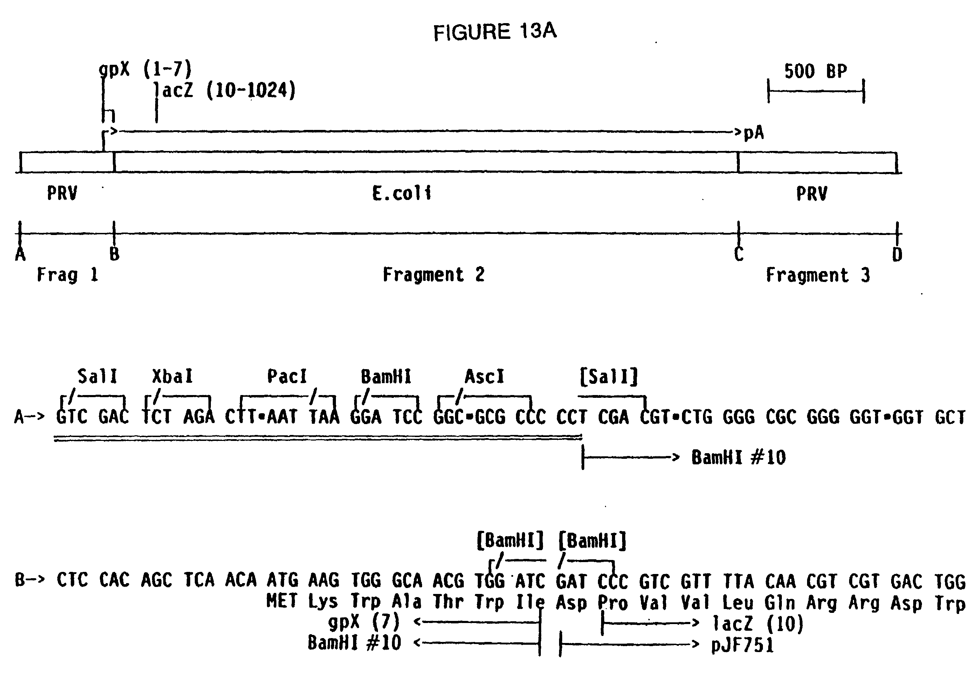

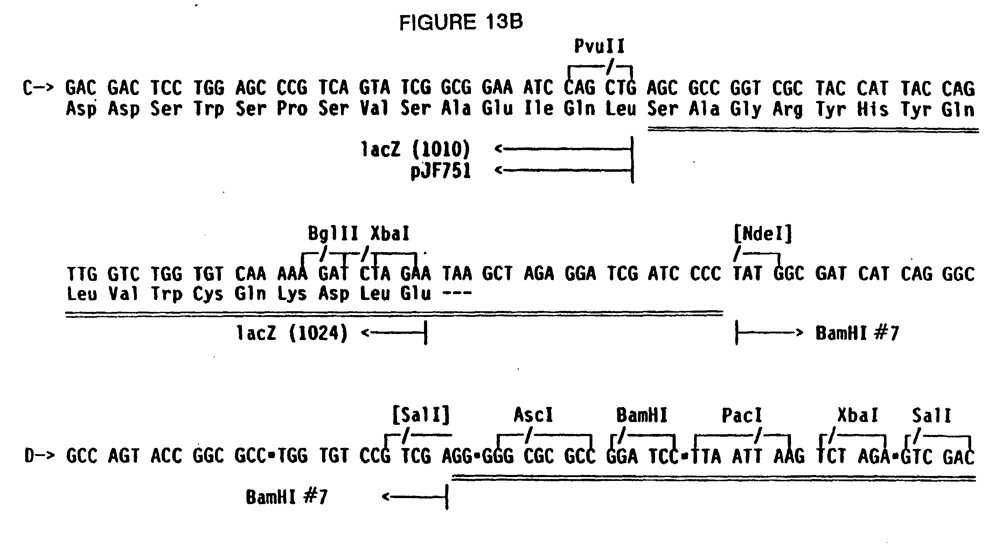

- FIGURE 13: Detailed description of the marker gene insertion in Homology Vector 593-31.2. The diagram shows the orientation of DNA fragments assembled in the marker gene. The origin of each fragment is described in the Materials and Methods section. The sequences located at the junctions between each fragment and at the ends of the marker gene are shown, including junction A (SEQ ID NO: 58), junction B (SEQ ID NO: 59), junction C (SEQ ID NO: 61), and junction D (SEQ ID NO: 63). The restriction sites used to generate each fragment are indicated at the appropriate junction. The location of the lacZ gene coding region is also given. The following two conventions are used: numbers in parenthesis () refer to amino acids, and restriction sites in brackets [] indicate the remnants of sites which were destroyed during construction. The following abbreviations are used, pseudorabies virus (PRV), lactose operon Z gene (lacZ), Escherichia coli (E.coli), poly adenylation signal (pA) and glycoprotein X (gpX).

The present invention provides a non-naturally occurring, recombinant equine herpesvirus. Thereby, this recombinant equine herpesvirus is of the species EHV-1 and EHV-4. More specifically, the present invention provides a live recombinant equine herpesvirus which comprises the genomic DNA of equine herpesvirus 1 (EHV-1) or 4 (EHV-4) from which a DNA sequence has been deleted from the US2 gene, shown for EHV-1 in SEQ ID NO: 1 and for EHV-4 in SEQ ID NO: 3 inactivating said US2 gene.

For purposes of this invention, the term "equine herpesvirus" includes the species EHV-1 and EHV-4, although other species would also be useful. These species were previously referred to in the literature as EHV-1, subtype 1 and EHV-1 subtype 2, respectively.

The invention thereby provides a recombinant equine herpesvirus wherein a DNA sequence which is not essential for replication of the virus has been deleted from the genomic DNA of the virus.

For purposes of this invention, "a DNA sequence which is not essential for replication of the virus" is a sequence located on the genome where it does not serve a necessary function for viral replication. Examples of necessary sequences include the following: complex protein binding sequences, sequences which code for reverse transcriptase or an essential glycoprotein, DNA sequences necessary for packaging, etc.

In the present invention the deleted sequence is deleted from the US2 gene of the virus. The present invention provides an example of such a reconbinant equine herpesvirus designated S-1EHV-002. The S-1EHV-002 virus has been deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852, U.S.A. under ATCC Accession No. VR 2358. The present invention further provides live recombinant equine herpesviruses as described above comprising at least one further deletion of a DNA sequence of the virus. This deleted DNA sequence is deleted from the gene which encodes the gpG glycoprotein, which encodes the gpE glycoprotein, from the thymidine kinase gene and/or from the gene encoding the large membrane glycoprotein (MGP). The present invention discloses a recombinant equine herpesvirus with a deletion in the TK gene designated S-1EHV-001. The S-1EHV-001 has been deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2357. A further recombinant equine herpesvirus with a deletion in the TK gene is designated S-4EHV-001. The S-4EHV-001 has been deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2361. However, it should be stressed that the above mentioned two specific herpesviruses are not part of the present invention.

Thus, the invention provides a recombinant equine herpesvirus with a deleted DNA sequence deleted from the thymidine kinase gene of the virus in addition to the delected DNA sequence deleted from the US2 gene of the virus. The present invention provides an example of such a recombinant equine herpesvirus designated S-1EHV-004. The S-1EHV-004 virus has been deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under with ATCC Accession No. VR 2360. The present invention provides an example of such a recombinant equine herpesvirus designated S-4EHV-002. The S-4EHV-002 virus has been deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2362. The present invention provides a further example of such a recombinant equine herpesviurs designated S-4EHV-023. The S-4EHV-023 has been deposited on August 5, 1993 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR

The invention also provides a recombinant equine herpesvirus with a deleted DNA sequence deleted from the thymidine kinase gene of the virus in addition to the deleted DNA sequence deleted from the US2 gene of the virus wherein a third DNA sequence which is not essential for the replication of the virus in deleted from the genomic DNA of the virus. An embodiment of this invention is a recombinant equine herpesvirus wherein the deleted third DNA sequence is deleted from the gpG gene of the virus. The present invention provides an example of such a recombinant equine herpesvirus designated S-1EHV-003. The S-1EHV-003 has been deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2359. A further embodiment of this invention is a recombinant equine herpesvirus wherein the deleted third DNA sequence is deleted from the gpE gene of the virus.

The present invention discloses isolated DNA encoding the US2 protein of the equine herpesvirus processed herein.

The present invention provides the above disclosed recombinant equine herpesvirus capable of replication which comprises viral DNA from a species of the naturally-occurring equine herpesvirus 1 or 4 in mutated form and foreign DNA encoding RNA which does not naturally occur in an animal into which the recombinant equine herpesvirus is introduced, the foreign DNA being inserted into the naturally-occurring equine herpesviral DNA at a site which is not essential for replication of the equine herpesvirus.

For purposes of this invention, "a recombinant equine herpesvirus capable of replication" is a live equine herpesvirus which has been generated by the reconbinant methods well known to those of skill in the art, e.g., the methods set forth in HOMOLOGOUS RECOMBINATION PROCEDURE FOR GENERATING RECOMBINANT EHV in Materials and Methods and has not had genetic material essential for the replication of the recombinant equine herpesvirus deleted.

For purposes of this invention, "an insertion site which is not essential for replication of the equine herpesvirus" is a location in the genome where a sequence of DNA is not necessary for viral replication. Examples of DNA sequences which are essential include the following: complex protein binding sequences, sequences which code for reverse transcriptase or an essential glycoprotein, DNA sequences necessary for packaging, etc.

The invention further discloses foreign DNA encoding RNA which encodes a polypeptide. Preferably, the polypeptide is antigenic in an animal into which the recombinant equine herpesvirus is introduced. In one embodiment of the polypeptide, the polypeptide is a detectable marker. Preferably, the polypeptide is E. coli β-galactosidase. Preferably, the polypeptide is E. coli β-glucuronidase. The present invention provides an example of such a recombinant equine herpesvirus designated S-4EHV-004.

For purposes of this invention, this antigenic polypeptide is a linear polymer of more than 10 amino acids linked by peptide bonds which stimulates the animal to produce antibodies.

In one embodiment of the polypeptide, the polypeptide is a polypeptide normally produced by an equine herpesvirus, a Streptococcus equi bacterium, an Infectious Anemic Virus, an equine influenza virus or an equine encephalitis virus. Preferably, the naturally occurring equine herpesvirus is EHV-1 and the foreign DNA is derived from EHV-4. Preferably, the naturally-occurring equine herpesvirus is EHV-4 and the foreign DNA is derived from EHV-1. Preferably, the foreign DNA encodes a gpB, gpC, gpD or gpH glycoprotein.

In one embodiment of the present invention, the naturally-occurring equine herpesvirus is EHV-4 and the antigenic polypeptide is or is from the gpD and gpB gene of the EHV-1 species of equine herpesvirus. The present invention provides an example of such a recombinant equine herpesvirus designated S-4EHV-010.

In another embodiment of the present invention, the naturally-occurring equine herpesvirus is EHV-4 and the antigenic polypeptide is or is from the hemagglutinin and neuraminidase genes of a subtype of equine influenza A virus. Preferably, the subtype of equine influenza A virus serotype is A1. Preferably, the subtype is further characterized as an isolate of the A1 subtype of equine influenza A virus. Preferably, the isolate is Influenza A/equine/Prague/56. The present invention provides an example of such a recombinant equine herpesvirus designated S-4EHV-011.

In another embodiment of the present invention, the subtype of equine influenza A virus is A2. Preferably, the subtype is further characterized as an isolate of the A2 subtype of equine influenza A virus. Preferably, the isolate is Influenza A/equine/Miami/63.
Preferably, the isolate is Influenza A/equine/Kentucky/81. Preferably, the isolate is Influenza A/equine/Alaska/91. The present invention provides examples of such recombinant equine herpesviruses designated S-4EHV-012, S-4EHV-013 and S-4ERV-014, respectively.

The present invention discloses a homology vector for producing a recombinant equine herpesvirus by inserting foreign DNA into a genome of an equine herpesvirus which comprises a double-stranded DNA molecule consisting essentially of: a) a double-stranded foreign DNA sequence encoding RNA which does not naturally occur in an animal into which the recombinant equine herpesvirus is introduced; b) at one end of the foreign DNA sequence, double-stranded equine herpesviral DNA homologous to genomic DNA located at one side of a site on the genome which is not essential for replication of the equine herpesvirus; and c) at the other end of the foreign DNA sequence, double-stranded equine herpesviral DNA homologous to genomic DNA located at the other side of the same site on the genome. In one embodiment of the homology vector, the equine herpesvirus is EHV-1.

In another embodiment of the homology vector, the equine herpesvirus is EHV-4. Thereby, the site on the genome which is not essential for replication is present within a DNA sequence included within the US2 gene and possibly additionally within the TK, gpG, gpE and/or MGP gene. In one embodiment of the homology vector, the double-stranded equine herpesviral DNA is homologous to genomic DNA present within the EHV-1 BglII restriction fragment b. Preferably, the double-stranded equine herpesviral DNA is homologous to a Sau3A restriction sub-fragment and a BstEII to PstI restriction sub-fragment. In another embodiment of the homology vector, the double-stranded equine herpesviral DNA is homologous to genomic DNA present within the EHV-1 BamHI restriction fragment n. Preferably, the double-stranded equine herpesviral DNA is homologous to genomic DNA present within the BamHI to NcoI restriction sub-fragment and the EcoRI to PstI restriction sub-fragment. In a further embodiment of the homology vector, the double-stranded equine herpesviral DNA is homologous to genomic DNA present within the EHV-1 EcoRI restriction fragment k. Preferably, the double-stranded equine herpesviral DNA is homologous to genomic DNA present within the EcoRI to PvuII restriction sub-fragment and the PstI to BamHI restriction sub-fragment. In another embodiment of the homology vector, the double-stranded equine herpesviral DNA is homologous to genomic DNA present within the EHV-4 BamHI restriction fragment c. Preferably, the double-stranded equine herpesviral DNA is homologous to genomic DNA present within the PvuII to FspI restriction sub-fragment and the PvuII to SmaI restriction sub-fragment. In a further embodiment of the homology vector, the double-stranded herpesviral DNA is homologous to genomic DNA present within the EHV-4 BamHI restriction fragment d. Preferably, the double-stranded herpesviral DNA is homologous to genomic DNA present within the XbaI to PstI restriction sub-fragment and the PstI to HindIII restriction sub-fragment. In another embodiment of the homology vector, the double-stranded herpesviral DNA is homologous to genomic DNA present within the EHV-4 EcoRI restriction fragment j. Preferably, the double-stranded herpesviral DNA is homologous to genomic DNA present within the EcoRI to AatII restriction sub-fragment and the FspI to FspI restriction sub-fragment.

The present invention also discloses a homology vector wherein the foreign DNA to be inserted corresponds to DNA encoding the gpH, gpB, gpD or gpC gene of an equine herpesvirus EHV-1 species. The present invention also describes a homology vector wherein the foreign DNA to be inserted corresponds to DNA encoding gpH, gpB, gpD or gpc glycoprotein of an equine herpesvirus EHV-4 species.

The present invention also discloses a vaccine which comprises an effective immunizing amount of the recombinant equine herpesvirus of the present invention and a suitable carrier.

Suitable carriers for the equine herpesvirus, which would be appropriate for use with the recombinant equine herpesviruses of the present invention, are well known in the art and include proteins, sugars, etc. One example of such a suitable carrier is a physiologically balanced culture medium containing one or more stabilizing-agents such as stabilized, hydrolyzed proteins, lactose, etc.

For purposes of this invention, an "effective immunizing amount" of the recombinant equine herpesvirus of the present invention is an amount necessary to stimulate the production of antibodies by the equine in which the virus was introduced in numbers sufficient to protect the equine from infection if it was confronted by a wild-type equine herpesvirus or other equine virus which the recombinant equine herpesvirus is directed to.

The present invention also discloses a method of immunizing an equine which comprises administering an effective immunizing dose of the vaccine disclosed in the present invention.

For purposes of this invention, the vaccine may be administered by any of the methods well known to those skilled in the art, for example, by intramuscular, subcutaneous, intraperitoneal or intravenous injection. Alternatively, the vaccine may be administered intranasally or orally.

The present invention also discloses for a method for testing an equine to determine whether the equine has been vaccinated with the vaccine disclosed in the present invention or is infected with a naturally-occurring equine herpesvirus which comprises: (a) obtaining from the equine to be tested a sample of a suitable body fluid: (b) detecting in the sample the presence of antibodies to equine herpesvirus, the absence of such antibodies indicating that the equine has been neither vaccinated nor infected; and (c) for the equine in which antibodies to equine herpesvirus are present, detecting in the sample the absence of antibodies to equine herpesviral antigens which are normally present in the body fluid of an equine infected by the naturally-occurring equine herpesvirus but which are not present in a vaccinated equine, the absence of such antibodies indicating that the equine was vaccinated and is not infected. In one embodiment of the method, the equine herpesviral antigen not present in the vaccinated equine is gpE glycoprotein.

The present invention discloses a method of producing the fetal-safe, live recombinant equine herpesvirus of the present invention which comprises treating viral DNA from a naturally-occurring live equine herpesvirus which comprises the genomic DNA of EHV-1 or -4 so as to delete from the virus DNA corresponding to the US2 region of the naturally-occurring equine herpesvirus.

The present invention also discloses a host cell infected with the recombinant equine herpesvirus of the present invention. In one embodiment, the host cell is a mammalian cell. Preferably, the mammalian cell is a vero cell.

For purposes of this invention, a "host cell" is a cell used to propagate a vector and its insert. Infecting the cells was accomplished by methods well known to those of skill in the art, for example, as set forth in INFECTION - TRANSFECTION PROCEDURE in Materials and Methods.

Methods for constructing, selecting and purifying recombinant equine herpesviruses are detailed below.

The following Examples do not only describe methods for producing the recombinant herpesviruses of the present invention with a deletion of DNA sequences from the US2 gene and possibly additionally from the TK, gpE, gpG and/or MGP gene, but also methods for producing recombinant herpesviruses with deletions in genes or combinations of genes not comprised by the present invention, however, which satisfy the purposes of the present invention. These Examples are not within the scope of the present invention. They are maintained for illustrative purposes and as a basis for understanding and performing the present invention.

PREPARATION OF EHV VIRUS STOCK SAMPLES. S-1EHV-000 and S-4EHV-000 are fresh isolates of EHV-1 and EHV-4, respectively, and were obtained from Dr. S. K. Dutta (College of Vetemary Medicine, University of Maryland, College Park, MD 20742). EHV virus stock samples were prepared by infecting Vero cells at a multiplicity of infection of 0.01 PFU/cell in Dulbecco's Modified Eagle Medium (DMEM) containing 2 mM glutamine, 100 units/ml penicillin, 100 units/ml streptomycin (these components were obtained from Irvine Scientific or equivalent supplier, and hereafter are referred to as complete DME medium) plus 1% fetal bovine serum. After cytopathic effect was complete, the medium and cells were harvested and the cells were pelleted at 3000 rpm for 5 minutes in a clinical centrifuge. Cells were resuspended in 1/10 the original volume of medium, and an equal volume of skim milk (9% skim milk powder in H₂O weight/volume) was added. The virus samples were frozen at -70°C. The titers were approximately 10⁸ PFU/ml for EHV-1 and approximately 10⁷ PFU/ml for EHV-4.

PREPARATION OF HERPESVIRUS DNA. For herpesvirus DNA preparation, a confluent monolayer of Vero cells in a 25 cm² flask or 60 mm petri dish was infected with 100 µl of virus sample. After overnight incubation, or when the cells were showing 100% cytopathic effect, the cells were scraped into the medium. The cells and medium were centrifuged at 3000 rpm for 5 minutes in a clinical centrifuge. The medium was decanted, and the cell pellet was gently resuspended in 0.5 ml solution containing 0.5% NONIDET P-40™ (octyl phenol ethylene oxide condensate containing an average of 9 moles of ethylene oxide per molecule) (NP-40, purchased from Sigma Chemical Co., St. Louis, MO.). The sample was incubated at room temperature for 10 minutes. Ten µl of a stock solution of RNase A (Sigma) were added (stock was 10 mg/ml, boiled for 10 minutes to inactivate DNAse). The sample was centrifuged to pellet nuclei. The DNA pellet was removed with a pasteur pipette or wooden stick and discarded. The supernatant fluid was decanted into a 1.5 ml Eppendorf tube containing 25 µl of 20% sodium dodecyl sulfate (Sigma) and 25 µl proteinase-K (10 mg/ml; Boehringer Mannheim). The sample was mixed and incubated at 37°C for 30-60 minutes. An equal volume of water-saturated phenol was added and the sample was mixed briefly. The sample was centrifuged in an Eppendorf minifuge for 5 minutes at full speed. The upper aqueous phase was removed to a new Eppendorf tube, and two volumes of absolute ethanol were added and the tube put at -20°C for 30 minutes to precipitate nucleic acid. The sample was centrifuged in an Eppendorf minifuge for 5 minutes. The supernatant was decanted, and the pellet was air dried and rehydrated in ∼16 µl H₂O. For the preparation of larger amounts of DNA, the procedure was scaled up to start with a 850 cm² roller bottle of Vero cells. The DNA was stored in 0.01 M tris pH 7.5, 1 mM EDTA at 4°C.

MOLECULAR BIOLOGICAL TECHNIQUES. Techniques for the manipulation of bacteria and DNA, including such procedures as digestion with restriction endonucleases, gel electrophoresis, extraction of DNA from gels, ligation, phosphorylation with kinase, treatment with phosphatase, growth of bacterial cultures, transformation of bacteria with DNA, and other molecular biological methods are described by Maniatis et al. (1982) and Sambrook et al. (1989). The polymerase chain reaction (PCR) was used to introduce restriction sites convenient for the manipulation of various DNAs. The procedures used are described by Innis et al (1990). In general, amplified fragments were less than 500 base pairs in size and critical regions of amplified fragments were confirmed by DNA sequencing. Except as noted, these techniques were used with minor variations.

LIGATION. DNA was joined together by the action of the enzyme T4 DNA ligase (BRL). Ligation reactions contained various amounts of DNA (from 0.2 to 20µg), 20mM Tris pH 7.5, 10mM MgCl₂, 10mM dithiothreitol (DTT), 200 µM ATP and 20 units T4 DNA ligase in 10-20 µl final reaction volume. The ligation proceeded for 3-16 hours at 15°C.

DNA SEQUENCING. Sequencing was performed using the USB Sequenase Kit and ³⁵S-dATP (NEN). Reactions using both the dGTP mixes and the dITP mixes were performed to clarify areas of compression. Alternatively, compressed areas were resolved on formamide gels. Templates were double-stranded plasmid subclones or single stranded M13 subclones, and primers were either made to the vector just outside the insert to be sequenced, or to previously obtained sequence. The sequence obtained was assembled and compared using Dnastar software. Manipulation and comparison of sequences obtained was performed with Superclone and Supersee programs from Coral Software.

SOUTHERN BLOTTING OF DNA. The general procedure for Southern blotting was taken from Maniatis et al. DNA was blotted to nitrocellulose filters and hybridized to appropriate labeled DNA probes. Probes for southern blots were prepared using either the Nonradioactive DNA Labeling and Detection Kit of Boehringer Mannheim or the nick translation kit of Bethesda Research Laboratories (BRL). In both cases the manufacturer's recommended procedures were followed.

DNA TRANSFECTION FOR GENERATING RECOMBINANT VIRUS. The method is based upon the calcium phosphate procedure of Graham and Van der eb (1973) with the following modifications. Virus and/or Plasmid DNA were diluted to 298 µl in 0.01 M Tris pH 7.5, 1mM EDTA. Forty µl 2M CaCl₂ was added followed by an equal volume of 2X HEPES buffered saline (10g N-2-hydroxyethyl piperazine N'-2-ethanesulfonic acid (HEPES), 16g NaCl, 0.74g KCl, 0.25g Na₂HPO₄·2H₂O, 2g dextrose per liter H₂O and buffered with NaOH to pH 7.4). The mixture was then incubated on ice for 10 minutes, and then added dropwise to an 80% confluent monolayer of Vero cells growing in a 60 mm petri dish under 5 ml of medium (DME plus 1% fetal bovine serum). The cells were incubated 4 hours at 37°C in a humidified incubator containing 5% CO₂. The cells were then washed once with 5 ml of 1XPBS (1.15g Na₂HPO₄, 0.2g KH₂PO₄, 0.89 NaCl, 0.2g KCl per liter H₂O), once with 5 ml of 20% glycerol/PBS (v/v), once more with 5 ml 1XPBS, and then fed with 5ml of medium (DME plus 2% fetal bovine serum). The cells were incubated at 37°C as above for 3-7 days until cytopathic effect from the virus was 50-100%. Virus was harvested as described above for the preparation of virus stocks. This stock was referred to as a transfection stock and was subsequently screened for recombinant virus by the SCREEN FOR RECOMBINANT HERPESVIRUS EXPRESSING ENZYMATIC MARKER GENES.

HOMOLOGOUS RECOMBINATION PROCEDURE FOR GENERATING RECOMBINANT HERPESVIRUS. This method relies upon the homologous recombination between herpesvirus DNA and plasmid homology vector DNA which occurs in tissue culture cells co-transfected with these elements. From 0.1-1.0 µg of plasmid DNA containing foreign DNA flanked by appropriate herpesvirus cloned sequences (the homology vector) were mixed with approximately 0.3µg of intact herpesvirus DNA. The DNAs were diluted to 298 µl in 0.01 M Tris pH 7.5, 1mM EDTA and transfected into Vero cells according to the DNA TRANSFECTION FOR GENERATING RECOMBINANT VIRUS (see above).

DIRECT LIGATION PROCEDURE FOR GENERATING RECOMBINANT HERPESVIRUS. Rather than using homology vectors and relying upon homologous recombination to generate recombinant virus, we have also developed the technique of direct ligation to engineer herpesviruses. In this instance, a cloned foreign gene did not require flanking herpesvirus DNA sequences but only required that it have restriction sites available to cut out the foreign gene fragment from the plasmid vector. A compatible restriction enzyme was used to cut herpesvirus DNA. A requirement of the technique is that the restriction enzyme used to cut the herpesvirus DNA must cut at a limited number of sites. For EHV-4 the restriction enzymes PmeI or PacI would be appropriate (see FIGURE 2). Restriction sites previously introduced into herpesviruses by other methods may also be used. The herpesvirus DNA is mixed with a 30-fold molar excess of plasmid DNA (typically 5µg of virus DNA to 10µg of plasmid DNA), and the mixture is cut with the appropriate restriction enzyme. The DNA mixture is phenol extracted and ethanol precipitated to remove restriction enzymes, and ligated together according to the ligation procedure detailed above. The ligated DNA mixture is then resuspended in 298 µl 0.01 M Tris pH 7.5, 1mM EDTA and transfected into Vero cells according to the DNA TRANSFECTION FOR GENERATING RECOMBINANT VIRUS (see above).

PROCEDURE FOR GENERATING RECOMBINANT HERPESVIRUS FROM SUBGENOMIC DNA FRAGMENTS. The ability to generate herpesviruses by cotransfection of cloned overlapping subgenomic fragments has been demonstrated for pseudorabies virus (48) and for herpesvirus of turkeys (47). If deletions and/or insertions are engineered directly into the subgenomic fragments prior to the cotransfection, this procedure results in a high frequency of viruses containing the genomic alteration, greatly reducing the amount of screening required to purify the recombinant virus. We anticipate utilizing this technique to engineer foreign gene insertions into specific attenuating deletions (US2, TK, and gpE) in EHV-4. In the first step of this procedure deletions are introduced into separate viruses via homologous recombination with enzymatic marker genes as described below. The homology vector used in this step is constructed such that the enzymatic marker gene is flanked by a restriction enzyme site that does not cut EHV-4 in the region of the DNA to be deleted. In the second step a library of overlapping subgenomic fragments, capable of regenerating wild-type virus, is constructed from randomly sheared 4EHV-000 DNA. In the third step subgenomic fragments are cloned from each of the individual recombinant viruses containing attenuating deletion/marker gene insertions, which were generated in the first step. In each case the subcloned fragment corresponds in size and location to one of the wild-type subgenomic fragments constructed in the second step. This is accomplished by screening a library of randomly sheared recombinant virus DNA subclones with probes generated from the ends of the appropriate wild-type subgenomic fragment. The restriction sites which had been engineered to flank the marker genes in the first step are now utilized to replace the marker genes in each subgenomic fragment with various foreign genes (such as 1EHV gpB, 1EHV gpD, equine influenza HA, or equine influenza NA). In the fourth step cotransfection of the appropriate overlapping wild type and deletion/insertion derived subgenomic fragments permits the generation of recombinant EHV-4 viruses incorporating any desired combination of deletions and/or insertions.

SCREEN FOR RECOMBINANT HERPESVIRUS EXPRESSING ENZYMATIC MARKER GENES. When the E.coli β-galactosidase (lacZ) or β-glucuronidase (uidA) marker gene was incorporated into a recombinant virus the plaques containing recombinants were visualized by a simple assay. The enzymatic substrate was incorporated (300 µg/ml) into the agarose overlay during the plaque assay. For the lacZ marker gene the substrate BLUOGAL™ (halogenated indolyl-β-D-galactosidase, GIBCO-Bethesda Research Labs) was used. For the uidA marker gene the substrate X-Glucuro Chx (5-bromo-4-chloro-3-indolyl-β-D-glucuronic acid Cyclohexylammonium salt, Biosynth AG) was used. Plaques that expressed active marker enzyme turned blue. The blue plaques were then picked onto fresh Vero cells and purified by further blue plaque isolation. In recombinant virus strategies in which the enzymatic marker gene is removed the assay involves plaque purifying white plaques from a background of parental blue plaques. In both cases viruses were typically purified with three rounds of plaque purification.

SELECTION OF ARA-T RESISTANT VIRUS. Many nucleoside analogs inhibit alpha-herpesvirus replication. One such antiviral drug is arabinosylthymine (Ara-T; Rayo Chemicals, Canada). Resistance of EHV mutants to Ara-T is due to mutations in the viral TK, so that TK negative (TK-) viruses are selected. The transfection stocks were grown on Vero cells in the presence of 200 µg/ml Ara-T in complete DME medium plus 1% fetal bovine serum. The selection was repeated one to two times. The virus stocks generated from Ara-T selection were assayed by thymidine plaque autoradiography (37, 38). Plaques picked from positive stocks were assayed for TK deletion by the SOUTHERN BLOTTING OF DNA procedure. Note that TK negative viruses constructed utilizing Ara-T selection (S-1EHV-001 and S-4EHV-001) exhibited changes in restriction fragments not related to the TK locus. Differences were observed in BamHI fragments c, d, and g in S-4EHV-001 and fragment p in S-1EHV-001. Since similar changes were not observed in S-4EHV-004 in which the TK deletion was introduced without Ara-T selection, we feel that this procedure is a less desirable procedure for the selection of recombinant viruses.

CONSTRUCTION OF DELETION VIRUSES. The strategy used to construct deletion viruses involved the use of either homologous recombination and/or direct ligation techniques. Initially a virus was constructed via homologous recombination, in which the DNA to be deleted was replaced with a marker gene such as E.coli β-galactosidase (lacZ) or β-glucuronidase (uidA). A second virus was then constructed in which the marker gene was deleted either by homologous recombination or via direct ligation. The advantage of this strategy is that both viruses may be purified by the SCREEN FOR RECOMBINANT HERPESVIRUS EXPRESSING ENZYMATIC MARKER GENES. The first virus is purified by picking blue plaques from a white plaque background, the second virus is purified by picking white plaques from a blue plaque background.

CLONING OF EQUINE INFLUENZA VIRUS HEMAGGLUTININ AND NEURAMINIDASE GENES. The equine influenza virus hemagglutinin (HA) and Neuraminidase (NA) genes may be cloned essentially as described by Katz et al. for the HA gene of human influenza virus. Viral RNA prepared from virus grown in MDBK cells is first converted to cDNA utilizing an oligo nucleotide primer specific for the target gene. The cDNA is then used as a template for PCR cloning (51) of the targeted gene region. The PCR primers are designed to incorporate restriction sites which permit the cloning of the amplified coding regions into vectors containing the appropriate signals for expression in EHV. One pair of oligo nucleotide primers will be required for each coding region. The HA gene coding regions from the serotype 2 (H3) viruses (Influenza A/equine/Miami/63, Influenza A/equine/Kentucky/81, and Influenza A/equine/Alaska/91) would be cloned utilizing the following primers 5'-GGGTCGACATGACAGACAACCATTATTTTGATAC-3' (SEQ ID NO: 64) for cDNA priming and combined with 5'-GGGTCGACTCAAATGCAAATGTTGCATCTGAT-3' (SEQ ID NO: 65) for PCR. The HA gene coding region from the serotype 1 (H7) virus (Influenza A/equine/Prague/56) would be cloned utilizing the following primers 5'-GGGATCCATGAACACTCAAATTCTAATATTAG-3' (SEQ ID NO: 66) for cDNA priming and combined with 5'-GGGATCCTTATATACAAATAGTGCACCGCA-3' (SEQ ID NO: 67) for PCR. The NA gene coding regions from the serotype 2 (N8) viruses (Influenza A/equine/Miami/63, Influenza A/equine/Kentucky/81, and Influenza A/equine/Alaska/91) would be cloned utilizing the following primers 5'-GGGTCGACATGAATCCAAATCAAAAGATAA-3' (SEQ ID NO: 68) for cDNA priming and combined with 5'-GGGTCGACTTACATCTTATCGATGTCAAA-3' (SEQ ID NO: 69) for PCR. The NA gene coding region from the serotype 1 (N7) virus (Influenza/A/equine/Prague/56) would be cloned utilizing the following primers 5'-GGGATCCATGAATCCTAATCAAAAACTCTTT-3' (SEQ ID NO: 68) for cDNA priming and combined with 5'-GGGATCCTTACGAAAAGTATTTAATTTGTGC-3' (SEQ ID NO: 71) for PCR. Note that this general strategy may be used to clone the coding regions of HA and NA genes from other strains of equine influenza A virus.

HOMOLOGY VECTOR 450-46.B4. The plasmid 450-46.B4 was constructed for the purpose of deleting a portion of the EHV-1 thymidine kinase gene. It may also be used to insert foreign DNA into EHV1. It contains a unique XbaI restriction enzyme site into which foreign DNA may be inserted. It may be constructed utilizing standard recombinant DNA techniques (23 and 34), by joining restriction fragments from the following sources with the synthetic DNA sequences indicated in figure 4. The plasmid vector is derived from an approximately 2978 base pair BamHI to HindIII restriction fragment of pSP65 (Promega). Fragment 1 is an approximately 779 base pair Sau3A restriction sub-fragment of the EHV1 BglII restriction fragment b (42). Fragment 2 is an approximately 1504 base pair BstEII to PstI restriction sub-fragment of EHV1 BglII restriction fragment b (42).

HOMOLOGY VECTOR 467-21.19. The plasmid 467-21.19 was constructed for the purpose of deleting a portion of the EHV1 unique short 2 gene. It may also be used to insert foreign DNA into EHV1. It contains a unique EcoRI restriction enzyme site into which foreign DNA may be inserted. It may be constructed utilizing standard recombinant DNA techniques (23, 34) by joining restriction fragments from the following sources as indicated in FIGURE 5. The plasmid vector is derived from an approximately 2983 base pair BamHI to PstI restriction fragment of pSP65 (Promega). Note that the EcoRI site has been removed from the plasmid vector by nuclease S1 digestion. Fragment 1 is an approximately 767 base pair BamHI to NcoI restriction sub-fragment of the EHV1 BamHI restriction fragment n (42). Fragment 2 is an approximately 1283 base pair EcoRI to PstI restriction sub-fragment of EHV1 BamHI restriction fragment n (42).

HOMOLOGY VECTOR 536-85.30. The plasmid 536-85.30 was constructed for the purpose of deleting the EHV1 glycoprotein G gene. It was used to insert foreign DNA into EHV1. It contains a pair of SalI restriction enzyme sites into which foreign DNA may be inserted. It may be constructed utilizing standard recombinant DNA techniques (23 and 34) by joining restriction fragments from the following sources as indicated in FIGURE 6. The plasmid vector is derived from an approximately 2643 base pair EcoRI to PstI restriction fragment of pNEB193 (New England Biolabs). Fragment 1 is an approximately 2292 base pair EcoRI to PvuII restriction sub-fragment of the EHV1 EcoRI restriction fragment k (42). Fragment 2 is an approximately 1077 base pair PstI to BamHI restriction sub-fragment of EHV1 EcoRI restriction fragment k (42).

HOMOLOGY VECTOR 495-61.39. The plasmid 495-61.39 was constructed for the purpose of deleting a portion of the EHV-4 thymidine kinase gene. It may also be used to insert foreign DNA into EHV-4. It contains a unique XbaI restriction enzyme site into which foreign DNA may be inserted. It may be constructed utilizing standard recombinant DNA techniques (23, 34) by joining restriction fragments from the following sources with the synthetic DNA sequences indicated in FIGURE 7. The plasmid vector is derived from an approximately 2988 base pair SmaI to HincII restriction fragment of pSP65 (Promega). Fragment 1 is an approximately 830 base pair PvuII to FspI restriction sub-fragment of the EHV-4 BamHI restriction fragment c (8). Fragment 2 is an approximately 1220 base pair PvuII to SmaI restriction sub-fragment of EHV-4 BamHI restriction fragment c (8).

HOMOLOGY VECTOR 523-38.9. The plasmid 523-38.9 was constructed for the purpose of deleting a portion of the EHV4 unique short 2 gene. It may also be used to insert foreign DNA into EHV4. It contains a unique PstI restriction enzyme site into which foreign DNA may be inserted. It may be constructed utilizing standard recombinant DNA techniques (23, 34) by joining restriction fragments from the following sources as indicated in FIGURE 8. The plasmid vector is derived from an approximately 2984 base pair XbaI to HindIII restriction fragment of pSP65 (Promega). Fragment 1 is an approximately 1098 base pair XbaI to PstI restriction sub-fragment of the EHV4 EcoRI restriction fragment g (8). Fragment 2 is an approximately 2799 base pair PstI to HindIII restriction sub-fragment of EHV4 BamHI restriction fragment d (8).

HOMOLOGY VECTOR 580-57.25. The plasmid 580-57.25 was constructed for the purpose of deleting the EHV4 gpE gene. It may also be used to insert foreign DNA into EHV4. It contains a unique BamHI restriction enzyme site into which foreign DNA may be inserted. It may be constructed utilizing standard recombinant DNA techniques (23, 34), by joining restriction fragments from the following sources as indicated in figure 9. The plasmid vector is derived from an approximately 2973 base pair EcoRI to HincII restriction fragment of pSP65 (Promega). Fragment 1 is an approximately 2046 base pair EcoRI to AatII restriction sub-fragment of the EHV4 EcoRI restriction fragment j (8). Fragment 2 is an approximately 1976 base pair FspI to FspI restriction sub-fragment of EHV4 EcoRI restriction fragment j (8).

HOMOLOGY VECTOR 467-22.A12. The plasmid 467-22.A12 was constructed for the purpose of deleting a portion of the US2 gene coding region from the EHV-1 virus. It incorporates an E.coli β-galactosidase (lacZ) marker gene flanked by EHV-1 virus DNA. The lacZ marker gene was inserted into the homology vector 467-21.19 at the unique EcoRI site. The marker gene is oriented opposite to the US2 gene in the homology vector. A detailed description of the marker gene is given in FIGURE 10. It may be constructed utilizing standard recombinant DNA techniques (23, 34) by joining restriction fragments from the following sources with the synthetic DNA sequences indicated in FIGURE 10. Fragment 1 is an approximately 413 base pair SalI to BamHI restriction sub-fragment of the PRV BamHI restriction fragment 10 (22). Fragment 2 is an approximately 3010 base pair BamHI to PvuII restriction fragment of plasmid pJF751 (11). Fragment 3 is an approximately 754 base pair NdeI to SalI restriction sub-fragment of the PRV BamHI restriction fragment #7 (22).

HOMOLOGY VECTOR 588-81.13. The plasmid 588-81.13 was constructed for the purpose of deleting a portion of the US2 gene coding region from the EHV-4 virus. It incorporates an E.coli β-galactosidase (lacZ) marker gene flanked by EHV-4 virus DNA. A lacZ marker gene was inserted as a PstI restriction fragment into the homology vector 523-38.9 at the unique PstI site. The marker gene is oriented in the opposite direction to the US2 gene in the homology vector. A detailed description of the marker gene is given in FIGURE 11. It was constructed utilizing standard recombinant DNA techniques (23, 34) by joining restriction fragments from the following sources with the synthetic DNA sequences indicated in FIGURE 11. Fragment 1 is an approximately 413 base pair SalI to BamHI restriction sub-fragment of the PRV BamHI restriction fragment 10 (22). Fragment 2 is an approximately 3010 base pair BamHI to PvuII restriction fragment of plasmid pJF751 (11). Fragment 3 is an approximately 754 base pair NdeI to SalI restriction sub-fragment of the PRV BamHI restriction fragment #7 (22).

HOMOLOGY VECTOR 552-45.19. The plasmid 552-45.19 was constructed for the purpose of deleting a portion of the TK gene coding region from the EHV-4 virus. It incorporates an E.coli β-glucuronidase (uidA) marker gene flanked by EHV-4 virus DNA. The uidA marker gene was inserted into the homology vector 495-61.39 at the unique XbaI site. The marker gene is oriented opposite to the TK gene in the homology vector. A detailed description of the marker gene is given in FIGURE 12. It may be constructed utilizing standard recombinant DNA techniques (23, 34) by joining restriction fragments from the following sources with the synthetic DNA sequences indicated in Figure 12. Fragment 1 is an approximately 404 base pair SalI to EcoRI restriction sub-fragment of the PRV BamHI restriction fragment #10 (22). Note that the EcoRI site was introduced at the loction indicated in FIGURE 12 by PCR cloning. Fragment 2 is an approximately 1823 base pair EcoRI to SmaI fragment of the plasmid pRAJ260. (Clonetech). Note that the EcoRI and SmaI sites were introduced at the locations indicated in figure 12 by PCR cloning. Fragment 3 is an approximately 784 base pair SmaI to SmaI restriction sub-fragment of the HSV-1 BamHI restriction fragment Q (24). Note that this fragment is oriented such that the polyadenylation sequence (AATAAA) is located closest to junction C.

HOMOLOGY VECTOR 593-31.2. The plasmid 593-31.2 was constructed for the purpose of deleting the gpE gene coding region from the EHV-4 virus. It incorporates an E.coli β-galactosidase (lacZ) marker gene flanked by EHV-4 virus DNA. The lacZ marker gene was inserted into the homology vector 580-57.25 at the unique BamHI site. The marker gene is oriented the same as the deleted gpE gene in the homology vector. A detailed description of the marker gene is given in FIGURE 13. It may be constructed utilizing standard recombinant DNA techniques (23, 34) by joining restriction fragments from the following sources with the synthetic DNA sequences indicated in figure 10. Fragment 1 is an approximately 413 base pair SalI to BamHI restriction sub-fragment of the PRV BamHI restriction fragment 10 (22). Fragment 2 is an approximately 3010 base pair BamHI to PvuII restriction fragment of plasmid pJF751 (11). Fragment 3 is an approximately 754 base pair NdeI to SalI restriction sub-fragment of the PRV BamHI restriction fragment #7 (22).

HOMOLOGY VECTOR 616-40. The plasmid 616-40 was constructed for the purpose of deleting a portion of the EHV-4 thymidine kinase gene. It is also used to insert foreign DNA into EHV-4. It contains a unique NotI site into which foreign DNA is inserted. The homology vector 616-40 is derived from a cosmid library made of sheared DNA from virus 4EHV-004. A library of subclones containing overlapping EHV subgenomic fragments was generated as follows. 4EHV-004 DNA was sheared and then size selected on a glycerol gradient as described (48) with 40-50 kb fragments chosen as the insert population. The pooled fractions were diluted twofold with TE, one-tenth volume of 3M NaAc and 2.5 volumes of ethanol were added, and the DNA was precipitated at 30,000 rpm in a Beckman SW41 rotor for 1 hr. The sheared fragments were polished to give blunt ends by initial treatment with T4 DNA polymerase, using low dNTP concentrations to promote 3' overhang removal, followed by treatment with Klenow polymerase to fill in recessed 3' ends. These insert fragments were then ligated to 384-94. Cosmid vector 384-94 is a derivative of pHC79 from Gibco BRL, Inc. from which the tetracycline resistance gene was deleted by restriction endonuclease digestion with HindIII and AvaI, and a DNA linker containing the NotI-BamHI-NotI restriction sited was inserted. Cosmid vector 384-94 was digested with BamHI, made blunt by treatment with Klenow polymerase and treated with calf intestinal phosphatase. The ligation mixture containing cosmid vector 384-94 and 4EHV-004 genomic DNA fragments was then packaged using Gigapack XL packaging extracts (Stratagene). Ligation and packaging were as recommended by the manufacturer. Colonies were grown in overnight cultures and cosmid DNA was extracted (23,34). Cosmid DNA was analyzed by restriction endonuclease digestion with NotI. The cosmid DNA clones were screened for the presence of a 3.0 kb *Not*I fragment indicating the presence of the PRV gX promoter-uidA foreign gene insert into a *Not*I site within the TK gene deletion. One cosmid, 607-21.16, containing the TK gene deletion with an insertion of the uidA gene was isolated. The cosmid, 607-21.16, was digested with *Not*I to remove the gX promoter/uidA gene and religated to obtain the homology vector, 616-40. The homology vector, digested with NotI to remove the gX promoter/uidA gene and religated to obtain the homology vector, 616-40. The homology vector, 616-40, contains DNA sequences surrounding the TK gene of approximately 22,600 base pairs which includes approximately 4000 base pairs of EcoRI e fragment, approximately 600 base pairs of the entire EcoRI q fragment and approximately 18,000 base pairs of the EcoRI a fragment. The vector is derived from an approximately 4,430 base pair BamHI restriction fragment from cosmid vector, 384-94 (derived from pHC79 Gibco-BRL). Homology vector 616-40 contains the 653 base pair deletion in the TK gene with a unique NotI site and no additional marker gene inserted.

HOMOLOGY VECTOR 593-20.5. The plasmid 593-20.5 was constructed for the purpose of deleting the EHV4 gpE gene and inserting the *B*-glucuronidase (uidA) marker gene under the control of the PRV gX promoter. It is also used to insert other foreign DNA including the equine influenza HA and NA genes into EHV4. It was constructed using standard recombinant DNA techniques (23, 34), by joining restriction fragments from the following sources. The plasmid is derived from an approximately 2973 base pair EcoRI to HincII restriction fragment of pSP65 (Promega). Fragment 1 is an approximately 2046 base pair EcoRI to AatII restriction subfragment of the EHV4 EcoRI restriction fragment j (8). Fragment 2 is an approximately 3011 base pair BamHI fragment containing the PRV gX promoter, uidA gene, and the HSV-1 polyadenylation site. Fragment 3 is an approximately 1976 base pair FspI to FspI restriction sub-fragment of EHV4 EcoRI restriction fragment j.

### EXAMPLES

### EXAMPLE 1

### UNIQUE SHORT 2 GENE

The deletion of the US2 gene in an Equine herpesvirus renders a recombinant equine herpesvirus safe for use in pregnant equines, that is, it renders the virus incapable of causing abortion of the fetus.

We have characterized the unique short regions of EHV-1 and EHV-4 by DNA sequence analysis. SEQ ID NO: 1 shows the sequence of the first 1322 bases of the BamHI fragment n (see FIGURE 1) reading away from the BamHI n - BamHI d junction. This sequence contains a 303 amino acid ORF which exhibits homology to several other herpesvirus US2 genes (see FIGURE 3). SEQ ID NO: 3 shows the 1252 bases of sequence which starts 198 bases upstream of the HindIII site located approximately in the middle of the EHV-4 EcoRI g fragment (see FIGURE 2). The sequence reads back toward the EcoRI g - EcoRI b junction and contains a 324 amino acid ORF. After we sequenced the unique short region, we found that it contained a US2 gene with homology to several other herpesvirus US2 genes (see FIGURE 5). Since we determined the location and sequence of the US2 gene in the equine herpes virus, we can delete the US2 gene of EHV-1 and EHV-4 and attenuate as well as render the virus safe for use in pregnant horses.

### EXAMPLE 2

### HOMOLOGY VECTOR 450-46.B4

The homology vector 450-46.B4 is a plasmid used for attenuating EHV-1 via inactivation of the TK gene. Inactivation of the TK gene is accomplished by a deletion of DNA which encodes Tk from EHV-1. Plasmid 450-46.B4 carries a copy of the TK gene (31) into which an approximately 202 bp deletion between amino acids 115 and' 182 has been introduced. The plasmid, used according to the HOMOLOGOUS RECOMBINATION PROCEDURE FOR GENERATING RECOMBINANT HERPESVIRUS and the SELECTION OF ARA-T RESISTANT VIRUS, generates an EHV-1 containing a deleted TK gene.

Plasmid 450-46.B4 is also useful for inserting foreign DNA into EHV-1. The plasmid contains a unique XbaI restriction site located at the site of the deletion. Foreign DNA cloned into this site results in a plasmid which should be used according to the HOMOLOGOUS RECOMBINATION PROCEDURE FOR GENERATING RECOMBINANT HERPESVIRUS to generate an EHV-1 containing the foreign DNA. Note that if an appropriate marker gene (e.g. E.coli lacZ) is inserted into the homology vector, then a recombinant virus is generated without the SELECTION OF ARA-T RESISTANT VIRUS.

For the procedures described above to be successful, it is important that the deletion/insertion site be in a region non-essential to the replication of the EHV-1 and that the site be flanked with equine herpesvirus DNA appropriate for mediating homologous recombination between virus and plasmid DNAs. Note that the deletion was designed so that it is limited to a specific portion of the TK coding region. This region contains amino acids important for TK enzymatic activity. The deletion does not remove sequences that are involved with flanking genes which are important for efficient viral growth (12). We have demonstrated that the insertion/deletion site in homology vector 450-46.B4 inserts foreign DNA into EHV-1 as represented by the two recombinant EHV-1 viruses in EXAMPLES 7 and 9.

### EXAMPLE 3

### HOMOLOGY VECTOR 467-21.19

The homology vector 467-21.19 is a plasmid used for attenuating EHV-1 via inactivation of the US2 gene. Inactivation of the US2 gene is accomplished by deletion of US2 encoding DNA from EHV-1. Plasmid 467-21.19 carries a copy of the US2 gene into which an approximately 93 bp deletion between amino acids 174 and 205 has been introduced. The plasmid should be used according to the CONSTRUCTION OF DELETION VIRUSES to generate an EHV-1 containing a deleted US2 gene.

Plasmid 467-21.19 is also useful for the insertion of foreign DNA into EHV-1. The plasmid contains a unique EcoRI restriction site located at the site of the deletion. Foreign DNA cloned into this site results in a plasmid which should be used according to the HOMOLOGOUS RECOMBINATION PROCEDURE FOR GENERATING RECOMBINANT HERPESVIRUS to generate an EHV-1 containing foreign DNA.

For the procedures described above to be successful, it is important that the deletion/insertion site be in a region non-essential to the replication of the EHV-1 and that the site be flanked with equine herpesvirus DNA appropriate for mediating homologous recombination between virus and plasmid DNAs. Note that the deletion was designed so that it is limited to the unique short region and does not remove sequences from the internal or terminal repeats. We have demonstrated that the insertion/deletion site in homology vector 467-21.19 inserts foreign DNA into EHV-1 as represented by the two recombinant EHV-1 viruses in EXAMPLES 7 and 9.

### EXAMPLE 4

### HOMOLOGY VECTOR 536-85.30

The homology vector 536-85.30 is a plasmid used for attenuating EHV-1 by removing the glycoprotein G (gpG) gene and a portion of the unique short region large membrane glycoprotein (MGP) gene. Plasmid 536-85.30 carries a portion of the unique short region into which a deletion of approximately 2384 base pairs which removes the entire gpG coding region and the N-terminal 307 amino acids of the MGP has been engineered. The plasmid may be used according to the CONSTRUCTION OF DELETION VIRUSES to generate a gpG/MGP deleted EHV-1.

Plasmid 536-85.30 is also useful for the insertion of foreign DNA into EHV-1. The plasmid contains a pair of SalI restriction sites located at the site of the deletion. Foreign DNA cloned into these sites results in a plasmid which should be used according to the HOMOLOGOUS RECOMBINATION PROCEDURE FOR GENERATING RECOMBINANT HERPESVIRUS to generate an EHV-1 containing foreign DNA.

### EXAMPLE 5

### HOMOLOGY VECTOR 495-61.39

The homology vector 495-61.39 is a plasmid used for attenuating EHV-4 via inactivation of the TK gene. Inactivation of the TK gene is accomplished by deletion of DNA which encodes Tk from EHV-4. Plasmid 495-61.39 carries a copy of the TK gene (27) into which an approximately 653 bp deletion between amino acids 98 and 317 has been engineered. The plasmid is used according to the HOMOLOGOUS RECOMBINATION PROCEDURE FOR GENERATING RECOMBINANT HERPESVIRUS and the SELECTION OF ARA-T RESISTANT VIRUS to generate an EHV-4 with a deletion of the gene which encodes Tk.

Plasmid 495-61.39 is also useful for the insertion of foreign DNA into EHV-4. The plasmid contains a unique XbaI restriction site located at the site of the deletion. Foreign DNA cloned into this site results in a plasmid which should be used according to the HOMOLOGOUS RECOMBINATION PROCEDURE FOR GENERATING RECOMBINANT HERPESVIRUS to generate an EHV-4 virus containing foreign DNA. Note that if an appropriate marker gene (e.g. E.coli lacZ) is inserted into the homology vector, then a recombinant virus is generated without the SELECTION OF ARA-T RESISTANT VIRUS.

For the procedures described above to be successful, it is important that the deletion/insertion site be in a region non-essential to the replication of the EHV-4 and that the site be flanked with equine herpesvirus DNA appropriate for mediating homologous recombination between virus and plasmid DNAs. Note that the deletion was designed so that it is limited to a specific portion of the TK coding region. This region contains amino acids important for TK enzymatic activity. The deletion does not remove sequences that are involved with flanking genes which are important for efficient viral growth (18, 12).

### EXAMPLE 6

### HOMOLOGY VECTOR 523-38.9

The homology vector 523-38.9 is a plasmid used for attenuating EHV-4 via inactivation of the US2 gene. Inactivation of the US2 gene is accomplished by deletion DNA which encodes US2 from EHV-4. Plasmid 523-38.9 carries a copy of the US2 gene into which an approximately 711 bp deletion between amino acids 131 and 324 has been engineered. The plasmid should be used according to the CONSTRUCTION OF DELETION VIRUSES to generate an EHV-4 with a deletion of the gene which encodes US2.

Plasmid 523-38.9 is also useful for the insertion of foreign DNA into EHV-4. The plasmid contains a unique PstI restriction site located at the site of the deletion. Foreign DNA cloned into this site results in a plasmid which should be used according to the HOMOLOGOUS RECOMBINATION PROCEDURE FOR GENERATING RECOMBINANT HERPESVIRUS to generate an EHV-4 containing foreign DNA.

For the procedures described above to be successful, it is important that the deletion/insertion site be in a region non-essential to the replication of the EHV-4 and that the site be flanked with equine herpesvirus DNA appropriate for mediating homologous recombination between virus and plasmid DNAs. Note that the deletion was designed so that it is limited to the unique short region and does not remove sequences from the internal or terminal repeats. We have demonstrated that the insertion/deletion site in homology vector 523-38.9 inserts foreign DNA into EHV-4 as represented by the two recombinant EHV-4 viruses in EXAMPLES 13 and 14.

### EXAMPLE 7

### HOMOLOGY VECTOR 580-57.25

We have determined that the deletion of the glycoprotein E gene from the equine herpesvirus is useful in attenuating the virus for use in a vaccine for horses and for providing a negative serological marker.

The homology vector 580-57.25 is a plasmid used to attenuate EHV-4 by removing the glycoprotein E (gpE) gene (8 and SEQ ID NOS: 5 & 6). Plasmid 580-57.25 carries a portion of the unique short region into which a deletion of approximately 1694 base pairs, which removes the entire gpE coding region, has been engineered. The plasmid may be used according to the CONSTRUCTION OF DELETION VIRUSES to generate an EHV-4 virus with a deletion of the gene which encodes gpE.

Plasmid 580-57.25 is also useful for the insertion of foreign DNA into EHV-4. The plasmid contains a unique BamHI restriction site located at the site of the deletion. Foreign DNA cloned into this site results in a plasmid which should be used according to the HOMOLOGOUS RECOMBINATION PROCEDURE FOR GENERATING RECOMBINANT HERPESVIRUS to generate an EHV-4 containing foreign DNA.

### EXAMPLE 8

### PREPARATION OF RECOMBINANT EQUINE HERPESVIRUS DESIGNATED S-1EHV-001

S-1EHV-001 is an equine herpesvirus type 1 (EHV-1) virus that has an approximately 202 base pair deletion in the TK gene. The S-1EHV-001 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2357.

S-1EHV-001 was derived from S-1EHV-000 (Dutta strain). This was accomplished utilizing the homology vector 450-46.B4 (see Materials and Methods) and virus S-1EHV-000 in the HOMOLOGOUS RECOMBINATION PROCEDURE FOR GENERATING RECOMBINANT HERPESVIRUS. The transfection stock was selected according to the SELECTION OF ARA-T RESISTANT VIRUS. Individual clones were picked after two rounds of selection and assayed by thymidine plaque autoradiography (37, 38). Plaques picked from TK negative stocks were assayed for TK deletion by the SOUTHERN BLOTTING OF DNA procedure. A plaque which was TK minus by both the thymidine incorporation assay and the southern analysis was chosen and designated S-1EHV-001.

The construction of this virus establishes the EHV-1 thymidine kinase gene as a non-essential gene and a viable site for the insertion of foreign DNA. This virus is useful because the inactivation of the TK gene attenuates the virus.

### EXAMPLE 9

### PREPARATION OF RECOMBINANT EQUINE HERPESVIRUS DESIGNATED S-1EHV-002

S-1EHV-002 is an equine herpesvirus type 1 (EHV-1) virus that has two deletions in the short unique region of the genome. The first deletion is approximately 93 base pairs and removes amino acids 174 to 205 of the US2 gene (SEQ ID NO: 1). The second deletion is approximately 2283 base pairs and removes portions of the gpG and MGP genes from the unique short region. The gene for E.coli β-galactosidase (lacZ gene) was inserted into the deletion in the US2 gene and is under the control of the PRV gpX promoter. The S-1EHV-002 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2358.

S-1EHV-002 was derived from S-1EHV-000 (Dutta strain). This was accomplished utilizing the homology vector 467-22.A12 (see Materials and Methods) and virus S-1EHV-000 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES. The final result of blue plaque purification was the recombinant virus designated S-1EHV-002. This virus was characterized by restriction mapping and the SOUTHERN BLOTTING DNA procedure. This analysis confirmed the insertion of the β-galactosidase (lacZ) marker gene and the deletion of approximately 93 base pairs of the US2 gene. To characterize the second unique short region deletion, the deleted EcoRI k fragment from S-1EHV-002 was subcloned and subjected to DNA sequence analysis. This analysis confirmed a deletion which begins with amino acid 14 of the gpG gene and continues through amino acid 303 of the MGP gene. The deletion occurred such that the remaining 13 amino acids of the gpG gene are in frame with the remaining 494 amino acids of the MGP gene.

The construction of this virus establishes the EHV-1 US2 and gpG genes as non-essential genes and are viable sites for the insertion of foreign DNA. This virus is useful because inactivation of the US2 gene attenuates the virus and the deletion of the glycoprotein G gene from this virus provides a negative serological marker for differentiating it from wild type EHV-1.

### EXAMPLE 10

### PREPARATION OF RECOMBINANT EQUINE HERPESVIRUS DESIGNATED S-1EHV-003

S-1EHV-003 is an equine herpesvirus type 1 (EHV-1) virus that has two deletions in the short unique region and one deletion in the unique long region of the genome. The first deletion is an approximately 202 base pair deletion in the TK gene. The second deletion is approximately 93 base pairs and removes nucleic acids 174 to 205 of the US2 gene (SEQ ID NO: 1). The third deletion is approximately 2283 base pairs and removes portions of the gpG and MGP genes from the unique short region. The gene for E.coli β-galactosidase (lacZ gene) was inserted into the deletion in the US2 gene and is under the control of the PRV gpX promoter. The S-1EHV-003 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2359.

S-1EHV-003 was derived from S-1EHV-002 (see EXAMPLE 9). This was accomplished utilizing the homology vector 450-46.B4 (see Materials and Methods) and virus S-1EHV-002 in the HOMOLOGOUS RECOMBINATION PROCEDURE FOR GENERATING RECOMBINANT HERPESVIRUS. The transfection stock was selected according to the SELECTION OF ARA-T RESISTANT IBR VIRUS. Individual clones were picked after two rounds of selection and assayed by thymidine plaque autoradiography (37, 38). Plaques picked from TK negative stocks were assayed for TK deletion by the SOUTHERN BLOTTING OF DNA procedure. A plaque which was TK minus by both the thymidine incorporation assay and the southern analysis was chosen and designated S-1EHV-003.

The construction of this virus establishes that multiple deletions inactivating the TK and US2 genes and removing the gpG genes can be made in a single EHV-1 virus. This virus is useful because the inactivation of the TK and US2 genes attenuates the virus and the deletion of the region which encodes glycoprotein G from this virus provides a negative serological marker for differentiating it from wild type EHV-1.

### EXAMPLE 11

### PREPARATION OF RECOMBINANT EQUINE HERPESVIRUS DESIGNATED S-1EHV-004

S-1EHV-004 is an equine herpesvirus type 1 (EHV-1) virus that has one deletion in the long unique region and one deletion in the short unique region of the genome. The first deletion is an approximately 202 base pair deletion in the TK gene. The second deletion is approximately 93 base pairs and removes DNA encoding nucleic acids 174 to 205 of the US2 gene (SEQ ID NO: 1). The gene for E.coli β-galactosidase (lacZ gene) was inserted into the deletion in the US2 gene and is under the control of the PRV gpX promoter. The S-1EHV-004 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2360.

S-1EHV-004 was derived from S-1EHV-001 (see EXAMPLE 8). This was accomplished utilizing the homology vector 467-22.A12 (see Materials and Methods) and virus S-1EHV-001 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES. The final result of blue plaque purification was the recombinant virus designated S-1EHV-004. This virus was characterized by restriction mapping and the SOUTHERN BLOTTING DNA procedure. This analysis confirmed the insertion of the β-galactosidase (lacZ) marker gene, the deletion of approximately 93 base pairs of the US2 gene, and the approximately 202 base pair deletion of the TK gene.

The construction of this virus establishes that the EHV-1 US2 and TK genes are non-essential and are viable sites for the insertion of foreign DNA. This virus is useful because the inactivation of the TK and US2 genes attenuates the virus.

### EXAMPLE 12

### PREPARATION OF RECOMBINANT EQUINE HERPESVIRUS DESIGNATED S-4EHV-001

S-4EHV-001 is an equine herpesvirus type 4 (EHV-4) virus that has an approximately 202 base pair deletion in the TK gene. The S-4EHV-001 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2361.

S-4EHV-001 was derived from S-4EHV-000 (Dutta strain). This was accomplished utilizing the homology vector 450-46.B4 (see Materials and Methods) and virus S-4EHV-000 in the HOMOLOGOUS RECOMBINATION PROCEDURE FOR GENERATING RECOMBINANT HERPESVIRUS. The transfection stock was selected according to the SELECTION OF ARA-T RESISTANT IBR VIRUS. Individual clones were picked after two rounds of selection and analyzed by the SOUTHERN BLOTTING OF DNA procedure. A plaque which was TK minus by the southern analysis was chosen and designated S-4EHV-001.

The construction of this virus establishes the EHV-4 thymidine kinase gene as a non-essential gene and a viable site for the insertion of foreign DNA. This virus is useful because the inactivation of the TK gene attenuates the virus. The construction of this virus also demonstrates that a homology vector derived from EHV-1 can engineer EHV-4 in an analogous manner.

### EXAMPLE 13

### PREPARATION OF RECOMBINANT EQUINE HERPESVIRUS DESIGNATED S-4EHV-002

S-4EHV-002 is an equine herpesvirus type 4 (EHV-4) virus that has one deletion in the long unique region and one deletion in the short unique region of the genome. The first deletion is an approximately 202 base pair deletion in the TK gene. The second deletion is approximately 705 base pairs and removes amino acids 131 to 324 of the US2 gene (SEQ ID NO: 3). The gene for E.coli β-galactosidase (lacZ gene) was inserted into the deletion in the US2 gene and is under the control of the PRV gpX promoter. The S-4EHV-002 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2362.

S-4EHV-002 was derived from S-4EHV-001 (see EXAMPLE 12). This was accomplished utilizing the homology vector 523-42.A18 (see Materials and Methods) and virus S-4EHV-001 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES. The final result of blue plaque purification was the recombinant virus designated S-4EHV-002. This virus was characterized by restriction mapping and the SOUTHERN BLOTTING DNA procedure. This analysis confirmed the insertion of the β-galactosidase (lacZ) marker gene, the deletion of approximately 705 base pairs of the US2 gene, and the approximately 202 base pair deletion of the TK gene.

The construction of this virus establishes the EHV-4 US2 and TK genes as non-essential genes and as viable sites for the insertion of foreign DNA. This virus is useful because the inactivation of the TK and US2 genes attenuates the virus.

### EXAMPLE 14

### PREPARATION OF RECOMBINANT EQUINE HERPESVIRUS DESIGNATED S-4EHV-003

S-4EHV-003 is an equine herpesvirus type 4 (EHV-4) virus that has one deletion in the short unique region of the genome. The deletion is approximately 705 base pairs and removes amino acids 131 to 324 of the US2 gene (SEQ ID NO: 3). The gene for E.coli β-galactosidase (lacZ gene) was inserted into the deletion in the US2 gene and is under the control of the PRV gpX promoter. The S-4EHV-003 equine herpesvirus was deposited on March 12, 1992 pursuant to the Budapest Treaty on the International Deposit of Microorganisms for the Purposes of Patent Procedure with the Patent Culture Depository of the American Type Culture Collection, 12301 Parklawn Drive, Rockville, Maryland 20852 U.S.A. under ATCC Accession No. VR 2363.

S-4EHV-003 was derived from S-4EHV-000 (Dutta strain). This was accomplished utilizing the homology vector 523-42.A18 (see Materials and Methods) and virus S-4EHV-000 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES. The final result of blue plaque purification was the recombinant virus designated S-4EHV-003. This virus was characterized by restriction mapping and the SOUTHERN BLOTTING DNA procedure. This analysis confirmed the insertion of the β-galactosidase (lacZ) marker gene and the deletion of approximately 705 base pairs of the US2 gene.

The construction of this virus establishes the EHV-4 US2 gene as non-essential and as a viable site for the insertion of foreign DNA. This virus is useful because the inactivation of the US2 gene attenuates the virus.

### EXAMPLE 15

### PREPARATION OF RECOMBINANT EQUINE HERPESVIRUS DESIGNATED S-4EHV-004

S-4EHV-004 is an equine herpesvirus type 4 (EHV-4) virus that has a deletion of approximately 653 base pairs between amino acids 98 and 317 of the thymidine kinase gene (28). The gene for E.coli β-glucuronidase (uidA gene) was inserted into the deletion in the TK gene and is under the control of the PRV gpX promoter.

S-4EHV-004 was derived from S-4EHV-000 (Dutta strain). This was accomplished utilizing the homology vector 552-45.19 (see Materials and Methods) and virus S-4EHV-000 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES. The final result of blue plaque purification was the recombinant virus designated S-4EHV-004. This virus was characterized by restriction mapping and the SOUTHERN BLOTTING DNA procedure. This analysis confirmed the insertion of the β-glucuronidase (uidA) marker gene and the deletion of approximately 653 base pairs of the TK gene.

The construction of this virus establishes that the EHV-4 TK gene is non-essential and is a viable site for the insertion of foreign DNA. This virus is useful because the inactivation of the TK gene attenuates the virus.

### EXAMPLE 16

### PREPARATION OF RECOMBINANT EQUINE HERPESVIRUS DESIGNATED S-4EHV-010

Recombinant EHV-4 viruses expressing glycoproteins from EHV-1 are utilized in vaccines to protect against infection by both EHV-1 and EHV-4. Similarly, recombinant EHV-1 viruses expressing EHV-4 glycoproteins are utilized in vaccines to protect against infection by both EHV-1 and EHV-4.

S-4EHV-010, a recombinant EHV-4 with deletions in the TK, US2, and gpE genes and with insertions of the genes for EHV-1 gpD and gpB in place of the TK and gpE genes, respectively, is constructed in the following manner. S-4EHV-010 is derived from S-4EHV-004 (see EXAMPLE 15) through the construction of four intermediate viruses. The first intermediate virus, S-4EHV-005, was constructed similarly to S-4EHV-003, utilizing the homology vector 588-81.13 (see Materials and Methods) and virus S-4EHV-004 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES for a blue plaque recombinant virus (lacZ substrate). The resulting virus has deletions of the TK and US2 genes and insertions of uidA and lacZ in the TK and US2 gene deletions, respectively. The second intermediate virus S-4EHV-006, was constructed, utilizing the homology vector 523-38.9 (see Materials and Methods) and virus S-4EHV-005 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES for a white plaque recombinant virus (lacZ substrate). The resulting virus has deletions of the TK and US2 genes and an insertion of uidA gene in the TK gene deletion. The third intermediate virus, S-4EHV-007, is constructed, utilizing the homology vector 593-31.2 (see Materials and Methods) and virus S-4EHV-006 in the HOMOLOGOUS RECOMBINATION PROCEDURE FOR GENERATING RECOMBINANT HERPESVIRUS. The transfection stock is screened by the SCREEN FOR RECOMBINANT HERPESVIRUS EXPRESSING ENZYMATIC MARKER GENES for a blue plaque recombinant virus (lacZ substrate). The resulting virus has deletions of the TK, US2, and gpE genes and insertions of the uidA and lacZ genes in the TK and gpE gene deletions, respectively. The fourth intermediate virus S-4EHV-009, is constructed, utilizing the homology vector 580-57.25, into which the EHV-1 gpB gene had been inserted, and virus S-4EHV-007 in the HOMOLOGOUS RECOMBINATION PROCEDURE FOR GENERATING RECOMBINANT HERPESVIRUS. Note that the EHV-1 gpB gene is cloned as an approximately 3665 bp FspI to ClaI sub-fragment of an approximately 5100 bp PstI fragment of EHV-1 (43). The transfection stock is screened by the SCREEN FOR RECOMBINANT HERPESVIRUS EXPRESSING ENZYMATIC MARKER GENES for a white plaque recombinant virus (lacZ substrate). The resulting virus has deletions of the TK, US2, and gpE genes and insertion of the uidA and EHV-1 gpB genes in the TK and gpE gene deletions, respectively. Finally, S-4EHV-010 is constructed, utilizing the homology vector 495-61.39, into which the EHV-1 gpD gene is inserted, and virus S-4EHV-009 in the HOMOLOGOUS RECOMBINATION PROCEDURE FOR GENERATING RECOMBINANT HERPESVIRUS. Note that the EHV-1 gpD gene is cloned as an approximately 1929 bp SmaI to EcoRV sub-fragment of the approximately 10,500 bp BamHI D fragment of EHV-1 (1). The transfection stock is screened by the SCREEN FOR RECOMBINANT HERPESVIRUS EXPRESSING ENZYMATIC MARKER GENES for a white plaque recombinant virus (uidA substrate). This virus is utilized in a vaccine to protect horses from infection with EHV-1 and EHV-4. The deletion of the glycoprotein E gene from this virus provides a negative serological marker for differentiating it from wild type EHV-1 and EHV-4.

### EXAMPLE 17

### PREPARATION OF RECOMBINANT EQUINE HERPESVIRUS DESIGNATED S-4EHV-011

Recombinant poxviruses encoding the hemagglutinin (HA) and the neuraminidase genes (NA) from influenza viruses have been reported to mediate protective immunity against infection with the homologous influenza virus (5, 44). Delivery of the HA and NA antigens from several subtypes of equine influenza virus via recombinant EHV viruses is utilized to provide protective immunity against equine influenza virus in addition to equine herpesvirus.

S-4EHV-011, a recombinant EHV-4 with deletions in the TK, US2, and gpE genes and with the genes for Influenza A/equine/Prague/56 hemagglutinin and neuraminidase of the isolate of equine influenza inserted in place of the gpE gene is constructed in the following manner. S-4EHV-011 is derived from S-4EHV-023 through the construction of an intermediate virus. S-4EHV-023 was constructed utilizing homology vector 616-40 (see Materials and Methods) and virus S-4EHV-006 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES for a white plaque recombinant virus (uidA substrate). The intermediate virus, S-4EHV-008, was constructed utilizing the homology vector 593-20.5 (see Materials and Methods) and virus S-4EHV-023 in the HOMOLOGOUS RECOMBINATION PROCEDURE FOR GENERATING RECOMBINANT HERPESVIRUS. The transfection stock was screened by the SCREEN FOR RECOMBINANT HERPESVIRUS EXPRESSING ENZYMATIC MARKER GENES for a blue plaque recombinant virus (uidA substrate). The resulting virus has deletions in the TK, US2, and gpE genes and an insertion of uidA in the gpE gene deletion. Finally S-4EHV-011 is constructed, utilizing the homology vector 580-57.25, into which the hemagglutinin and neuraminidase genes of the Influenza A/equine/Prague/56 isolate of equine influenza were inserted, and virus S-4EHV-008 in the HOMOLOGOUS RECOMBINATION PROCEDURE FOR GENERATING RECOMBINANT HERPESVIRUS. Note that the influenza virus genes were cloned using the techniques described in the Materials and Methods section. The hemagglutinin gene was placed under the control of the HCMV immediate early promoter and the neuraminidase gene was placed under the control of the PRV gpX promoter. The transfection stock is screened by the SCREEN FOR RECOMBINANT HERPESVIRUS EXPRESSING ENZYMATIC MARKER GENES for a white plaque recombinant virus (uidA substrate). This virus is utilized in vaccines to protect horses from infection with EHV-4 and equine influenza virus. An effective vaccine requires antigens from several different influenza strains. This is accomplished by construction of multiple recombinant viruses expressing HA and NA from several different influenza strains (see EXAMPLES 18-20). A more efficacious vaccine is formulated by mixing this recombinant virus with those described in EXAMPLES 18-20.

### EXAMPLE 18

### PREPARATION OF RECOMBINANT EQUINE HERPESVIRUS DESIGNATED S-4EHV-012

S-4EHV-012, a recombinant EHV-4 with deletions in the TK, US2, and gpE genes and the genes for hemagglutinin and neuraminidase of the isolate of Influenza A/equine/Miami/63 equine influenza inserted in place of the gpE gene is constructed in the following manner. S-4EHV-012 is derived from S-4EHV-023 (see EXAMPLE 16) through the construction of an intermediate virus. The intermediate virus, S-4EHV-008, was constructed as described in EXAMPLE 17. S-4EHV-012 is constructed, utilizing the homology vector 580-57.25, into which the hemagglutinin and neuraminidase genes of the Influenza A/equine/Miami/63 isolate of equine influenza are inserted, and virus S-4EHV-008 in the HOMOLOGOUS RECOMBINATION PROCEDURE FOR GENERATING RECOMBINANT HERPESVIRUS. Note that the influenza virus genes were cloned using the techniques described in the Materials and Methods section. The hemagglutinin gene was placed under the control of the HCMV immediate early promoter and the neuraminidase gene was placed under the control of the PRV gpX promoter. The transfection stock is screened by the SCREEN FOR RECOMBINANT HERPESVIRUS EXPRESSING ENZYMATIC MARKER GENES for a white plaque recombinant virus (uidA substrate). This virus is utilized in a vaccine to protect horses from infection by EHV-4 and equine influenza virus. A more efficacious vaccine is formulated by mixing this recombinant virus with those described here and in EXAMPLES 17, 19 and 20.

### EXAMPLE 19

### PREPARATION OF RECOMBINANT EQUINE HERPESVIRUS DESIGNATED S-4EHV-013

S-4EHV-013, a recombinant EHV-4 with deletions in the TK, US2, and gpE genes and the genes for hemagglutinin and neuraminidase of the Influenza A/equine/Kentucky/81 isolate of equine influenza inserted in place of the gpE gene is constructed in the following manner. S-4EHV-013 is derived from S-4EHV-023 (see EXAMPLE 16) through the construction of an intermediate virus. The intermediate virus, S-4EHV-008, was constructed as described in EXAMPLE 17. S-4EHV-013 is constructed, utilizing the homology vector 580-57.25, into which the hemagglutinin and neuraminidase genes of the Influenza A/equine/Kentucky/81 isolate of equine influenza is inserted, and virus S-4EHV-008 in the HOMOLOGOUS RECOMBINATION PROCEDURE FOR GENERATING RECOMBINANT HERPESVIRUS. Note that the influenza virus genes were cloned using the techniques described in the Materials and Methods section. The hemagglutinin gene was placed under the control of the HCMV immediate early promoter and the neuraminidase gene was placed under the control of the PRV gpX promoter. The transfection stock is screened by the SCREEN FOR RECOMBINANT HERPESVIRUS EXPRESSING ENZYMATIC MARKER GENES for a white plaque recombinant virus (uidA substrate). This virus is utilized in a vaccine to protect horses from infection by EHV-4 and equine influenza virus. A more efficacious vaccine is formulated by mixing this recombinant virus with those described here and in EXAMPLES 17, 18 and 20.

### EXAMPLE 20

### PREPARATION OF RECOMBINANT EQUINE HERPESVIRUS DESIGNATED S-4EHV-014

S-4EHV-014, a recombinant EHV-4 with deletions in the TK, US2, and gpE genes and the genes for hemagglutinin and neuraminidase of the Influenza A/equine/Alaska/91 isolate of equine influenza inserted in place of the gpE gene is constructed in the following manner. S-4EHV-014 is derived from S-4EHV-023 (see EXAMPLE 17) through the construction of an intermediate virus. The intermediate virus, S-4EHV-008, was constructed as described in EXAMPLE 17. S-4EHV-014 is constructed, utilizing the homology vector 580-57.25, into which the hemagglutinin and neuraminidase genes of the Influenza A/equine/Alaska/91 isolate of equine influenza were inserted, and virus S-4EHV-008 in the HOMOLOGOUS RECOMBINATION PROCEDURE FOR GENERATING RECOMBINANT HERPESVIRUS. Note that the influenza virus genes were cloned using the techniques described in the Materials and Methods section. The hemagglutinin gene was placed under the control of the HCMV immediate early promoter and the neuraminidase gene was placed under the control of PRV gpX promoter. The transfection stock is screened by the SCREEN FOR RECOMBINANT HERPESVIRUS EXPRESSING ENZYMATIC MARKER GENES for a white plaque recombinant virus (uidA substrate). This virus is useful as a vaccine to protect horses from infection by EHV-4 and equine influenza virus. A more efficacious vaccine is formulated by mixing this recombinant virus with those described here and in EXAMPLES 17, 18 and 19.

### EXAMPLE 21

### VACCINES UTILIZING EHV TO EXPRESS ANTIGENS FROM VARIOUS DISEASE CAUSING MICROORGANISMS

### STREPTOCOCCUS EQUI

The M protein (14) has been shown to play an important role in the immune response to Streptococcus equi, the causative agent of the severe respiratory disease Strangles. Delivery of this antigen via a recombinant EHV virus would result in strong protective immunity without the post-vaccinal sequelae that often accompany whole culture and protein extracted Streptococcus equi bacterins. It is contemplated that the procedures that have been used to express the marker genes (lacZ and uidA) in S-1EHV-002, S-1EHV-003, S-1EHV-004, S-4EHV-002, S-4EHV-003, and S-4EHV-004 and which are disclosed herein are applicable to the expression of this and other potential Streptococcus equi antigens.

Antigens from the following microorganisms are utilized to develop equine vaccines: equine infectious anemia virus, equine encephalitis virus, equine rhinovirus, equine rotavirus, equine viral arteritis, rabies, equine adenovirus pneumonia, African horse sickness, equine coital exanthema, equine papillomatosis, equine cytomegalovirus, leptospirosis, tetanus, anthrax, colibacillosis, salmonellosis, pasteurellosis, Ehrlichia risticii, brucella-associated disease, actinomycosis, Taylorella equigenitolia, and mycoplasma-associated disease.

### EXAMPLE 22:

### REGENERATION OF S-4EHV-004 FROM CLONED SUBGENOMIC FRAGMENTS WITH HELPER WILD TYPE VIRAL DNA FRAGMENTS:

The protocol was used to generate a recombinant equine herpesvirus by combining EHV genomic fragments cloned into cosmids and genomic fragments of wild type helper virus containing less than one plaque forming unit. The presence of wild type EHV genomic DNA in the transfection mixture increases the efficiency of obtaining a recombinant equine herpesvirus. Overlapping subgenomic fragments were cloned from 4EHV-000 (wild type) and 4EHV-004 viral DNA. DNA from cosmid subclones of 4EHV-000 and 4EHV-004 was digested with the appropriate restriction endonucleases to release the inserts from the cosmid vector. Transfection with an appropriate mixture of these five fragments covering the entire EHV genome and very low concentrations of wild type viral DNA (less than one plaque-forming unit) resulted in 4EHV-004 virus production. One hundred percent of the viruses in the cotransfection stock were recombinant viruses carrying the uidA gene.

### REFERENCES

1. J. Audonnet, et al., Journal of General Virology **71**, 2969-2978 (1990).
2. T. Ben-Porat et al., Virology **154,** 325-334 (1986).
3. R. A. Bhat, et al., Nucleic Acids Research **17,** 1159-1176 (1989)
4. J. L. Cantello, et al., Journal of Virology **65,** 1584-1588 (1991).
5. T. M. Chambers, et al., Virology **167,** 414-421 (1988).
6. C. F. Colle III, et al., Virology **188,** 545-557 (1992).
7. M. L. Cook & J. G. Stevens, Journal of General Virology **31,** 75-80 (1976).
8. A. A. Cullinane, et al., Journal of General Virology **69,** 1575-1590 (1988).
9. R. C. Desrosiers et al., Molecular and Cellular Biology **5,** 2796-2803 (1985).
10. S. J. Edwards, et al., Plasmodium falciparum antigens in recombinant HSV-1, Technological Advances in Vaccine Development, pp. 223-234, Alan Riss, Inc. (1988).
11. F. A. Ferrari, et al., Journal of Bacteriology **161,** 556-562 (1985).
12. A. Forrester, et al., Journal of Virology **66,** 314-348 (1992).
13. K. Fulcuchi et al., Proc. Natl. Acad. Sci. U.S.A. **82,** 751-754 (1985).
14. J. E. Galan and J.F. Timoney, Infection and Immunity **55,** 3181-3187 (1987).
15. F. L. Grahm and A. Van der Eb., Virolgy **52,** 556-567 (1973).
16. R. W. Honess, Journal of General Virology **65,** 2077-2107 (1984).
17. D. R. Hustead, Large Animal Veterinarian **46** (2), March/April, 23-24, (1991).
18. J. G. Jacobson, et al., Journal of Virology **63,** 1839-1843, (1089).
19. S. Joshi, et al., Journal of Virology **65,** 5524-5530 (1991).
20. Kit et al., Proceedings of the 94th Annual Meeting of the United States Animal Health Association, pp. 66-75 (1990).
21. J. M. Koomey et al., Journal of Virology **50,** 662-665 (1984).
22. B. Lomniczi et al., Journal of Virology **49,** 970-979 (1984).
23. T. Maniatis, et al., Molecular Cloning: A Laboratory Manual, Cold Spring Harbor, N.Y. (1982).
24. D. J. McGeoch, et al., Journal of Molecular Biology **181,** 1-13 (1985).
25. D. J. McGeoch, et al., Journal of General Virology **68,** 19-38 (1987).
26. D. J. McGeoch, et al., Journal of General Virology **69,** 1531-1574 (1988).
27. L. Nicolson, et al., Journal of General Virology **71,** 1801-1805 (1990).
28. L. Nicolson, et al., Virology **179,** 378-387 (1990).
29. R. W. Price and A. Kahn, Infection and Immunity, **34,** 571-580 (1981).
30. M. P. Riggio, et al., Journal of Virology **63,** 1123-1133 (1989).
31. G. R. Robertson and J.M. Whalley, Nucleic Acids Research **16**, 11303-11317 (1988).
32. B. Roizman, et al., Cold Spring Harbor Conference on New Approaches to Viral Vaccines (September 1983).
33. B. Roizman, et al., Archives of Virology **123,** 425-449 (1992).
34. J. Sambrook, et al., Molecular Cloning: A Laboratory Manual Second Edition, Cold Spring Harbor Press, Cold Spring Harbor, N.Y. (1989).
35. M. Shih, et al., Proceedings of the National Academy of Sciences U.S.A. **81,** 5867-5870 (1984).
36. R. R. Spaete and E.S. Mocarski, Proceedings of the National Academy of Sciences U.S.A. **84,** 7213-7217 (1987).
37. R. B. Tenser, et al., Journal of General Virology **64,** 1369-1373 (1983).
38. R. B. Tenser, et al., Journal of Clinical Microbiology **17,** 122-127 (1983).
39. R. L. Thompson et al., Virology **131,** 180-192 (1983).
40. D. R. Thomson, et al., Gene **57,** 261-265 (1987).
41. M. Wachsnan, et al., Journal of General Virology **70,** 2513-2520 (1989).
42. J. M. Whalley, et al., Journal of General Virology **57,** 307-323 (1981).
43. J. M. Whalley, et al., Journal of General Virology **70,** 383-394 (1989).
44. R. G. Webster, et al., Virology **164,** 230-237 (1988).
45. J. P. Weir and P.R. Narayanan, Nucleic Acids Research **16,** 10267-10282 (1988).
46. M. E. Whealy, et al., Journal of Virology **62,** 4185-4194 (1988).
47. M. A. Wild, et al., 15th International Herpesvirus Workshop, Abstract No. 122, Washington, D.C. (1990).
48. M. Zijil, et al., Journal of Virology **62,** 2191-2195 (1988).
49. M. Zijil, et al., Journal of Virology **71,** 1747-1755 (1990).
50. F. Zuckerman et al., Vaccination and Control of Aujesky's Disease, pp. 107-117 Ed. J. van Oirschot, Kluwer, London (1989).
51. M. A. Innis, et al., PCR Protocols; A Guide To Methods And Applications, pp. 84-91, Academic Press, Inc., San Diego, CA (1990).
52. Katz et al., Journal of Virology, **64,** 1808-1811 (1990).
53. WO 92/01045

## Claims

1. A live recombinant equine herpesvirus which comprises the genomic DNA of equine herpesvirus 1 (EHV-1) or 4 (EHV-4) from which a DNA sequence has been deleted from the US2 gene, shown for EHV-1 in SEQ ID NO: 1 and for EHV-4 in SEQ ID NO: 3 inactivating said US2 gene.

2. The herpesvirus of claim 1 comprising at least one further deletion of a DNA sequence.

3. The herpesvirus of claim 2, wherein the DNA sequence is deleted from the gene encoding the thymidine kinase (TK), from the gene encoding glycoprotein E (gpE), from the gene encoding glycoprotein G (gpG), and/or from the gene encoding the large membrane glycoprotein (MGP).

4. The herpesvirus of claim 1-3 which further contains a foreign DNA sequence inserted therein.

5. The herpesvirus of claim 4 wherein the foreign DNA sequence encodes a detectable marker.

6. The herpesvirus of claim 5 wherein the detectable marker is β-galactosidase.

7. The herpesvirus of claim 4 wherein the foreign DNA is from an equine influenza virus.

8. The herpesyirus of claim 4, wherein the foreign DNA is from a gene encoding equine herpesvirus 1 (EHV-1) glycoprotein B (gpB) or EHV-1 glycoprotein D(gpD)

9. The herpesvirus of claim 7 wherein the foreign DNA is from the hemagglutinin and neuraminidase genes of the Influenza A/equine/Prague/56 isolate of equine influenza virus.

10. The herpesvirus of claim 7, wherein the foreign DNA is from the hemagglutinin and neuraminidase genes of the Influenza A/equine/Miarni/63 isolate of equine/influenza virus.

11. The live recombinant herpesvirus of claim 7 wherein the foreign DNA is from the hemagglutinin and neuraminidase genes of the Influenza A/equine/Kentucky/81 isolate of equine influenza virus.

12. The equine herpesvirus of claim 7, wherein the foreign DNA is from the hemagglutinin and neuraminidase genes of the Influenza A/equine/Alaska/91 isolate of equine influenza virus.

13. The herpesvirus of claim 4 having a deletion in the US2 gene and having a DNA sequence encoding *E. coli* β-galactosidase inserted in place of the US2 gene deletion.

14. The herpesvirus of claim 13 also having a deletion in the gene encoding TK.

15. The herpesvirus of claim 13 wherein the gpG gene and a portion of the MGP gene are also deleted.

16. The herpesvirus of claim 14 wherein the gpG gene and a portion of the MGP gene are also deleted.

17. The herpesvirus of claim 3 having a deletion in the gene encoding TK and in the gene encoding gpE.

18. The herpesvirus of claim 17 having a DNA sequence encoding the EHV1 gpB inserted in place of the gpE deletion and EHV1 gpD inserted in place of the TK deletion.

19. The herpesvirus of claim 17 having a DNA sequence of the hemagglutinin and neuraminidase gene of an equine influenza virus inserted in place of the gpE deletion.

20. The herpesvirus of claim 19 wherein the hemagglutinin neuraminidase genes are from the Influenza A/equine/Prague/56 isolate of equine influenza virus.

21. The herpesvirus of claim 19 wherein the hemagglutinin neuraminidase genes are from the Influenza A/equine/Miami/63 isolate of equine/influenza virus.

22. The herpes-virus of claim 19 wherein the hemagglutinin neuraminidase genes are from the Influenza A/equine/Kentucky/81 isolate of equine influenza virus.

23. The herpesvirus of claim 19 wherein the hemagglutinin neuraminidase genes are from the Influenza A/equine/Alaska/91 isolate of equine influenza virus.

24. The herpesvirus of claim 13 designated by the ATCC Accession No. VR 2363 (S-4EHV-003).

25. The herpesvirus of claim 14 designated by the ATCC Accession No. VR 2362 (S-4EHV-002).

26. The herpesvirus of claim 14 designated by the ATCC Accession No. VR 2360 (S-1EHV-004).

27. The herpesvirus of claim 15 designated by the ATCC Accession No. VR 2358 (S-1EHV-002).

28. The herpesvirus of claim 16 designated by the ATCC Accession No. VR 2359 (S-1EHV-003).

29. The herpesvirus of claim 18 designated S-4EHV-010 constructed as described in Example 16.

30. The herpesvirus of claim 20 designated S-4EHV-011 constructed as described in Example 17.

31. The herpesvirus of claim 21 designated S-4EHV-012 constructed as described in Example 18.

32. The herpesvirus of claim 22 designated S-4EHV-013 constructed as described in Example 19.

33. The herpesvirus of claim 23 designated S-4EHV-014 constructed as described in Example 20.

## Patentansprüche

1. Lebendes rekombinantes Pferde-Herpesvirus, das die genomische DNA des Pferde-Herpesvirus 1 (EHV-1) oder 4 (EHV-4) umfasst, von der eine DNA-Sequenz des US2-Gens deletiert wurde, gezeigt für EHV-1 in SEQ ID Nr: 1 und für EHV-4 in SEQ ID Nr. 3, wobei das US2-Gen inaktiviert wird.

2. Herpesvirus nach Anspruch 1, umfassend mindestens eine weitere Deletion einer DNA-Sequenz.

3. Herpesvirus nach Anspruch 2, worin die DNA-Sequenz aus dem Gen deletiert wurde, das die Thymidinkinase (TK) codiert, aus dem Gen, das Glykoprotein E (gpE) codiert, aus dem Gen, das Glykoprotein G (gpG) codiert, und/oder aus dem Gen, das das große Membranglykoprotein (MGP) codiert.

4. Herpesvirus nach Anspruch 1 bis 3, das weiterhin darin eingefügt eine fremde DNA-Sequenz enhält.

5. Herpesvirus nach Anspruch 4, worin die fremde DNA-Sequenz einen nachweisbaren Marker codiert.

6. Herpesvirus nach Anspruch 5, worin der nachweisbare Marker β-Galactosidase ist.

7. Herpesvirus nach Anspruch 4, worin die fremde DNA von einem Pferdeinfluenzavirus ist.

8. Herpesvirus nach Anspruch 4, worin die fremde DNA von einem Gen ist, das Pferde-Herpesvirus 1 (EHV-1)-Glykoprotein B (gpB) oder EHV-1-Glykoprotein D (gpD) codiert.

9. Herpesvirus nach Anspruch 7, worin die fremde DNA von den Hämagglutinin- und Neuraminidase-Genen des Influenza A/Pferde/Prag/56-Isolats des Pferde-Influenzavirus ist.

10. Herpesvirus nach Anspruch 7, worin die fremde DNA von den Hämagglutinin- und Neuraminidase-Genen des Influenza A/Pferde/Miami/63-lsolats des Pferde-Influenzavirus ist.

11. Lebendes rekombinantes Herpesvirus nach Anspruch 7, worin die fremde DNA von den Hämagglutinin- und Neuraminidase-Genen des Influenza A/Pferde/Kentucky/81-lsolats des Pferde-Influenzavirus ist.

12. Pferde-Herpesvirus nach Anspruch 7, worin die fremde DNA von den Hämagglutinin- und Neuraminidase-Genen des Influenza A/Pferde/Alaska/91-lsolats des Pferde-Influenzavirus ist.

13. Herpesvirus nach Anspruch 4, das eine Deletion im US2-Gen hat und eine in der Position der US2-Gen-Deletion eingefügte DNA-Sequenz aufweist, die E. coli-β-Galactosidase codiert.

14. Herpesvirus nach Anspruch 13, das auch eine Deletion in dem Gen aufweist, das TK codiert.

15. Herpesvirus nach Anspruch 13, worin das gpG-Gen und ein Teil des MGP-Gens ebenfalls deletiert ist.

16. Herpesvirus nach Anspruch 14, worin das gpG-Gen und ein Teil des MGP-Gens ebenfalls deletiert ist.

17. Herpesvirus nach Anspruch 3, das eine Deletion in dem Gen aufweist, das TK codiert, und in dem Gen, das gpE codiert.

18. Herpesvirus nach Anspruch 17, mit einer an der Stelle der gpE-Deletion eingefügten DNA-Sequenz, die EHV1-gpB codiert, und einer an der Stelle der TK-Deletion eingefügten DNA-Sequenz, die EHV1-gpD codiert.

19. Herpesvirus nach Anspruch 17, mit einer an der Stelle der gpE-Deletion eingefügten DNA-Sequenz des Hämagglutinin- und Neuraminidase-Gens eines Pferde-Influenzavirus.

20. Herpesvirus nach Anspruch 19, worin die Hämagglutinin-Neuraminidase-Gene von dem Influenza A/Pferde/Prag/56-Isolat des Pferde-Influenzavirus sind.

21. Herpesvirus nach Anspruch 19, worin die Hämagglutinin-Neuraminidase-Gene von dem Influenza A/Pferde/Miami/63-lsolat des Pferdeinfluenzavirus sind.

22. Herpesvirus nach Anspruch 19, worin die Hämaggiutinin-Neuraminidase-Gene von dem Influenza A/Pferde/Kentucky/81-lsolat des Pferdeinfluenzavirus sind.

23. Herpesvirus nach Anspruch 19, worin die Hämaggiutinin-Neuraminidase-Gene von dem Influenza A/Pferde/Alaska/91-Isolat des Pferde-Influenzavirus sind.

24. Herpesvirus nach Anspruch 13, das bezeichnet ist durch die ATCC-Hinteriegungsnummer VR 2363 (S-4EHV-003).

25. Herpesvirus nach Anspruch 14, das bezeichnet ist durch die ATCC-Hinterlegungsnummer VR 2362 (S-4EHV-002).

26. Herpesvirus nach Anspruch 14, das bezeichnet ist durch die ATCC-Hinterlegungsnummer VR 2360 (S-1EHV-004).

27. Herpesvirus nach Anspruch 15, das bezeichnet ist durch die ATCC-Hinterlegungsnummer VR 2358 (S-1EHV-002).

28. Herpesvirus nach Anspruch 16, das bezeichnet ist durch die ATCC-Hinterlegungsnummer VR 2359 (S-1EHV-003).

29. Herpesvirus nach Anspruch 18, bezeichnet als S-4EHV-010, konstruiert wie in Beispiel 16 beschrieben.

30. Herpesvirus nach Anspruch 20, bezeichnet als S-4EHV-011, konstruiert wie in Beispiel 17 beschrieben.

31. Herpesvirus nach Anspruch 21, bezeichnet als S-4EHV-012, konstruiert wie in Beispiel 18 beschrieben.

32. Herpesvirus nach Anspruch 22, bezeichnet als S-4EHV-013, konstruiert wie in Beispiel 19 beschrieben.

33. Herpesvirus nach Anspruch 23, bezeichnet als S-4EHV-014, konstruiert wie in Beispiel 20 beschrieben.

## Revendications

1. Herpesvirus équin recombiné vivant, qui comporte l'ADN génomique de l'herpesvirus équin de type 1 (EHV-1) ou 4 (EHV-4) d'où a été délétée une séquence d'ADN située dans le gène US2, présentée en tant que Séquence n° 1 dans le cas d'EHV-1 et en tant que Séquence n° 3 dans le cas d'EHV-4, ce qui fait que ce gène US2 est inactivé.

2. Herpesvirus conforme à la revendication 1, d'où a été délétée au moins une autre séquence d'ADN.

3. Herpesvirus conforme à la revendication 2, dans lequel ladite séquence d'ADN délétée se trouvait dans le gène codant la thymidine kinase (TK), dans le gène codant la glycoprotéine E (gpE), dans le gène codant la glycoprotéine G (gpG), et/ou dans le gène codant la grande glycoprotéine de membrane (MGP).

4. Herpesvirus conforme à l'une des revendications 1 à 3, qui contient en outre une séquence d'ADN étranger insérée dedans.

5. Herpesvirus conforme à la revendication 4, dans lequel la séquence d'ADN étranger code un marqueur détectable.

6. Herpesvirus conforme à la revendication 5, dans lequel le marqueur détectable est la β-galactosidase.

7. Herpesvirus conforme à la revendication 4, dans lequel l'ADN étranger provient d'un virus de grippe équine.

8. Herpesvirus conforme à la revendication 4, dans lequel l'ADN étranger provient d'un gène codant la glycoprotéine B (gpB) d'EHV-1 ou la glycoprotéine D (gpD) d'EHV-1 (herpesvirus équin de type 1).

9. Herpesvirus conforme à la revendication 7, dans lequel l'ADN étranger provient des gènes d'hémagglutinine et de neuraminidase de l'isolat Influenza A/Equi/Prague/56 du virus de la grippe équine.

10. Herpesvirus conforme à la revendication 7, dans lequel l'ADN étranger provient des gènes d'hémagglutinine et de neuraminidase de l'isolat Influenza A/Equi/Miami/63 du virus de la grippe équine.

11. Herpesvirus recombiné vivant conforme à la revendication 7, dans lequel l'ADN étranger provient des gènes d'hémagglutinine et de neuraminidase de l'isolat Influenza A/Equi/Kentucky/81 du virus de la grippe équine.

12. Herpesvirus équin conforme à la revendication 7, dans lequel l'ADN étranger provient des gènes d'hémagglutinine et de neuraminidase de l'isolat Influenza A/Equi/Alaska/91 du virus de la grippe équine.

13. Herpesvirus conforme à la revendication 4, comportant une délétion dans le gène US2 et portant, insérée à la place de la séquence délétée du gène US2, une séquence d'ADN codant la β-galactosidase d'*E. coli*.

14. Herpesvirus conforme à la revendication 13, comportant également une délétion dans le gène codant la TK.

15. Herpesvirus conforme à la revendication 13, d'où ont également été délétés le gène de gpG et une partie du gène de MGP.

16. Herpesvirus conforme à la revendication 14, d'où ont également été délétés le gène de gpG et une partie du gène de MGP.

17. Herpesvirus conforme à la revendication 3, comportant une délétion dans le gène codant la TK et dans le gène codant la gpE.

18. Herpesvirus conforme à la revendication 17, comportant une séquence d'ADN codant la gpB d'EHV1, insérée à la place de la séquence délétée du gène de gpE, et une séquence d'ADN codant la gpD d'EHV1, insérée à la place de la séquence délétée du gène de TK.

19. Herpesvirus conforme à la revendication 17, comportant une séquence d'ADN des gènes d'hémagglutinine et de neuraminidase d'un virus de grippe équine, insérée à la place de la séquence délétée du gène de gpE.

20. Herpesvirus conforme à la revendication 19, dans lequel les gènes d'hémagglutinine et de neuraminidase sont ceux de l'isolat Influenza A/Equi/Prague/56 du virus de la grippe équine.

21. Herpesvirus conforme à la revendication 19, dans lequel les gènes d'hémagglutinine et de neuraminidase sont ceux de l'isolat Influenza A/Equi/Miami/63 du virus de la grippe équine.

22. Herpesyirus conforme à la revendication 19, dans lequel les gènes d'hémagglutinine et de neuraminidase sont ceux de l'isolat Influenza A/Equi/Kentucky/81 du virus de la grippe équine.

23. Herpesvirus conforme à la revendication 19, dans lequel les gènes d'hémagglutinine et de neuraminidase sont ceux de l'isolat Influenza A/Equi/Alaska/91 du virus de la grippe équine.

24. Herpesvirus conforme à la revendication 13, désigné par le numéro d'accès ATCC VR 2363 (S-4EHV-003).

25. Herpesvirus conforme à la revendication 14, désigné par le numéro d'accès ATCC VR 2362 (S-4EHV-002).

26. Herpesvirus conforme à la revendication 14, désigné par le numéro d'accès ATCC VR 2360 (S-1EHV-004).

27. Herpesvirus conforme à la revendication 15, désigné par le numéro d'accès ATCC VR 2358 (S-1EHV-002).

28. Herpesvirus conforme à la revendication 16, désigné par le numéro d'accès ATCC VR 2359 (S-1EHV-003).

29. Herpesvirus conforme à la revendication 18, appelé S-4EHV-010 et construit de la façon décrite dans l'exemple 16.

30. Herpesvirus conforme à la revendication 20, appelé S-4EHV-011 et construit de la façon décrite dans l'exemple 17.

31. Herpesvirus conforme à la revendication 21, appelé S-4EHV-012 et construit de la façon décrite dans l'exemple 18.

32. Herpesvirus conforme à la revendication 22, appelé S-4EHV-013 et construit de la façon décrite dans l'exemple 19.

33. Herpesvirus conforme à la revendication 23, appelé S-4EHV-014 et construit de la façon décrite dans l'exemple 20.


### SEQUENCE LISTING

(1) GENERAL INFORMATION:
   (i) APPLICANT: Cochran Ph.D., Mark D
   (ii) TITLE OF INVENTION: Recombinant Equine Herpesvirus
   (iii) NUMBER OF SEQUENCES: 71
   (iv) CORRESPONDENCE ADDRESS:
      (A) ADDRESSEE: John P. White
      (B) STREET: 30 Rockefeller Plaza
      (C) CITY: New York
      (D) STATE: New York
      (E) COUNTRY: USA
      (F) ZIP: 10112
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: white, John P
   (ix) TELECOMMUNICATION INFORMATION:
      (A) TELEPHONE: (212)977-9550
      (B) TELEFAX: (212)664-0525
      (C) TELEX: 422523
(2) INFORMATION FOR SEQ ID NO:1:
   (i) SEQUENCE CHARACTERISTICS:
      (A) LENGTH: 1322 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Equine herpesvirus 1
      (B) STRAIN: Dutta
      (C) INDIVIDUAL ISOLATE: S-1EHV-000
   (vii) IMMEDIATE SOURCE:
      (B) CLONE: 432-54.N17
   (viii) POSITION IN GENOME:
      (B) MAP POSITION: -83
      (C) UNITS: %G
   (ix) FEATURE:
      (A) NAME/KEY: CDS
      (B) LOCATION: 249..1157
      (D) OTHER INFORMATION: /codon_start= 249
         /product= "US2 gene product"
         /gene= "US2"
   (xi) SEQUENCE DESCRIPTION: SEQ in NO:1:
   GGATCCACCG AGGGTGTGGG AGGTGGTAGC GGAGGCGTGG TGTCCATCGA TTCTGACGCG 60
   TCGCTCGTAG TGGAAAACCA GTCGGTTAGG TGGTCGCATT GTTTATTTTC CATTCCGATG 120
   CCGTGGCGGT GTGCCTATAA AGCTATAGGG CTTGGCGCAC GGGCAGTCTT TTTCACAACA 180
   GAGTGTGTAT CTAGAGCAGC TCTGCTGAAA TTTATGGAGT TGGTTCAACC CACCCATTTG 240
   TTAATAAC ATG GGT GTG GTC TTA ATT ACA GTT GTT ACA GTT GTC GAC AGA 290
   Met Gly Val Val Leu Ile Thr Val Val Thr Val Val Asp Arg
   1 5 10
   CAC AAA GCA TTG CCA AAC AGT TCC ATC GAC GTC GAT GGA CAT CTG TGG 338
   His Lys Ala Leu Pro Asn Ser Ser Ile Asp Val Asp Gly His Leu Trp
   15 20 25 30
   GAG TTT TTG AGC CGA CAA TGT TTC GTA TTG GCA TCT GAA CCG CTT GGA 386
   Glu Phe Leu Ser Arg Gln Cys Phe Val Leu Ala Ser Glu Pro Leu Gly
   35 40 45
   ATA CCC ATA GTG GTA CGC TCC GCC GAT CTC TAC AGA TTT TCA TCG AGT 434
   Ile Pro Ile Val Val Arg Ser Ala Asp Leu Tyr Arg Phe Ser Ser Ser
   50 55 60
   TTA TTG ACC CTA CCA AAG GCG TGT AGG CCA ATA GTC AGA ACC AGG GGG 482
   Leu Leu Thr Leu Pro Lys Ala Cys Arg Pro Ile Val Arg Thr Arg Gly
   65 70 75
   GCT ACA GCT ATA GCT CTA GAT AGA AAC GGG GTG GTT TAC CAC GAA GAT 530
   Ala Thr Ala Ile Ala Leu Asp Arg Asn Gly Val Val Tyr His Glu Asp
   80 85 90
   AGA ATG GGT GTG AGC ATA GAG TGG CTC TCT GTA CTC TCT GGC TAT AAC 578
   Arg Met Gly Val Ser Ile Glu Trp Leu Ser Val Leu Ser Gly Tyr Asn
   95 100 105 110
   CAT CTC AAC TCC AGC CTT ATC ATT AAT CAG CCC TAT CAC CTC TGG GTG 626
   His Leu Asn Ser Ser Leu Ile Ile Asn Gln Pro Tyr His Leu Trp Val
   115 120 125
   CTG GGG GCA GCA GAC TTG TGC AAG CCG GTG TTT GAC CTG ATA CCC GGT 674
   Leu Gly Ala Ala Asp Leu Cys Lys Pro Val Phe Asp Leu Ile Pro Gly
   130 135 140
   CCT AAA CGA ATG GTA TAC GCA GAG ATA GCA GAT GAG TTT CAT AAA TCT 722
   Pro Lys Arg Met Val Tyr Ala Glu Ile Ala Asp Glu Phe His Lys Ser
   145 150 155
   TGG CAG CCT CCC TTC GTG TGT GGA AAA CTG TTT GAG ACA ATA CCA TGG 770
   Trp Gln Pro Pro Phe Val Cys Gly Lys Leu Phe Glu Thr Ile Pro Trp
   160 165 170
   ACC ACC GTT GAG CAT AAT CAT CCG CTC AAA TTA AGA GCG GCG GGT GGA 818
   Thr Thr Val Glu His Asn His Pro Leu Lys Leu Arg Ala Ala Gly Gly
   175 180 185 190
   GAA GAC ACC GTA GTG GGT GAG TGT GGG TTT TCC AAA CAT AGC TCG AAT 866
   Glu Asp Thr Val Val Gly Glu Cys Gly Phe Ser Lys His Ser Ser Asn
   195 200 205
   TCA TTA GTT CGT CCA CCC ACA GTT AAG CGG GTG ATT TAC GCG GTG GTC 914
   Ser Leu Val Arg Pro Pro Thr Val Lys Arg Val Ile Tyr Ala Val Val
   210 215 220
   GAC CCC GCG CGC CTT CGG GAA ATT CCC GCC CCG GGG CGG CCG CTG CCG 962
   Asp Pro Ala Arg Leu Arg Glu Ile Pro Ala Pro Gly Arg Pro Leu Pro
   225 230 235
   CGG CGG CGG CCG TCG GAG GGG GGG ATG CGC GCC CCG AGG CGG CGC TCG 1010
   Arg Arg Arg Pro Ser Glu Gly Gly Met Arg Ala Pro Arg Arg Arg Ser
   240 245 250
   CGC GCT CCC GCG GCC GCT CGG TCC ACG GCC GCC GCC GCG ACG CCG CCC 1058
   Arg Ala Pro Ala Ala Ala Arg Ser Thr Ala Ala Ala Ala Thr Pro Pro
   255 260 265 270
   CGC CCC GGG GAC CCG CGG GCG CCC GCC GCC CGC CGG GCG GGA GAC GTG 1106
   Arg Pro Gly Asp Pro Arg Ala Pro Ala Ala Arg Arg Ala Gly Asp Val
   275 280 285
   ACG TGG ATG GAA CGC CTA CTC TGG GGA GTG TTC GGC CGG ACA TCC ACA 1154
   Thr Trp Met Glu Arg Leu Leu Trp Gly Val Phe Gly Arg Thr Ser Thr
   290 295 300
   CGT TAAAAGGTAG GGGACTCTCG CCAGTACCTC ACCTCGCTTT GTGGGTTGAG 1207
   Arg
   CAGTGGTTTC TTGCCTTGCA AAAGCCTCGC CTTTACACCC ACCACCGCCT AGCCCTGCAC 1267
   AACATCCCCT CCATTTTGAA GGGAGAAAAG AGAGAAGACA CCTTTGAAGA TAACA 1322
(2) INFORMATION FOR SEQ ID NO:2:
   (i) SEQUENCE CHARACTERISTICS:
      (A) LENGTH: 303 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Gly Val Val Leu Ile Thr Val Val Thr Val Val Asp Arg His Lys
     1               5                  10                  15
   Ala Leu Pro Asn Ser Ser Ile Asp Val Asp Gly His Leu Trp Glu Phe
                20                  25                  30
   Leu Ser Arg Gln Cys Phe Val Leu Ala Ser Glu Pro Leu Gly Ile Pro
            35                  40                  45
   Ile Val Val Arg Ser Ala Asp Leu Tyr Arg Phe Ser Ser Ser Leu Leu
        50                  55                  60
   Thr Leu Pro Lys Ala Cys Arg Pro Ile Val Arg Thr Arg Gly Ala Thr
    65                  70                  75                  80
   Ala Ile Ala Leu Asp Arg Asn Gly Val Val Tyr His Glu Asp Arg Met
                    85                  90                  95
   Gly Val Ser Ile Glu Trp Leu Ser Val Leu Ser Gly Tyr Asn His Leu
               100                 105                 110
   Asn Ser Ser Leu Ile Ile Asn Gln Pro Tyr His Leu Trp Val Leu Gly
           115                 120                 125
   Ala Ala Asp Leu Cys Lys Pro Val Phe Asp Leu Ile Pro Gly Pro Lys
       130                 135                 140
   Arg Met Val Tyr Ala Glu Ile Ala Asp Glu Phe His Lys Ser Trp Gln
   145                 150                 155                 160
   Pro Pro Phe Val Cys Gly Lys Leu Phe Glu Thr Ile Pro Trp Thr Thr
                   165                 170                 175
   Val Glu His Asn His Pro Leu Lys Leu Arg Ala Ala Gly Gly Glu Asp
               180                 185                 190
   Thr Val Val Gly Glu Cys Gly Phe Ser Lys His Ser Ser Asn Ser Leu
           195                 200                 205
   Val Arg Pro Pro Thr Val Lys Arg Val Ile Tyr Ala Val Val Asp Pro
       210                 215                 220
   Ala Arg Leu Arg Glu Ile Pro Ala Pro Gly Arg Pro Leu Pro Arg Arg
   225                 230                 235                 240
   Arg Pro Ser Glu Gly Gly Met Arg Ala Pro Arg Arg Arg Ser Arg Ala
                   245                 250                 255
   Pro Ala Ala Ala Arg Ser Thr Ala Ala Ala Ala Thr Pro Pro Arg Pro
               260                 265                 270
   Gly Asp Pro Arg Ala Pro Ala Ala Arg Arg Ala Gly Asp Val Thr Trp
           275                 280                 285
   Met Glu Arg Leu Leu Trp Gly Val Phe Gly Arg Thr Ser Thr Arg
       290                 295                 300
(2) INFORMATION FOR SEQ ID NO:3:
   (i) SEQUENCE CHARACTERISTICS:
      (A) LENGTH: 1252 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGIMAL SOURCE:
      (A) ORGANISM: Equine herpesvirus 4
      (B) STRAIN: Dutta
      (C) INDIVIDUAL ISOLATE: S-4EHV-000
   (vii) IMMEDIATE SOURCE:
      (B) CLONE: 497-52.53 and 480-18.9
   (viii) POSITION IN GENOME:
      (B) MAP POSITION: -83
      (C) UNITS: %G
   (ix) FEATURE:
      (A) NAME/KEY: CDS
      (B) LOCATION: 153..1124
      (D) OTHER INFORMATION: /codon_start= 153
         /product= "US2 gene product"
   (xi) SEQUENCE DESCRIPTION: SEQ ID NO:3:
   AAGTGTCGAG GTATTTCCAT GCCGATGCTG TGGCTGTGCT ATAAAGCTAC GAATTTCCCG      60
   TAACACAGCA AGTCTTTTTC ACAACAAAGT GTGTAGCTAG AGCAGCTCTG CTGAAATTTA     120
   TTGGGTTGGT TAACACACCC ATTGCTAATA AC ATG GGT GTG GTT TTA ATT ACA      173
                                       Met Gly Val Val Leu Ile Thr
                                        1               5
   GTT GTC ATG GTG GTT GAC AGG CAT AAA GCT TTG CCC GAC AGT TCT ATC       221
   Val Val Met Val Val Asp Arg His Lys Ala Leu Pro Asp Ser Ser Ile
   10 15 20
   GAC GTA GAT GGA AAA CTG TGG GAG TTT TTG GGA CGA CTA TGT TTT GTA 269
   Asp Val Asp Gly Lys Leu Trp Glu Phe Leu Gly Arg Leu Cys Phe Val
   25 30 35
   TTA GCC TCA GAA CCT CTA GGA ATA CCA ATA GTG GTG CGT TCT GCT GAC 317
   Leu Ala Ser Glu Pro Leu Gly Ile Pro Ile Val Val Arg Ser Ala Asp
   40 45 50 55
   CTG TAC AAA TTT TCT TCG AGT CTC TTA GCC CTG CCA AAA GCA TGC AGG 365
   Leu Tyr Lys Phe Ser Ser Ser Leu Leu Ala Leu Pro Lys Ala Cys Arg
   60 65 70
   CCT ATA GTG AGA ACT AGG GGG GCT ACT GCT ATA GCC CTA GAA AGA AAT 413
   Pro Ile Val Arg Thr Arg Gly Ala Thr Ala Ile Ala Leu Glu Arg Asn
   75 80 85
   GGC GTG ATT TAT CAA GAG GAT AGA ATT GGC ATT AGT ATA GAG TGG CTT 461
   Gly Val Ile Tyr Gln Glu Asp Arg Ile Gly Ile Ser Ile Glu Trp Leu
   90 95 100
   TCT GTA CTA TCC GGC TAC AAC TAC CTC AAC TCC AGC ATT ATC ATC AAT 509
   Ser Val Leu Ser Gly Tyr Asn Tyr Leu Asn Ser Ser Ile Ile Ile Asn
   105 110 115
   AGG CCA TAC CAC CTA TGG GTT TTG GGA GCT GCA GAT TTA TGC AGG CCT 557
   Arg Pro Tyr His Leu Trp Val Leu Gly Ala Ala Asp Leu Cys Arg Pro
   120 125 130 135
   GTG TTC AAC CTC ATA CCG GGC CCC AAG CGA ATT GTG TAT GTG GAG ATC 605
   Val Phe Asn Leu Ile Pro Gly Pro Lys Arg Ile Val Tyr Val Glu Ile
   140 145 150
   GAA GAT GAG TTT AAT AAA TCT TGG CAG CCC AGC TTC GTG TGC GGA AAA 653
   Glu Asp Glu Phe Asn Lys Ser Trp Gln Pro Ser Phe Val Cys Gly Lys
   155 160 165
   CTA TTC GAA ACA ATA CCG TTG ACA ACC GTG GAT TAT AAG CAT CTA CTA 701
   Leu Phe Glu Thr Ile Pro Leu Thr Thr Val Asp Tyr Lys His Leu Leu
   170 175 180
   AAA CAA AAG GTT TTA CCC GGA CAA GAC CAC CCT GAG AGC GCG CGC AGT 749
   Lys Gln Lys Val Leu Pro Gly Gln Asp His Pro Glu Ser Ala Arg Ser
   185 190 195
   TTA TTA CAA CAT AAA TCA TCT TTT GTA TCT CCC CCG CCA AAT TTT AAG 797
   Leu Leu Gln His Lys Ser Ser Phe Val Ser Pro Pro Pro Asn Phe Lys
   200 205 210 215
   CGG TTA ATT TAT GCG GTT GTA GAC CCT ATG CGT TTA CAA GAG AAT TTA 845
   Arg Leu Ile Tyr Ala Val Val Asp Pro Met Arg Leu Gln Glu Asn Leu
   220 225 230
   TGT CCA CAA ATA ACT AAC AGA ACA AAA ACT AAA AGA CGT TCT AAA AAA 893
   Cys Pro Gln Ile Thr Asn Arg Thr Lys Thr Lys Arg Arg Ser Lys Lys
   235 240 245
   ACT TAT AAT GGC CTG TTT TGC CAA GAG TCT ACA GCC AGC CTA AAC GAT 941
   Thr Tyr Asn Gly Leu Phe Cys Gln Glu Ser Thr Ala Ser Leu Asn Asp
   250 255 260
   AAG ATG TGT TTT ACT CCA CAG CCA TCA AAA GGC AAA AAC TTG CAG CGC 989
   Lys Met Cys Phe Thr Pro Gln Pro Ser Lys Gly Lys Asn Leu Gln Arg
   265 270 275
   GTT AGC ACG TCG ATG CAA GCC AAC TCT ACA ATA CCA CCT AGC ACC CTA 1037
   Val Ser Thr Ser Met Gln Ala Asn Ser Thr Ile Pro Pro Ser Thr Leu
   280 285 290 295
   TCT CCT CGT GCA GCT GCC CGG AAA CCC ACA GAA ATG ACG TGG AAA TCA 1085
   Ser Pro Arg Ala Ala Ala Arg Lys Pro Thr Glu Met Thr Trp Lys Ser
   300 305 310
   CGC CTA CTA GGG GGT GTG TTT GAT AGA ACA GCC AGA CGT TAAAAGGTTG 1134
   Arg Leu Leu Gly Gly Val Phe Asp Arg Thr Ala Arg Arg
   315 320
   GGGAAGCTCT TTGCTAGTCA CTGCGCTTTG CCAAGTGTGG TTTCCTGTGA GATTTTTACT 1194
   TACAAACTTC ACGTCTATCT TTAGACATGA GCTCCGACAT GCTTACAGCC GCCACTGC 1252
(2) INFORMATION FOR SEQ ID NO:4:
   (i) SEQUENCE CHARACTERISTICS:
      (A) LENGTH: 324 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Gly Val Val Leu Ile Thr Val Val Met Val Val Asp Arg His Lys
    1               5                  10                  15
   Ala Leu Pro Asp Ser Ser Ile Asp Val Asp Gly Lys Leu Trp Glu Phe
               20                  25                  30
   Leu Gly Arg Leu Cys Phe Val Leu Ala Ser Glu Pro Leu Gly Ile Pro
           35                  40                  45
   Ile Val Val Arg Ser Ala Asp Leu Tyr Lys Phe Ser Ser Ser Leu Leu
       50                  55                  60
   Ala Leu Pro Lys Ala Cys Arg Pro Ile Val Arg Thr Arg Gly Ala Thr
   65                  70                  75                  80
   Ala Ile Ala Leu Glu Arg Asn Gly Val Ile Tyr Gln Glu Asp Arg Ile
                           85                      90                      95
   Gly Ile Ser Ile Glu Trp Leu Ser Val Leu Ser Gly Tyr Asn Tyr Leu
               100                 105                 110
   Asn Ser Ser Ile Ile Ile Asn Arg Pro Tyr His Leu Trp Val Leu Gly
           115                 120                 125
   Ala Ala Asp Leu Cys Arg Pro Val Phe Asn Leu Ile Pro Gly Pro Lys
       130                 135                 140
   Arg Ile Val Tyr Val Glu Ile Glu Asp Glu Phe Asn Lys Ser Trp Gln
   145                 150                 155                 160
   Pro Ser Phe Val Cys Gly Lys Leu Phe Glu Thr Ile Pro Leu Thr Thr
                   165                 170                 175
   Val Asp Tyr Lys His Leu Leu Lys Gln Lys Val Leu Pro Gly Gln Asp
               180                 185                 190
   His Pro Glu Ser Ala Arg Ser Leu Leu Gln His Lys Ser Ser Phe Val
           195                 200                 205
   Ser Pro Pro Pro Asn Phe Lys Arg Leu Ile Tyr Ala Val Val Asp Pro
       210                 215                 220
   Met Arg Leu Gln Glu Asn Leu Cys Pro Gln Ile Thr Asn Arg Thr Lys
   225                 230                 235                 240
   Thr Lys Arg Arg Ser Lys Lys Thr Tyr Asn Gly Leu Phe Cys Gln Glu
                   245                 250                 255
   Ser Thr Ala Ser Leu Asn Asp Lys Met Cys Phe Thr Pro Gln Pro Ser
               260                 265                 270
   Lys Gly Lys Asn Leu Gln Arg Val Ser Thr Ser Met Gln Ala Asn Ser
           275                 280                 285
   Thr Ile Pro Pro Ser Thr Leu Ser Pro Arg Ala Ala Ala Arg Lys Pro
       290                 295                 300
   Thr Glu Met Thr Trp Lys Ser Arg Leu Leu Gly Gly Val Phe Asp Arg
   305                 310                 315                 320
   Thr Ala Arg Arg
(2) INFORMATION FOR SEQ ID NO:5:
   (i) SEQUENCE CHARACTERISTICS:
      (A) LENGTH: 1149 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Equine herpesvirus 4
      (B) STRAIN: Dutte
      (C) INDIVIDUAL ISOLATE: S-4EHV-000
   (vii) IMMEDIATE SOURCE:
      (B) CLONE: 467-42.A12
   (viii) POSITION IN GENOME:
      (B) MAP POSITION: -89
      (C) UNITS: %G
   (ix) FEATURE:
      (A) MAME/KEY: CDS
      (B) LOCATION: 271..1149
      (D) OTHER INFORMATION: /partial
         /codon_start= 271
         /function= "menbrane glycoprotein"
         /product = "Glycoprotein E N-terminus"
         /gene= "gpE"
   (xi) SEQUENCE DESCRIPTION: SEQ ID ND:5:
   TCTAGAACAG TTGAACCGTA AACTGGAGGC CATAAAAGAG GAAGACTAAT AATGGGGGGT 60
   TTTTAAAGTT TATGTATTAT TGTTTCTATA TATTAAAAAT TGTTGAAATA TAAATATCTT 120
   ATGTAATGTT TACATTATTC GTGATTGGGA CGGTCTTAGG GGAGGTGGTG CAACTAGGGT 180
   TTAAAGCCCT GAATGTTCTG GAGTGAACCC ACAGTTCTCC TCTTTGGGCG TCAAAGCAAT 240
   CAGACGTCCA ATCTAAAGTA GAACGTCACA ATG GAG CTG TTA GAC TCC CGC CGT 294
   Met Glu Leu Leu Asp Ser Arg Arg
   1 5
   GCT TTT TTC TTT TTT GTA CTA ATA ACA GTA CTC GAT GCG TGG GGA GTT 342
   Ala Phe Phe Phe Phe Val Leu Ile Thr Val Leu Asp Ala Trp Gly Val
   10 15 20
   CAA CGG GTT GAA CTC ACC GAG GGG GCA TGG GCC ATG ATC GAC GGA AGA 390
   Gln Arg Val Glu Leu Thr Glu Gly Ala Trp Ala Met Ile Asp Gly Arg
   25 30 35 40
   GAC GTT TTA ACC CCA ACT AAC ACG ACC ACT AGG GTT ACA AAG GCC TGG 438
   Asp Val Leu Thr Pro Thr Asn Thr Thr Thr Arg Val Thr Lys Ala Trp
   45 50 55
   ACA TTT TTG GAA ACC CCA CCG GGA TGT GCT GGT GAT ATA ACA GTC AAG 486
   Thr Phe Leu Glu Thr Pro Pro Gly Cys Ala Gly Asp Ile Thr Val Lys
   60 65 70
   ACT   GTG   TGC   GTA   CAA   GCT   AGT   CTG   TGC   GAA   GAT   AAC   ATT   ATA   ATA   GGA         534
   Thr   Val   Cys   Val   Gln   Ala   Ser   Leu   Cys   Glu   Asp   Asn   Ile   Ile   Ile   Gly
               75                            80                            85
   AAT   CAC   TGT   AAC   CTA   CTA   ACC   GGG   GAG   CAT   GGC   ATT   GCG   CTT   GCA   GAG         582
   Asn   His   Cys   Asn   Leu   Leu   Thr   Gly   Glu   His   Gly   Ile   Ala   Leu   Ala   Glu
         90                            95                            100
   TTT   AAC   GTA   GTT   AAC   GGA   TCG   CTA   CAA   AGG   ACC   AAA   GAT   GTG   TAC   TTT         630
   Phe   Asn   Val   Val   Asn   Gly   Ser   Leu   Gln   Arg   Thr   Lys   Asp   Val   Tyr   Phe
   105                           110                           115                           120
   GTT   AAT   GGA   ACA   GTT   TTT   CCT   ATT   CTG   GCA   GAA   ACC   CGC   AGC   GTG   TTA         678
   Val   Asn   Gly   Thr   Val   Phe   Pro   Ile   Leu   Ala   Glu   Thr   Arg   Ser   Val   Leu
                           125                           130                           135
   CAA   ATT   CAG   AGG   GCA   ACC   CCA   TCC   ATA   GCT   GGA   GTT   TAT   ACT   CTT   CAT         726
   Gln   Ile   Gln   Arg   Ala   Thr   Pro   Ser   Ile   Ala   Gly   Val   Tyr   Thr   Leu   His
                     140                           145                           150
   GTT   TCC   ATA   AAC   GGA   CAC   ATA   AAA   CAC   TCT   GTT   GTG   TTG   CTC   ACC   GTA         774
   Val   Ser   Ile   Asn   Gly   His   Ile   Lys   His   Ser   Val   Val   Leu   Leu   Thr   Val
               155                           160                           165
   AAG   AAA   CCA   CCA   ACA   CGC   GTA   CAT   GTC   AAG   ACG   CCT   CCA   CCC   ATA   CTA         822
   Lys   Lys   Pro   Pro   Thr   Arg   Val   His   Val   Lys   Thr   Pro   Pro   Pro   Ile   Leu
         170                           175                           180
   GTT   CCC   CAG   GTT   ACA   CCA   GAG   GCA   CAT   ACA   GAT   TTC   ATA   GTG   CGC   GGA         870
   Val   Pro   Gln   Val   Thr   Pro   Glu   Ala   His   Thr   Asp   Phe   Ile   Val   Arg   Gly
   185                           190                           195                           200
   TAC   CAC   TCG   CGC   GTA   TAT   GCT   GTG   GGT   GAG   TCC   TTT   GAC   CTG   TCT   GTG         918
   Tyr   His   Ser   Arg   Val   Tyr   Ala   Val   Gly   Glu   Ser   Phe   Asp   Leu   Ser   Val
                           205                           210                           215
   CAC   CTA   GAA   TCC   CAC   ATA   CAG   GAG   TCT   AGC   TTT   AAC   GCT   GAA   ATC   CAA         966
   His   Leu   Glu   Ser   His   Ile   Gln   Glu   Ser   Ser   Phe   Asn   Ala   Glu   Ile   Gln
                     220                           225                           230
   TGG   TAT   TAT   ATG   AAT   ACG   TCA   TCG   TCA   TCA   TGC   GAT   TTG   TTT   CGA   GTT        1014
   Trp   Tyr   Tyr   Met   Asn   Thr   Ser   Ser   Ser   Ser   Cys   Asp   Leu   Phe   Arg   Val
               235                           240                           245
   TTT   GAA   ACA   TGC   ATT   TTT   CAC   CCA   ACC   GCT   ATG   GCC   TGC   CTG   CAC   CCC        1062
   Phe   Glu   Thr   Cys   Ile   Phe   His   Pro   Thr   Ala   Met   Ala   Cys   Leu   His   Pro
         250                           255                           260
   GAA   CAA   CAC   GCC   TGC   TGC   TTT   ACA   TCT   CCC   GTC   AGG   GCT   ACG   AAG   ATT        1110
   Glu   Gln   His   Ala   Cys   Cys   Phe   Thr   Ser   Pro   Val   Arg   Ala   Thr   Lys   Ile
   265                           270                           275                           280
   CTT   CAT   CGA   GTA   TAT   GGT   AAC   TGC   AGC   AAT   CGT   GGA   TCC                          1149
   Leu   His   Arg   Val   Tyr   Gly   Asn   Cys   Ser   Asn   Arg   Gly   Ser
                           285                           290
(2) INFORMATION FOR SEQ ID NO:6:
   (i) SEQUENCE CHARACTERISTICS:
      (A) LENGTH: 293 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Glu Leu Leu Asp Ser Arg Arg Ala Phe Phe Phe Phe Val Leu Ile
   1               5                   10                  15
   Thr Val Leu Asp Ala Trp Gly Val Gln Arg Val Glu Leu Thr Glu Gly
               20                  25                  30
   Ala Trp Ala Met Ile Asp Gly Arg Asp Val Leu Thr Pro Thr Asn Thr
           35                  40                  45
   Thr Thr Arg Val Thr Lys Ala Trp Thr Phe Leu Glu Thr Pro Pro Gly
       50                  55                  60
   Cys Ala Gly Asp Ile Thr Val Lys Thr Val Cys Val Gln Ala Ser Leu
   65                  70                  75                  80
   Cys Glu Asp Asn Ile Ile Ile Gly Asn His Cys Asn Leu Leu Thr Gly
                   85                  90                  95
   Glu His Gly Ile Ala Leu Ala Glu Phe Asn Val Val Asn Gly Ser Leu
               100                 105                 110
   Gln Arg Thr Lys Asp Val Tyr Phe Val Asn Gly Thr Val Phe Pro Ile
           115                 120                 125
   Leu Ala Glu Thr Arg Ser Val Leu Gln Ile Gln Arg Ala Thr Pro Ser
       130                 135                 140
   Ile Ala Gly Val Tyr Thr Leu His Val Ser Ile Asn Gly His Ile Lys
   145                 150                 155                 160
   His Ser Val Val Leu Leu Thr Val Lys Lys Pro Pro Thr Arg Val His
                   165                 170                 175
   Val Lys Thr Pro Pro Pro Ile Leu Val Pro Gln Val Thr Pro Glu Ala
               180                 185                 190
   His Thr Asp Phe Ile Val Arg Gly Tyr His Ser Arg Val Tyr Ala Val
           195                 200                 205
   Gly Glu Ser Phe Asp Leu Ser Val His Leu Glu Ser His Ile Gln Glu
       210                 215                 220
   Ser Ser Phe Asn Ala Glu Ile Gln Trp Tyr Tyr Met Asn Thr Ser Ser
   225                 230                 235                 240
   Ser Ser Cys Asp Leu Phe Arg Val Phe Glu Thr Cys Ile Phe His Pro
                   245                 250                 255
   Thr Ala Met Ala Cys Leu His Pro Glu Gln His Ala Cys Cys Phe Thr
               260                 265                 270
   Ser Pro Val Arg Ala Thr Lys Ile Leu His Arg Val Tyr Gly Asn Cys
           275                 280                 285
   Ser Asn Arg Gly Ser
       290
(2) INFORMATION FOR SEQ ID NO:7:
   (i) SEQUENCE CHARACTERISTICS:
      (A) LENGTH: 18 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Equine herpesvirus 1
      (B) STRAIN: Dutta
      (C) INDIVIDUAL ISOLATE: S-1EHV-000
   (viii) POSITION IN GENOME:
      (B) MAP POSITION: -83
      (C) UNITS: %G
   (ix) FEATURE:
      (A) NAME/KEY: Region
      (B) LOCATION: 1..18
      (D) OTHER INFORMATION: /label= EHV1-US2
         /note= "Conserved region of US2 gene starting at amino acid 123."
   (xi) SEQUENCE DESCRIPTION: SEQ ID NO:7:
   His Leu Trp Val Leu Gly Ala Ala Asp Leu Cys Lys Pro Val Phe Asp
   1               5                   10                  15
   Leu Ile
(2) INFORMATION FOR SEQ ID NO:8:
   (i) SEQUENCE CHARACTERISTICS:
      (A) LENGTH: 18 amino acids
      (B) TYPE: amino acids
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Equine herpesvirus 4
      (B) STRAIN: Dutta
      (C) INDIVIDUAL ISOLATE: S-4ENV-000
   (viii) POSITION IN GENOME:
      (B) MAP POSITION: -83
      (C) UNITS: XG
   (ix) FEATURE:
      (A) MAME/KEY: Region
      (B) LOCATION: 1..18
      (D) OTHER INFORMATION: /label= EHV4-US2
         /note= "Conserved region of US2 gene starting at amino acid 123."
   (xi) SEQUENCE DESCRIPTION: SEQ ID NO:8:
   His Leu Trp Val Leu Gly Ala Ala Asp Leu Cys Arg Pro Val Phe Asn
   1               5                   10                  15
   Leu Ile
(2) INFORMATION FOR SEO ID NO:9:
   (i) SEQUENCE CHARACTERISTICS:
      (A) LENGTH: 18 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Herpes simplex virus 1
      (B) STRAIN: 17
   (viii) POSITION IN GENOME:
      (B) MAP POSITION: -88
      (C) UNITS: %G
   (ix) FEATURE:
      (A) NAME/KEY: Region
      (B) LOCATION: 1..18
      (D) OTHER INFORMATION: /labels HSV1-US2
         /note= "Conserved region of US2 gene starting at amino acid 124."
   (xi) SEQUENCE DESCRIPTION: SEQ ID NO:9:
   His Leu Trp Val Val Gly Ala Ala Asp Leu Cys Val Pro Phe Leu Glu
   1 5 10 15
   Tyr Ala
(2) INFORMATION FOR SEQ ID NO:10:
   (i) SEQUENCE CHARACTERISTICS:
      (A) LENGTH: 18 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Herpes simplex virus 2
      (B) STRAIN: NG52
   (viii) POSITION IN GENOME:
      (B) MAP POSITION: -88
      (C) UNITS: %G
   (ix) FEATURE:
      (A) NAME/KEY: Region
      (B) LOCATION: 1..18
      (D) OTHER INFORMATION: /label* HSV2-US2
         /note= "Conserved region of US2 gene starting at amino acid 123."
   (xi) SEQUENCE DESCRIPTION: SEQ ID NO:10:
   His Leu Trp Val Val Gly Ala Ala Asp Leu Cys Val Pro Phe Phe Glu
   1 5 10 15
   Tyr Ala
(2) INFORMATION FOR SEQ ID NO:11:
   (i) SEQUENCE CHARACTERISTICS:
      (A) LENGTH: 18 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Pseudorabies virus
      (B) STRAIN: NIA-3
   (viii) POSITION IN GENOME:
      (B) MAP POSITION: -90
      (C) UNITS: %G
   (ix) FEATURE:
      (A) MAME/KEY: Region
      (B) LOCATION: 1..18
      (D) OTHER INFORMATION: /label = PRV-US2
         /note= "Conserved region of US2 gene starting at amino acid 148."
   (xi) SEQUENCE DESCRIPTION: SEQ ID NO:11:
   His Leu Trp Ile Leu Gly Ala Ala Asp Leu Cys Asp Gln Val Leu Leu
   1 5 10 15
   Ala Ala
(2) INFORMATION FOR SEQ ID NO:12:
   (i) SEQUENCE CHARACTERISTICS:
      (A) LENGTH: 19 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Marek's disease gammaherpesvirus
      (B) STRAIN: RB1B
   (viii) POSITION IN GENOME:
      (B) MAP POSITION: -88
      (C) UNITS: %G
   (ix) FEATURE:
      (A) NAME/KEY: Region
      (B) LOCATION: 1..19
      (D) OTHER INFORMATION: /label* NOV-US2
         /note= "Conserved region of US2 gene starting at amino acid 132."
   (xi) SEQUENCE DESCRIPTION: SEQ 10 NO:12:
   His Ser Leu Trp Ile Val Gly Ala Ala Asp Ile Cys Arg Ile Ala Leu
   1 5 10 15
   Glu Cys Ile
(2) INFORMATION FOR SEQ ID NO:13:
   (i) SEQUENCE CHARACTERISTICS:
      (A) LENGTH: 18 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bovine herpesvirus 1
      (B) STRAIN: Cooper
      (C) INDIVIDUAL ISOLATE: S-IBR-000
   (viii) POSITION IN GENOME:
      (B) MAP POSITION: -85
      (C) UNITS: %G
   (ix) FEATURE:
      (A) NAME/KEY: Region
      (B) LOCATION: 1..18
      (D) OTHER INFORMATION: /label= IBR-US2
         /note= "Conserved region of US2 gene starting at amino acid 115."
   (xi) SEQUENCE DESCRIPTION: SEQ ID NO:13:
   His Met Trp Val Phe Gly Ala Ala Asp Leu Tyr Ala Pro Ile Phe Ala
   1 5 10 15
   His Ile
(2) INFORMATION FOR SEQ ID NO:14:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 450-46.B4 (Figure 4 Junction A)
   (xi) SEQUENCE DESCRIPTION: SEQ ID NO:14:
   AGGTGACACT ATAGAATACA CGGAATTCGA GCTCGCCCGG GGATCTCACC GCTTCCCGGG 60
   GGTAGC 66
(2) INFORMATION FOR SEQ ID NO:15:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 450-46.B4 (Figure 4 Junction B)
   (ix) FEATURE:
      (A) NAME/KEY: CDS
      (B) LOCATION: 1..66
      (D) OTHER INFORMATION: /product= "Region of deleted EHV1 thymidine kinase gene"
   (xi) SEQUENCE DESCRIPTION: SEQ ID NO:15:
   ACC ACG CCC TAC CTT ATC CTA CAC GAT CCT CTA GAG TCC ACC CTC AAT      48
   Thr Thr Pro Tyr Leu Ile Leu His Asp Pro Leu Glu Ser Thr Leu Asn
    1               5                  10                  15
   GTG GAC GAG CAC GTG CGA                                              66
   Val Asp Glu His Val Arg
                20
(2) INFORMATION FOR SEQ ID NO:16:
   (i) SEQUENCE CHARACTERISTICS:
      (A) LENGTH: 22 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Thr Thr Pro Tyr Leu Ile Leu His Asp Pro Leu Glu Ser Thr Leu Asn
     1               5                  10                  15
   Val Asp Glu His Val Arg
               20
(2) INFORMATION FOR SEQ ID NO:17:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (generic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 450-46.B4 (Figure 4 Junction C)
   (ix) FEATURE:
      (A) NAME/KEY: CDS
      (B) LOCATION: 1..30
      (D) OTHER INFORMATION: /partial
         /codon_start= 1
         /product= "Region of EHV1 glycoprotein H gene"
   (xi) SEQUENCE DESCRIPTION: SEQ ID NO:17:
   TAT CTC GGC CAT GGG TTT ATG GGC CTG CAG CCCAAGCTTG GCGTAATCAT      50
   Tyr Leu Gly His Gly Phe Met Gly Leu Gln
     1               5                  10
   GGTCATAGCT GTTTCC                                                  66
(2) INFORMATION FOR SEQ ID NO:18:
   (i) SEQUENCE CHARACTERISTICS:
      (A) LENGTH: 10 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Tyr Leu Gly His Gly Phe Met Gly Leu Gln
     1               5                  10
(2) INFORMATION FOR SEQ ID NO:19:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 467-21.19 (Figure 5 Junction A)
   (xi) SEQUENCE DESCRIPTION: SEQ ID NO:19:
   CACTATAGAA TACACGGCGA GCTCGCCCGG GGATCCACCG AGGGTGTGGG   AGGTGGTAGC   60
   GGAGGC                                                                66
(2) INFORMATION FOR SEQ ID NO:20:
   (i) SEQUENCE CHARACTERISTICS:
      (A) LENGTH: 66 base poirs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 467-21.19 (Figure 5 Junction B)
   (ix) FEATURE:
      (A) NAME/KEY: CDS
      (B) LOCATION: 1..30
      (D) OTHER INFORMATION: /partial
         /codon_start= 1
         /product= "Region of EHV1 US2 gene"
   (ix) FEATURE:
      (A) NAME/KEY: CDS
      (B) LOCATION: 33..65
      (D) OTHER INFORMATION: /partial
         /codon_start= 33
         /product= "Region of EHV1 US2 gene"
   (xi) SEQUENCE DESCRIPTION: SEQ ID NO:20:
   GTG TGT GGA AAA CTG TTT GAG ACA ATA CCA TG AAT TCA TTA GTT CGT      47
   Val Cys Gly Lys Leu Phe Glu Thr Ile Pro    Asn Ser Leu Val Arg
     1             5                   10       1               5
   CCA CCC ACA GTT AAG CGG G                                           66
   Pro Pro Thr Val Lys Arg
                   10
(2) INFORMATION FOR SEQ ID NO:21:
   (i) SEQUENCE CHARACTERISTICS:
      (A) LENGTH: 10 amino acids
      (B) TYPE: amino acid
      (D) TOPOLOGY: linear
   (ii) MOLECULE TYPE: protein
   (xi) SEQUENCE DESCRIPTION: SEQ ID NO:21:
   Val Cys Gly Lys Leu Phe Glu Thr Ile Pro
     1             5                   10
(2) INFORMATION FOR SEQ ID NO:22:
   (i) SEQUENCE CHARACTERISTICS:
      (A) LENGTH: 11 amino acide
      (B) TYPE: amino acid
      (D) TOPOLOGY: linear
   (ii) MOLECULE TYPE: protein
   (xi) SEQUENCE DESCRIPTION: SEQ ID NO:22:
   Asn Ser Leu Val Arg Pro Pro Thr Val Lys Arg
     1             5                   10
(2) INFORMATION FOR SEQ ID NO:23:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (fv) ANTI-SENSE: NO
   (vi) ORIGINAL SOURCE:
      (A) ORGANISM: Plasmid
   (vii) IMMEDIATE SOURCE:
      (B) CLONE: 467-21.19 (Figure 5 Junction C)
   (xi) SEQUENCE DESCRIPTION: SEQ ID NO:23:
   GGGCCAGGCA GCCCCGCAGC CGCGCGCACG TGTCTGCAGC CCAAGCTTGG CGTAATCATG    60
   GTCATA                                                               66
(2) INFORMATION FOR SEQ ID NO:24:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 536-85.30 (Figure 6 Junction A)
   (xi) SEQUENCE DESCRIPTION: SEQ ID NO:24:
   CAGTCACGAC GTTGTAAAAC GACGGCCAGT GAATTCACCA AGAAACCGAC GTGTAAAAAC    60
   TTCTCC                                                               66
(2) INFORMATION FOR SEQ ID NO:25:
   (i) SEQUENCE CHARACTERISTICS:
      (A) LENGTH: 132 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 536-85.30 (Figure 6 Junction B)
   (xi) SEQUENCE DESCRIPTION: SEQ ID NO:25:
   ACTCTGCTGA TGTTGCAGCA GGATCCTTAA TTAAGTCTAG AGTCGACTGT TTAAACCGGT   60
   TTAAACAGTC GACTCTAGAC TTAATTAAGG ATCCGGCGCG CCCCCGCTTA CTACCGCTTA  120
   CAGTTGGTGG CA                                                      132
(2) INFORMATION FOR SEQ ID NO:26:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 536-85.30 (Figure 6 Junction C)
   (xi) SEQUENCE DESCRIPTION: SEQ ID NO:26:
   GGCGCACGCT GTAGCTGGAT CGGGTACCGA GCTCGAATTG GCATGCAAGC TTGGCGTAAT   60
   CATGGT                                                              66
(2) INFORMATION FOR SEQ ID NO:27:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 495-61.39 (Figure 7 Junction A)
   (xi) SEQUENCE DESCRIPTION: SEQ ID NO:27:
   AGGTGACACT ATAGAATACA CGGAATTCGA GCTCGCCCCT GCGTTGCTAT ACACTACCTG 60
   TGGCGA 66
(2) INFORMATION FOR SEQ ID NO:28:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 495-61.39 (Figure 7 Junction 8)
   (ix) FEATURE:
      (A) NAME/KEY: COS
      (B) LOCATION: 1..24
      (D) OTHER INFORMATION: /partial
         /codon_start= 1
         /product= "Region of deleted EHV4 thymidine kinase gene"
   (ix) FEATURE:
      (A) NAME/KEY: CDS
      (B) LOCATION: 46..66
      (D) OTHER INFORMATION: /partial
         /codon_start= 46
         /product= "Region of deleted EHV4 thymidine kinase gene"
   (xi) SEQUENCE DESCRIPTION: SEQ ID NO:28:
   GTT GAT GAC GCG GCA TTA ATA ACT GCGGGGATCC TCTAGAGTCC T GTT GTA    51
   Val Asp Asp Ala Ala Leu Ile Thr                             Val Val
    1               5                                           1
   GAG TCT TTG CTG CCA                                                66
   Glu Ser Leu Leu Pro
            5
(2) INFORMATION FOR SEQ ID NO:29:
   (i) SEQUENCE CHARACTERISTICS:
      (A) LENGTH: 8 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Val Asp Asp Ala Ala Leu Ile Thr
    1               5
(2) INFORMATION FOR SEQ ID NO:30:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Val Val Glu Ser Leu Leu Pro
    1               5
(2) INFORMATION FOR SEQ ID NO:31:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 495-61.39 (Figure 7 Junction C)
   (ix) FEATURE:
      (A) NAME/KEY: CDS
      (B) LOCATION: 1..33
      (D) OTHER INFORMATION: /partial
         /codon_start= 1
         /product= "Region of EHV4 glycoprotein H gene"
   (xi) SEQUENCE DESCRIPTION: SEQ ID NO:31:
   CGC CTT CCC CCA AGA CGT CGA CTT GAG CCA CCC GACCTGCAGC  CCAAGCTTGG   53
   Arg Leu Pro Pro Arg Arg Arg Leu Glu Pro Pro
     1             5                   10
   CGTAATCATG  GTC                                                     66
(2) INFORMATION FOR SEQ ID NO:32:
   (i) SEQUENCE CHARACTERISTICS:
      (A) LENGTH: 11 amino acids
      (B) TYPE: amino acid
      (D) TOPOLOGY: linear
   (ii) MOLECULE TYPE: protein
   (xi) SEQUENCE DESCRIPTION: SEQ ID NO:32:
   Arg Leu Pro Pro Arg Arg Arg Leu Glu Pro Pro
     1             5                   10
(2) INFORMATION FOR SEQ ID NO:33:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-38.9 (Figure 8 Junction A)
   (xi) SEQUENCE DESCRIPTION: SEQ ID NO:33:
   ATACACATAC GATTTAGGTG ACACTATAGA ATACACGGAA TTCGAGCTCG CCCGGGGATC    60
   CTCTAG                                                               66
(2) INFORMATION FOR SEQ ID NO:34:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-38.9 (Figure 8 Junction B)
   (ix) FEATURE:
      (A) NAME/KEY: CDS
      (B) LOCATION: 1..33

      (D) OTHER INFORMATION: /partial
      /codon_start= 1
      /product= "Region of deleted EHV4 US2 gene"
   (xi) SEQUENCE DESCRIPTION: SEQ ID NO:34:
   AGG CCA TAC CAC CTA TGG GTT TTG GGA GCT GCA GGCACCGAAG TTTTTCGCTG    53
   Arg Pro Tyr His Leu Trp Val Leu Gly Ala Ala
     1             5                  10
   TAACTCTTGC TCG                                                       66
(2) INFORMATION FOR SEQ ID NO:35:
   (i) SEQUENCE CHARACTERISTICS:
      (A) LENGTH: 11 amino acids
      (B) TYPE: amino acid
      (D) TOPOLOGY: linear
   (ii) MOLECULE TYPE: protein
   (xi) SEQUENCE DESCRIPTION: SEQ ID NO:35:
   Arg Pro Tyr His Leu Trp Val Leu Gly Ala Ala
   1 5 10
(2) INFORMATION FOR SEQ ID NO:36:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-38.9 (Figure 8 Junction C)
   (xi) SEQUENCE DESCRIPTION: SEQ ID NO:36:
   CCCGTGCAAC AAGAGTCGTC TTCCTCGTCC GAAAAGCTTG GCGTAATCAT GGTCATAGCT 60
   GTTTCC 66
(2) INFORMATION FOR SEQ ID NO:37:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 530-57.25 (Figure 9 Junction A)
   (xi) SEQUENCE DESCRIPTION: SEQ ID NO:37:
   ATTAATACAT AACCTTATGT ATCATACACA TACGATTTAG GTGACACTAT AGAATACACG    60
   GAATTC                                                               66
(2) INFORMATION FOR SEQ ID NO:38:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 580-57.25 (Figure 9 Junction B)
   (xi) SEQUENCE DESCRIPTION: SEQ ID NO:38:
   TCTCCTCTTT GGGCGTCAAA GCAATCAGGG GGATCCTCTA GAGTCGCAGG AAATGTGTGC    60
   TATGCT                                                               66
(2) INFORMATION FOR SEQ ID NO:39:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 580-57.25 (Figure 9 Junction C)
   (xi) SEQUENCE DESCRIPTION: SEQ ID NO:39:
   GATCCCGAGT CTCGCTTCGA AAAACCGTGC GACCTGCAGC CCAAGCTTGG CGTAATCATG 60
   GTCATA 66
(2) INFORMATION FOR SEQ ID NO:40:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SEMSE: NO
   (vi) ORIGINAL SOURCE:
      (A) ORGANISM: Plasmid
   (vii) IMMEDIATE SOURCE:
      (B) CLONE: 467-22.A12 (Figure 10 Junction A)
   (xi) SEQUENCE DESCRIPTION: SEQ ID NO:40:
   GAATTCGAGC TCGCCCGGGG ATCCTCTAGA GTCGACGTCT GGGGCGCGGG GGTGGTGCTC 60
   TTCGAG 66
(2) INFORMATION FOR SEQ ID NO:41:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 467-22.A12 (Figure 10 Junction B)
   (ix) FEATURE:
      (A) NAME/KEY: CDS
      (B) LOCATION: 16..66
      (D) OTHER INFORMATION: /partial
         /codon_start= 16
         /product= "N-terminal peptide of hybrid protein"
   (xi) SEQUENCE DESCRIPTION: SEQ ID NO:41:
   CTCCACAGCT CAACA ATG AAG TGG GCA ACG TGG ATC GAT CCC GTC GTT TTA      51
                    Met Lys Trp Ala Thr Trp Ile Asp Pro Val Val Leu
                     1               5                  10
   CAA CGT CGT GAC TGG                                                   66
   Gln Arg Arg Asp Trp
           15
(2) INFORMATION FOR SEQ ID NO:42:
   (i) SEQUENCE CHARACTERISTICS:
      (A) LENGTH: 17 amino acids
      (B) TYPE: amino acid
      (D) TOPOLOGY: linear
   (ii) MOLECULE TYPE: protein
   (xi) SEQUENCE DESCRIPTION: SEQ ID NO:42:
   Met Lys Trp Ala Thr Trp Ile Asp Pro Val Val Leu Gln Arg Arg Asp
    1               5                  10                  15
   Trp
(2) INFORMATION FOR SEQ ID NO:43:
   (i) SEQUENCE CHARACTERISTICS:
      (A) LENGTH: 132 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 467-22.A12 (Figure 10 Junction C)
   (ix) FEATURE:
      (A) MAME/KEY: CDS
      (B) LOCATION: 1..93

      (D) OTHER INFORMATION: /partial
      /codon_start= 1
      /function= "Translational finish of hybrid protein"
      /product= "C-terminal peptide"
      /standard_name= "Translation of synthetic DNA sequence"
   (xi) SEQUENCE DESCRIPTION: SEQ ID NO:43:
   GAC GAC TCC TGG AGC CCG TCA GTA TCG GCG GAA ATC CAG CTG AGC GCC      48
   Asp Asp Ser Trp Ser Pro Ser Val Ser Ala Glu Ile Gln Leu Ser Ala
     1               5                  10                  15
   GGT CGC TAC CAT TAC CAG TTG GTC TGG TGT CAA AAA GAT CTA GAA          93
   Gly Arg Tyr His Tyr Gln Leu Val Trp Cys Gln Lys Asp Leu Glu
                20                  25                  30
   TAAGCTAGAG GATCGATCCC CTATGGCGAT CATCAGGGC                          132
(2) INFORMATION FOR SEQ ID NO:44:
   (i) SEQUENCE CHARACTERISTICS:
      (A) LENGTH: 31 amino acids
      (B) TYPE: amino acid
      (D) TOPOLOGY: linear
   (ii) MOLECULE TYPE: protein
   (xi) SEQUENCE DESCRIPTION: SEQ ID NO:44:
   Asp Asp Ser Trp Ser Pro Ser Val Ser Ala Glu Ile Gln Leu Ser Ala
     1               5                  10                  15
   Gly Arg Tyr His Tyr Gln Leu Val Trp Cys Gln Lys Asp Leu Glu
                20                  25                  30
(2) INFORMATION FOR SEQ ID NO:45:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 467-22.A12 (Figure 10 Junction D)
   (xi) SEQUENCE DESCRIPTION: SEQ ID NO:45:
   AACGAGGGCC AGTACCGGCG CCTGGTGTCC GTCGACTCTA GAGGATCCCC GGGCGAGCTC 60
   GAATTC 66
(2) INFORMATION FOR SEQ ID NO:46:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-42.A18 (Figure 11 Junction A)
   (xi) SEQUENCE DESCRIPTION: SEQ ID NO:46:
   AAGCTTGGCC TCGAGGGCCG CGGCCGCCTG CAGGTCGACG TCTGGGGCGC GGGGGTGGTG 60
   CTCTTC 66
(2) INFORMATION FOR SEQ ID NO:47:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-42.A18 (Figure 11 Junction 8)
   (ix) FEATURE:
      (A) NAME/KEY: CDS
      (B) LOCATION: 16..66
      (D) OTHER INFORMATION: /partial
         /codon_start= 16
         /product= "N-terminal peptide of hybrid protein"
   (xi) SEQUENCE DESCRIPTION: SEQ ID NO:47:
   CTCCACAGCT CAACA ATG AAG TGG GCA ACG TGG ATC GAT CCC GTC GTT TTA    51
                    Met Lys Trp Ala Thr Trp Ile Asp Pro Val Val Leu
                     1               5                  10
   CAA CGT CGT GAC TGG                                                 66
   Gln Arg Arg Asp Trp
           15
(2) INFORMATION FOR SEQ ID NO:48:
   (i) SEQUENCE CHARACTERISTICS:
      (A) LENGTH: 17 amino acids
      (B) TYPE: amino acid
      (D) TOPOLOGY: linear
   (ii) MOLECULE TYPE: protein
   (xi) SEQUENCE DESCRIPTION: SEQ ID NO:48:
   Met Lys Trp Ala Thr Trp Ile Asp Pro Val Val Leu Gln Arg Arg Asp
    1               5                  10                  15
   Trp
(2) INFORMATION FOR SEQ ID NO:49:
   (i) SEQUENCE CHARACTERISTICS:
      (A) LENGTH: 132 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-42.A18 (Figure 11 Junction C)
   (ix) FEATURE:
      (A) MAME/KEY: CDS
      (B) LOCATION: 1..93

      (D) OTHER INFORMATION: /partial
      /codon_start= 1
      /function= "Translational fininsh of hybrid protein"
      /product= "C-terminal peptide"
      /standard_name= "Translation of synthetic DNA sequence"
   (xi) SEQUENCE DESCRIPTION: SEQ ID NO:49:
   GAC GAC TCC TGG AGC CCG TCA GTA TCG GCG GAA ATC CAG CTG AGC GCC      48
   Asp Asp Ser Trp Ser Pro Ser Val Ser Ala Glu Ile Gln Leu Ser Ala
     1               5                  10                  15
   GGT CGC TAC CAT TAC CAG TTG GTC TGG TGT CAA AAA GAT CTA GAA          93
   Gly Arg Tyr His Tyr Gln Leu Val Trp Cys Gln Lys Asp Leu Glu
                20                  25                  30
   TAAGCTAGAG GATCGATCCC CTATGGCGAT CATCAGGGC                          132
(2) INFORMATION FOR SEQ ID NO:50:
   (i) SEQUENCE CHARACTERISTICS:
      (A) LENGTH: 31 amino acids
      (B) TYPE: amino acid
      (D) TOPOLOGY: linear
   (ii) MOLECULE TYPE: protein
   (xi) SEQUENCE DESCRIPTION: SEQ ID NO:50:
   Asp Asp Ser Trp Ser Pro Ser Val Ser Ala Glu Ile Gln Leu Ser Ala
   1 5 10 15
   Gly Arg Tyr His Tyr Gln Leu Val Trp Cys Gln Lys Asp Leu Glu
   20 25 30
(2) INFORMATION FOR SEQ ID NO:51:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 523-42.A18 (Figure 11 Junction D)
   (xi) SEQUENCE DESCRIPTION: SEQ ID NO:51:
   GAGGGCCAGT ACCGGCGCCT GGTGTCCGTC GACCTGCAGG CGGCCGCGGC CCTCGAGGCC 60
   AAGCTT 66
(2) INFORMATION FOR SEQ ID NO:52:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 552-45.19 (Figure 12 Junetion A)
   (xi) SEQUENCE DESCRIPTION: SEQ ID NO:52:
   TCTAGAGTCA GCTTGGCCTC GAGGGCCGCG GCCGCCTGCA GGTCGAGATC CCCTCGACGT   60
   CTGGGG                                                              66
(2) INFORMATION FOR SEQ ID NO:53:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 552-45.19 (Figure 12 Junction B)
   (ix) FEATURE:
      (A) NAME/KEY: CDS
      (B) LOCATION: 31..66
      (D) OTHER INFORMATION: /partial
         /codon_start= 31
         /product= "N-terminal peptide of hybrid protein"
   (xi) SEQUENCE DESCRIPTION: SEQ ID NO:53:
   CACACCTTTG CGCATCTCCA CAGCTCAACA ATG AAT TCC ATG TTA CGT CCT GTA   54
                                    Met Asn Ser Met Leu Arg Pro Val
                                     1               5
   GAA ACC CCA ACC                                                    66
   Glu Thr Pro Thr
        10
(2) INFORMATION FOR SEQ ID NO:54:
   (i) SEQUENCE CHARACTERISTICS:
      (A) LENGTH: 12 amino acids
      (B) TYPE: amino acid
      (D) TOPOLOGY: linear
   (ii) MOLECULE TYPE: protein
   (xi) SEQUENCE DESCRIPTION: SEQ ID NO:54:
   Met Asn Ser Met Leu Arg Pro Val Glu Thr Pro Thr
   1               5                   10
(2) INFORMATION FOR SEQ ID NO:55:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 552-45.19 (Figure 12 Junction C)
   (ix) FEATURE:
      (A) NAME/KEY: CDS
      (B) LOCATION: 1..15
      (D) OTHER INFORMATION: /partial
         /codon_start= 1
         /product= "C-terminal peptide of hybrid protein"
   (xi) SEQUENCE DESCRIPTION: SEQ ID NO:55:
   CAG GGA GGC AAA CAA TGAATCAACA ACTCTCCCGG GAGATGGGGG AGGCTAACTG    55
   Gln Gly Gly Lys Gln
    1               5
   AAACACGGAA G                                                       66
(2) INFORMATION FOR SEQ ID NO:56:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:56:
   Gln Gly Gly Lys Gln
   1               5
(2) INFORMATION FOR SEQ ID NO:57:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 552-45.19 (Figure 12 Junction D)
   (xi) SEQUENCE DESCRIPTION: SEQ ID NO:57:
   TGCTGCGTTC CCGGGGATCT CGACCTGCAG GGCGGCCGCG GCCCTCGAGG CCAAGCTGAC   60
   TCTAGA                                                              66
(2) INFORMATION FOR SEQ ID NO:58:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 593-31.2 (Figure 13 Junction A)
   (xi) SEQUENCE DESCRIPTION: SEQ ID NO:58:
   GTCGACTCTA GACTTAATTA AGGATCCGGC GCGCCCCCTC GACGTCTGGG GCGCGGGGGT 60
   GGTGCT 66
(2) INFORMATION FOR SEQ ID NO:59:
   (i) SEQUENCE CHARACTERISTICS:
      (A) LENGTH: 66 base pairs
      (B) TYPE: nuclaic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 593-31.2 (Figure 13 Junction B)
   (ix) FEATURE:
      (A) NAME/KEY: COS
      (B) LOCATION: 16..66

      (D) OTHER INFORMATION: /partial
      /product= "N-terminal peptide of hybrid protein"
      /gene= "16"
   (xi) SEQUENCE DESCRIPTION: SEQ ID NO:59:
   CTCCACAGCT CAACA ATG AAG TGG GCA ACG TGG ATC GAT CCC GTC GTT TTA 51
                    Met Lys Trp Ala Thr Trp Ile Asp Pro Val Val Leu
                     1               5                  10
   CAA CGT CGT GAC TGG 66
   Gln Arg Arg Asp Trp
            15
(2) INFORMATION FOR SEQ ID NO:60:
   (i) SEQUENCE CHARACTERISTICS:
      (A) LENGTH: 17 amino acids
      (B) TYPE: amino acid
      (D) TOPOLOGY: linear
   (ii) MOLECULE TYPE: protein
   (xi) SEQUENCE DESCRIPTION: SEQ ID NO:60:
   Met Lys Trp Ala Thr Trp Ile Asp Pro Val Val Leu Gln Arg Arg Asp
     1               5                  10                  15
   Trp
(2) INFORMATION FOR SEQ ID NO:61:
   (i) SEQUENCE CHARACTERISTICS:
      (A) LENGTH: 132 base pairs
      (B) TYPE: nucleic acid
      (C) STRANDEDNESS: double
      tD) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 593-31.2 (Figure 13 Junction C)
   (ix) FEATURE:
      (A) MAME/KEY: CDS
      (B) LOCATION: 1..93
      tD) OTHER INFORMATION: /partial
         /product= "C-terminal peptide of hybrid protein"
         /gene= "1"
   (xi) SEQUENCE DESCRIPTION: SEQ ID NO:61:
   GAC GAC TCC TGG AGC CCG TCA GTA TCG GCG GAA ATC CAG CTG AGC GCC     48
   Asp Asp Ser Trp Ser Pro Ser Val Ser Ala Glu Ile Gln Leu Ser Ala
    1               5                  10                  15
   GGT CGC TAC CAT TAC CAG TTG GTC TGG TGT CAA AAA GAT CTA GAA         93
   Gly Arg Tyr His Tyr Gln Leu Val Trp Cys Gln Lys Asp Leu Glu
               20                  25                  30
   TAAGCTAGAG GATCGATCCC CTATGGCGAT CATCAGGGC                          132
(2) INFORMATION FOR SEQ ID NO:62:
   (i) SEQUENCE CHARACTERISTICS:
      (A) LENGTH: 31 amino acids
      (B) TYPE: amino acid
      (D) TOPOLOGY: linear
   (ii) MOLECULE TYPE: protein
   (xi) SEQUENCE DESCRIPTION: SEQ ID NO:62:
   Asp Asp Ser Trp Ser Pro Ser Val Ser Ala Glu Ile Gln Leu Ser Ala
    1               5                  10                  15
   Gly Arg Tyr His Tyr Gln Leu Val Trp Cys Gln Lys Asp Leu Glu
               20                  25                  30
(2) INFORMATION FOR SEQ ID NO:63:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:63:
   GGGTCGACAT GAAGACAACC ATTATTTTGA TAC                                33
(2) INFORMATION FOR SEQ ID NO:64:
   (i) SEQUENCE CHARACTERISTICS:
      (A) LENGTH: 66 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Plasmid
   (vii) IMMEDIATE SOURCE:
      (B) CLONE: 593-31.2 (figure 13 Junction D)
   (xi) SEQUENCE DESCRIPTION: SEQ ID NO:64:
   GCCAGTACCG GCGCCTGGTG TCCGTCGAGG GGGCGCGCCG GATCCTTAAT TAAGTCTAGA 60
   GTCGAC 66
(2) INFORMATION FOR SEQ ID NO:65:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:65:
   GGGTCGACTC AAATGCAAAT GTTGCATCTG AT 32
(2) INFORMATION FOR SEQ ID NO:66:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:66:
   GGGATCCATG AACACTCAAA TTCTAATATT AG 32
(2) INFORMATION FOR SEQ ID NO:67:
   (1) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:67:
   GGGATCCTTA TATACAAATA GTGCACCGCA 30
(2) INFORMATION FOR SEQ ID NO:68:
   (i) SEQUENCE CHARACTERISTICS:
      (A) LENGTH: 30 base pairs
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:68:
   GGGATCCTTA TATACAAATA GTGCACCGCA 30
(2) INFORMATION FOR SEQ ID NO:69:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:69:
   GGGTCGACTT ACATCTTATC GATGTCAAA 29
(2) INFORMATION FOR SEQ ID NO:70:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:70:
(2) INFORMATION FOR SEQ ID NO:71:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Synthetic oligonucleotide primer
   (xi) SEQUENCE DESCRIPTION: SEQ ID NO:71:
   GGGATCCTTA CGAAAAGTAT TTAATTTGTG C 31